# EUROPEAN PATENT SPECIFICATION

(11) **EP 1 833 996 B1**
(45) Date of publication and mention of the grant of the patent: **05.12.2012**
(21) Application number: 06733637.0
(22) Date of filing: 05.01.2006
(51) Int. Cl.: C12Q 1/68

(54) **CANCER PROGNOSTIC METHODS**
VERFAHREN ZUR PROGNOSE VON KREBS
PROCEDES DE PRONOSTIC DU CANCER

(30) Priority: 06.01.2005 US 642164 P
(43) Date of publication of application: 19.09.2007
(73) Proprietor: Genentech, Inc., South San Francisco CA 94080-4990 (US)
(72) Inventor: JUBB, Adrian, M., San Francisco, CA 94110 (US); KOEPPEN, Hartmut, Berkeley, CA 94709 (US)
(74) Representative: Kiddle, Simon John
(86) International application number: PCT/US2006/000497
(87) International publication number: WO 2006/074392

(56) References cited:
- WO-A1-2005/014818
- MAO WEIGUANG ET AL: "EphB2 as a therapeutic antibody drug target for the treatment of colorectal cancer" CANCER RESEARCH, AMERICAN ASSOCIATION FOR CANCER RESEARCH, BALTIMORE, MD, US, vol. 64, no. 3, 1 February 2004 (2004-02-01), pages 781-788, XP002343724 ISSN: 0008-5472 cited in the application
- KIYOKAWA E ET AL: "Overexpression of ERK, an EPH family receptor protein tyrosine kinase, in various human tumors" CANCER RESEARCH, AMERICAN ASSOCIATION FOR CANCER RESEARCH, BALTIMORE, MD, US, vol. 54, no. 14, 15 July 1994 (1994-07-15), pages 3645-3650, XP001156923 ISSN: 0008-5472
- DODELET V C ET AL: "EPH RECEPTORS AND EPHRIN LIGANDS: EMBRYOGENSIS TO TUMORIGENESIS" ONCOGENE, BASINGSTOKE, HANTS, GB, vol. 19, 2000, pages 5614-5619, XP002951836 ISSN: 0950-9232
- NAKADA MITSUTOSHI ET AL: "The phosphorylation of EphB2 receptor regulates migration and invasion of human glioma cells" CANCER RESEARCH, AMERICAN ASSOCIATION FOR CANCER RESEARCH, BALTIMORE, MD, US, vol. 64, no. 9, 1 May 2004 (2004-05-01), pages 3179-3185, XP002382537 ISSN: 0008-5472
- LIU WENBIAO ET AL: "Coexpression of ephrin-Bs and their receptors in colon carcinoma." CANCER. 15 FEB 2002, vol. 94, no. 4, 15 February 2002 (2002-02-15), pages 934-939, XP001156840 ISSN: 0008-543X
- DORONINA S O ET AL: "Development of potent monoclonal antibody auristatin conjugates for cancer therapy" NATURE BIOTECHNOLOGY, NATURE PUBLISHING GROUP, NEW YORK, NY, US, vol. 21, no. 7, July 2003 (2003-07), pages 778-784, XP002280966 ISSN: 1087-0156
- JUBB ADRIAN M ET AL: "EphB2 is a prognostic factor in colorectal cancer" CLINICAL CANCER RESEARCH, THE AMERICAN ASSOCIATION FOR CANCER RESEARCH, US, vol. 11, no. 14, 15 July 2005 (2005-07-15), pages 5181-5187, XP002382565 ISSN: 1078-0432
- GUO DONG LI ET AL: "Reduced expression of EphB2 that parallels invasion and metastasis in colorectal tumours" CARCINOGENESIS, IRL PRESS, LONDON, GB, vol. 27, no. 3, 4 November 2005 (2005-11-04), pages 454-464, XP002382564 ISSN: 0143-3334
- LUGLI ALESSANDRO ET AL: "EphB2 expression across 138 human tumor types in a tissue microarray: high levels of expression in gastrointestinal cancers." CLINICAL CANCER RESEARCH : AN OFFICIAL JOURNAL OF THE AMERICAN ASSOCIATION FOR CANCER RESEARCH. 15 SEP 2005, vol. 11, no. 18, 15 September 2005 (2005-09-15), pages 6450-6458, XP002399817 ISSN: 1078-0432
- BATLLE EDUARD ET AL: "EphB receptor activity suppresses colorectal cancer progression." NATURE. 23 JUN 2005, vol. 435, no. 7045, 23 June 2005 (2005-06-23), pages 1126-1130, XP002399818 ISSN: 1476-4687
- ALAZZOUZI HAFID ET AL: "Mechanisms of inactivation of the receptor tyrosine kinase EPHB2 in colorectal tumors." CANCER RESEARCH. 15 NOV 2005, vol. 65, no. 22, 15 November 2005 (2005-11-15), pages 10170-10173, XP002399819 ISSN: 0008-5472

## Description

### FIELD OF THE INVENTION

The invention concerns detection of EphB2 polypeptide and/or polynucleotide for colorectal cancer prognostic methods, and methods for selecting cancer treatment.

### BACKGROUND OF THE INVENTION

Cancer remains to be one of the most deadly threats to human health. In the U.S., cancer affects nearly 1.3 million new patients each year, and is the second leading cause of death after heart disease, accounting for approximately 1 in 4 deaths. It is also predicted that cancer may surpass cardiovascular diseases as the number one cause of death within 5 years. Solid tumors are responsible for most of those deaths. Although there have been significant advances in the medical treatment of certain cancers, the overall 5-year survival rate for all cancers has improved only by about 10% in the past 20 years. Cancers, or malignant tumors, metastasize and grow rapidly in an uncontrolled manner, making timely detection and treatment extremely difficult. Colorectal cancer is the third most common cause of cancer mortality in the United States. It was estimated that approximately 129,000 new cases of colorectal cancer would be diagnosed and 56,000 deaths would occur due to colorectal cancer in the United States in 1999 (Landis et al., Cancer J Clin. 49:8-31 (1999)).

Cancer treatment, such as chemotherapy, radiation and/or surgery, has associated risks, and it would be useful to be able to optimally select patients most likely to benefit. Prognostic testing is useful to, for example, identify patients with poor prognoses such that a more aggressive, higher risk treatment approach is identified, and to identify patients with good prognoses for whom risky therapy would not provide enough benefit to warrant the risks. There is an urgent need for new cancer prognostic factors.

Eph receptors make up the largest family of receptor tyrosine kinases in the human genome and interact with ligands called ephrins (reviewed in Kullander et al., Nat Rev Mol Cell Biol 2002;3:475-86.). The family is divided by sequence identity into two classes, EphA and EphB, with corresponding transmembrane ligand families, referred to as type-A and type-B ephrins. EphB2 receptor ("EphB2" or "EphB2R") has an extracellular region with a cysteine-rich motif extending over its amino-terminal half followed by two fibronectin type II motifs. There is an intracellular domain featuring a conserved kinase region and a transmembrane domain. EphB2 expression has been described in cancer. See, e.g., Cairns et al., WO2003/000113; Mao et al, Cancer Res. 64, 781-788 (2004).

### SUMMARY OF THE INVENTION

In one aspect the invention provides a method for cancer prognosis in a human subject diagnosed with colorectal cancer, as defined in the claims, the method comprising: (a) comparing expression of EphB2 in a colorectal tissue or cell sample from the patient with expression of EphB2 in a control sample (control reference value); and (b) predicting cancer prognosis of the patient based on the comparison in (a), wherein EphB2 expression in the patient sample relative to a control sample is prognostic for cancer in the subject. Increased EphB2 expression in the patient biological sample relative to the control sample is prognostic for cancer in the subject.

In accordance with the claims, prognostic for cancer comprises providing the forecast or prediction of (prognostic for) duration of survival of a patient diagnosed with a cancer, or duration of recurrence-free survival .

Increased EphB2 expression in the patient biological sample relative to the control sample is prognostic for longer duration of survival or longer recurrence-free survival.

In another aspect, as defined in the claims the invention provides methods for selection of colorectal cancer treatment for a human patient, the methods comprising (a) comparing expression of EphB2 in a colorectal tissue or cell sample from the patient with expression of EphB2 in a control sample; (b) predicting cancer prognosis of the patient based on the comparison in (a), wherein EphB2 expression in the patient increased sample relative to a control sample is prognostic for increased duration of survival or increased duration of recurrence-free survival in the subject and (c) subsequent to steps (a) and (b), selecting cancer treatment for the patient, wherein the selection of treatment is based on the patient prognosis determined in step (b). In some embodiments, increased EphB2 expression in the patient biological sample relative to the control sample is prognostic for cancer in the subject. In some embodiments, decreased EphB2 expression in the patient biological sample relative to the control sample is prognostic for cancer in the subject.

Also disclosed is a method for detection of EphB2 polynucleotide or polypeptide in a biological sample from a patient having or suspected of having a colon adenoma disorder, the method comprising comparing expression of EphB2 polynucleotide or polypeptide in the biological sample with expression of EphB2 in a control sample. In some embodiments, the biological sample comprises colon adenoma cells and/or tissue.

Also disclosed is a method for diagnosis of a colon adenoma disorder, the method comprising detecting expression of EphB2 polynucleotide or polypeptide in the biological sample

method: for treating a patient having or suspected of having a colon adenoma disorder by administering an effective amount of an anti-EphB2 antibody (such as an anti-EphB2 antagonist antibody) to the patient.

Also disclosed is a method: for treating a patient having or suspected of having a colon adenoma disorder by administering an effective amount of an anti-EphB2 immunoconjugate to the patient.

Also disclosed is a method for treating a patient having or suspected of having a colon adenoma disorder by administering an effective amount of an anti-EphB2 antibody, or an effective amount of an anti-EphB2 immunoconjugate to the patient, further wherein EphB2 expression is detected in colon adenoma cells and/or tissue from the human patient before, during or after administration of an anti-EphB2 antibody or an anti-EphB2 immunoconjugate. In some embodiments, EphB2 over-expression is detected before, during and/or after administration of an anti-EphB2 antibody or an anti-EphB2 immunoconjugate. Expression may be detected before; during; after; before and during; before and after; during and after; or before, during and after administration of an anti-EphB2 antibody or an anti-EphB2 immunoconjugate.

In some embodiments, the colon adenoma disorder may be selected from the group consisting of familial adenomatous polyposis, Peutz-Jegher's syndrome, Juvenile Polyposis Syndrome, Hereditary Mixed Polyposis syndrome, Cowden disease, and Bannayan-Ruvalcaba-Riley syndrome.

In embodiments involving selection of cancer treatment or selection of colon adenoma disorder treatment, the cancer treatment or colon adenoma disorder treatment may comprise administering an effective amount of an anti-EphB2 antibody, or an effective amount of an immunoconjugate comprising an anti-EphB2 antibody. In still other embodiments, the treatment comprises any one or more of chemotherapy, radiation, and surgery.

In some embodiments involving administration of antibodies, the anti-EphB2 antibody is selected from the group consisting of a monoclonal antibody, an affinity matured antibody, a human antibody, a humanized antibody, and an antibody fragment. In some embodiments, the anti-EphB2 antibody is the antibody produced by hybridoma cell line 2H9.11.14 having American Tissue Type Culture (ATCC) No. PTA-6606 (deposited February 24, 2005). In some embodiments, the anti-EphB2 antibody is an antibody comprising heavy and/or light chain variable domain(s) of the antibody produced by hybridoma cell line 2H9.11.14 having American Tissue Type Culture (ATCC) No. PTA-6606, wherein said antibody specifically binds human EphB2. In some embodiments, the anti-EphB2 antibody comprises at least one (at least 2, at least 3, at least 4, at least 5, and/or 6) hypervariable sequence(s) (HVR(s)) comprising a sequence selected from the group consisting of HVR-L1, HVR-L2, HVR-L3, HVR-H1, HVR-H2, and/or HVR-H3 of the antibody produced by hybridoma cell line 2H9.11.14 having American Tissue Type Culture (ATCC) No. PTA-6606, wherein said antibody specifically binds human EphB2. In some embodiments, the anti-EphB2 antibody is an antibody that binds to the same epitope on human EphB2 as the antibody produced by hybridoma cell line 2H9.11.14 having American Tissue Type Culture (ATCC) No. PTA-6606. In some embodiments, the anti-EphB2 antibody is an antibody that competes with the antibody produced by hybridoma cell line 2H9.11.14 having American Tissue Type Culture (ATCC) No. PTA-6606 for binding to human EphB2. In some embodiments, the anti-EphB2 antibody comprises: at least one, two, three, four, five, and/or six hypervariable region (HVR) sequences selected from the group consisting of: (a) HVR-L1 comprising sequence KSSQSLLNSGNQENYLA (SEQ ID NO: 1); (b) HVR-L2 comprising sequence GASTRES (SEQ ID NO:2); (c) HVR-L3 comprising sequence QNDHSYPFT (SEQ ID NO:3); (d) HVR-H1 comprising sequence SYWMH (SEQ ID NO:4); (e) HVR-H2 comprising sequence FINPSTGYTDYNQKFKD (SEQ ID NO:5); and (f) HVR-H3 comprising sequence RLKLLRYAMDY (SEQ ID NO:6). In one embodiment, the anti-EphB2 antibody comprises a light chain variable domain having the sequence:
DIVMTQSPSSLSVSAGEKVTMNCKSSQSLLNSGNQENYLAWYQQKPGQPPKLLIYGASTRESGVPDRF TGSGSGTDFTLTISSVQAEDLAVYYCQNDHSYPFTFGAGTKVEIKR (SEQ ID NO:7). In one embodiment, the anti-EphB2 antibody comprises a heavy chain variable domain having the sequence:
QVQLQQSGAELAKPGASVKMSCKASGYTFTSYWMHWVKQRPGQGLEWIGFINPSTGYTDYNQKFKD KATLTVKSSNTAYMQLSRLTSEDSAVYYCTRRLKLLRYAMDYWGQGTTLTVSA (SEQ ID NO:8). In one embodiment, the anti-EphB2 antibody comprises a light chain variable domain having the sequence: DIVMTQSPSSLSVSAGEKVTMNCKSSQSLLNSGNQENYLAWYQQKPGQPPKLLIYGASTRESGVPDRF TGSGSGTDFTLTISSVQAEDLAVYYCQNDHSYPFTFGAGTKVEIKR (SEQ ID NO:7); and comprises a heavy chain variable domain having the sequence:

In some embodiments, the anti-EphB2 immunoconjugate further comprises a toxin, a chemotherapy agent, a growth inhibitory agent, or radioactive material. In some embodiments, the immunoconjugate comprises a maytansinoid. In some embodiments, the immunoconjugate comprises MMAE.

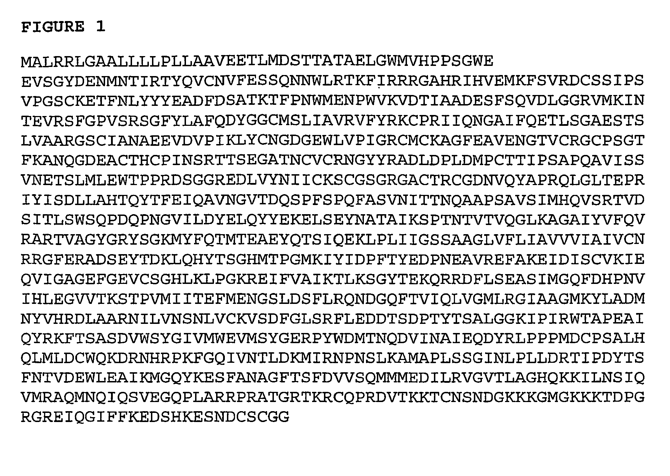

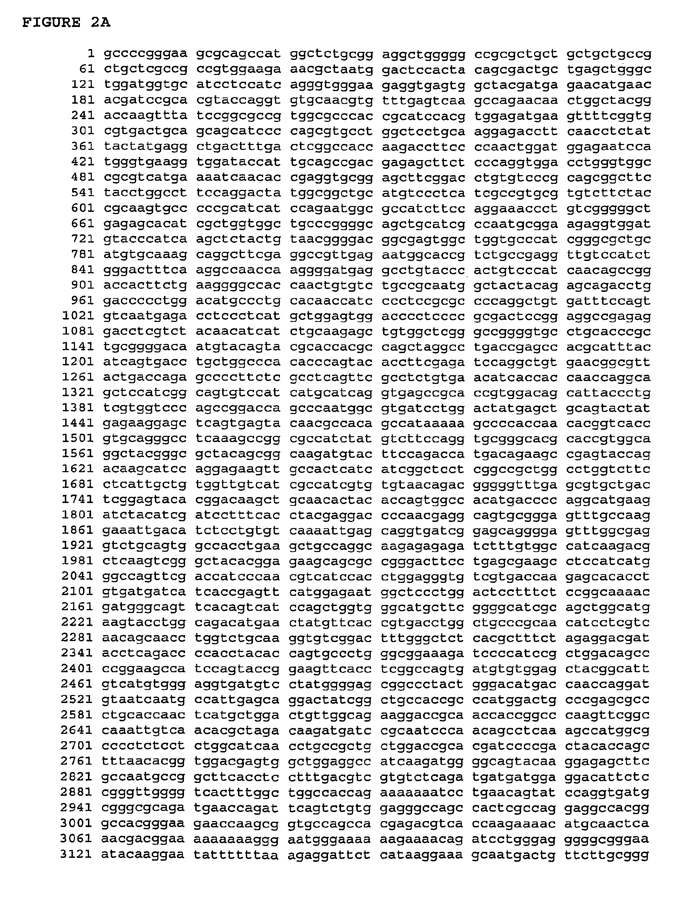

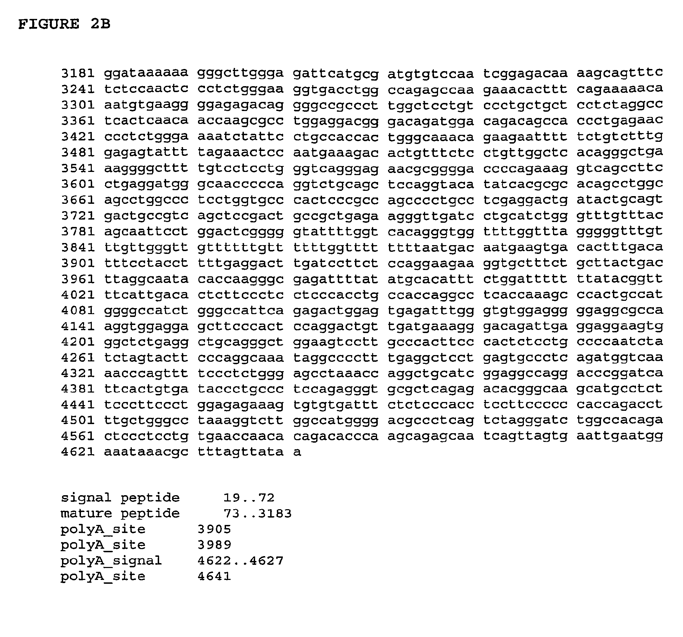

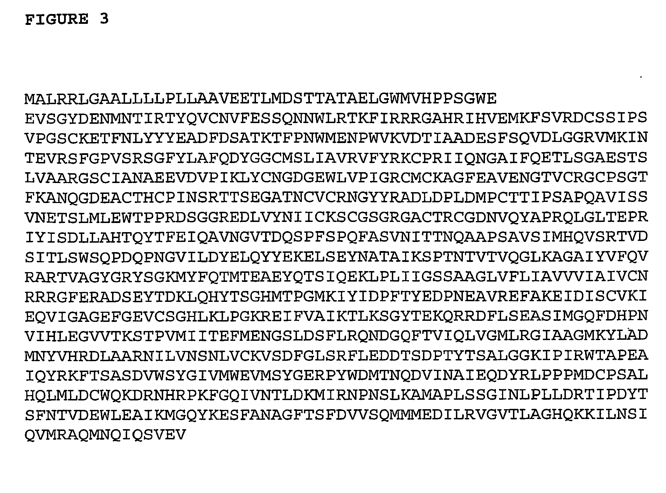

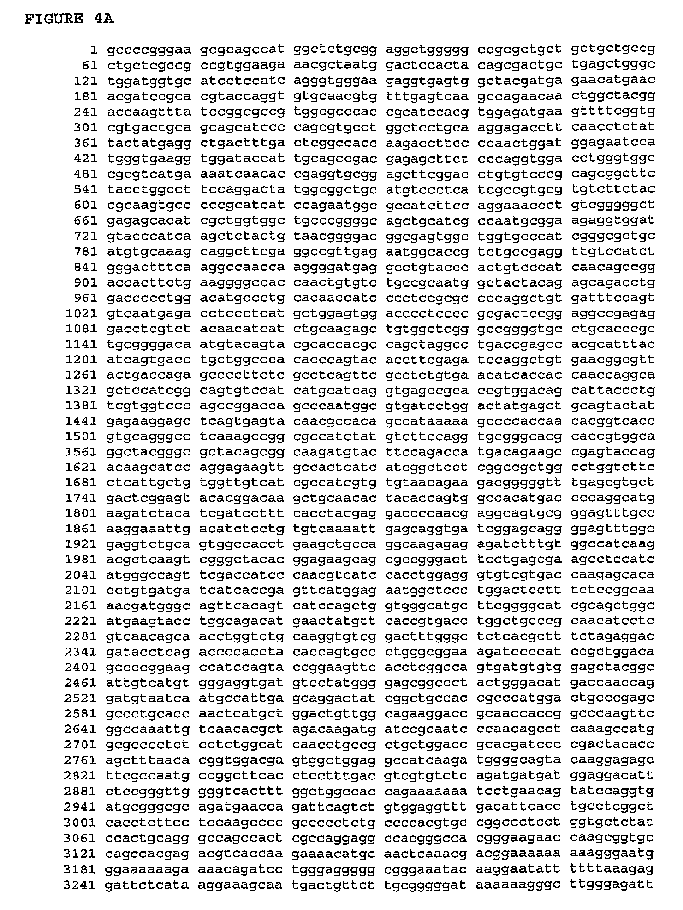

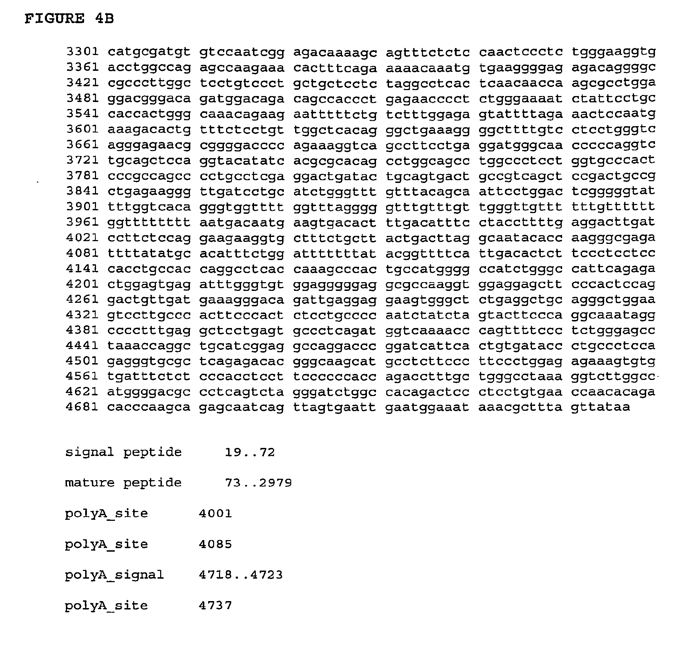

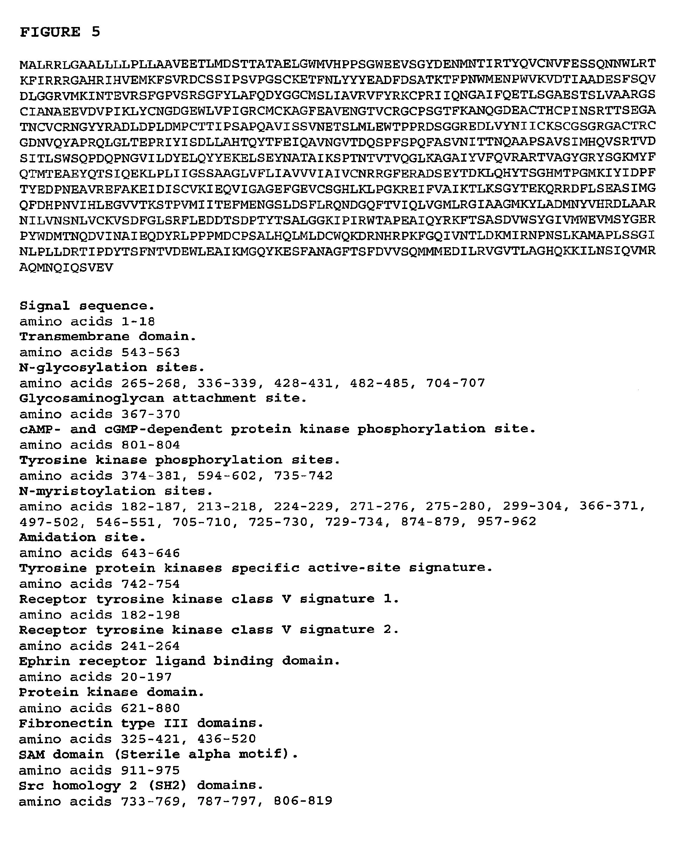

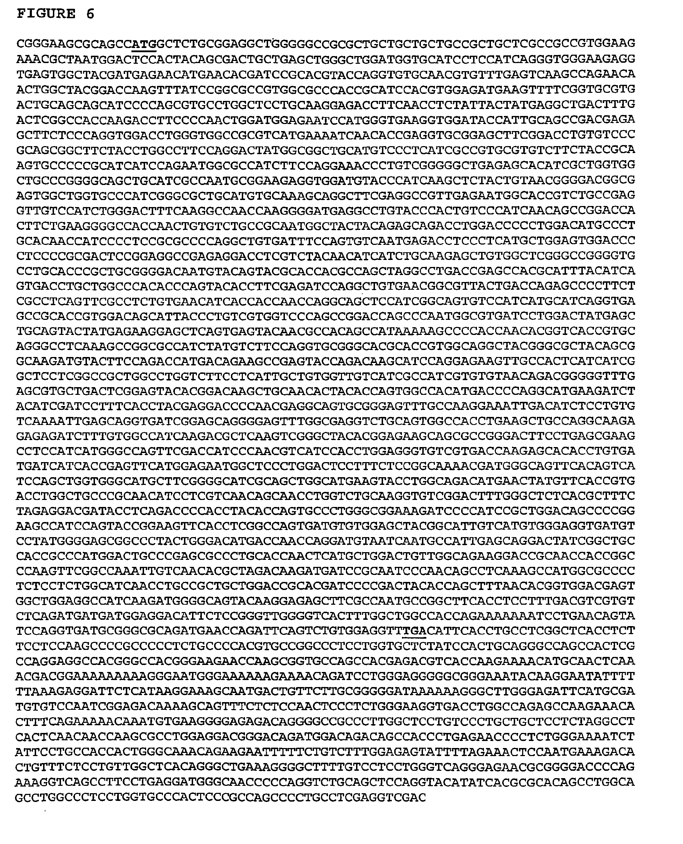

In embodiments involving detection of EphB2 expression, EphB2 polynucleotide expression and/or EphB2 polypeptide expression may be detected. In embodiments involving detection of EphB2 expression, EphB2 mRNA expression is detected. In other embodiments, EphB2 polypeptide expression is detected using an anti-EphB2 agent. In some embodiments, EphB2 polypeptide expression is detected using an antibody. Any suitable antibody may be used for detection, including monoclonal and/or polyclonal antibodies, a human antibody, a chimeric antibody, an affinity-matured antibody, a humanized antibody, and/or an antibody fragment. In some embodiments, EphB2 polypeptide expression is detected using immunohistochemistry (IHC). In some embodiments, EphB2 expression is scored at 2 or higher using an IHC. In some embodiments, an EphB2 variant and/or fragment is detected. In some embodiments, the variant polypeptide has at least about 80%, 81%, 82%, 83%, 84%, 85%, 86%, 87%, 88%, 89%, 90%, 91%, 92%, 93%, 94%, 95%, 96%, 97%, 98%, or 99% amino acid sequence identity with a native sequence polypeptide, in some embodiments, the polypeptide shown in Figures 1, 3, and/or 5. In other embodiments, the variant polypeptide is encoded by a polynucleotide sequence which hybridizes under stringent conditions with an EphB2 polynucleotide sequence, in some embodiments, the polynucleotide sequences shown in figures 2, 4, and/or 6. In some embodiments, the variant polynucleotide has at least about 80%, 81%, 82%, 83%, 84%, 85%, 86%, 87%, 88%, 89%, 90%, 91%, 92%, 93%, 94%, 95%, 96%, 97%, 98%, or 99% polynucleotide sequence identity with a native sequence polynucleotide, in some embodiments, the polynucleotide shown in Figures 2, 4, and/or 6. In other embodiments, the variant polynucleotide comprises a polynucleotide sequence which hybridizes under stringent conditions with an EphB2 polynucleotide sequence, in some embodiments, the polynucleotide sequences shown in figures 1, 3, and/or 5. In some embodiments, the EphB2 variants are biologically active. EphB2 biological activities are well known in the art and include, but are not limited to, any one or more of the following: (a) bind EphB2 ligand(s) (such as ephrin-B1, ephrin-B2, ephrin-B3 and/or ephrin-A4); (b) bind EphB2 ligand and activate EphB2 ligand biological activity or downstream pathways mediated by EphB2 ligand; (c) signal in response to EphB2 ligand binding.

In some embodiments involving detection of EphB2 expression, presence and/or absence and/or level of EphB2 expression may be detected. EphB2 expression may be increased. It is understood that absence of EphB2 expression includes insignificant, or de minimus levels. In some embodiments, EphB2 expression in the test biological sample is higher than that observed for a control biological sample (or control level of expression). In some embodiments, EphB2 expression is at least about 2-fold, 5-fold, 10-fold, 20-fold, 30-fold, 40-fold, 50-fold, 75-fold, 100-fold, 150-fold higher, or higher in the test biological sample than in the control biological sample. In some embodiments, EphB2 polypeptide expression is determined in an immunohistochemistry ("IHC") assay to score at least 2 or higher for staining intensity. In some embodiments, EphB2 polypeptide expression is determined in an IHC assay to score at least 1 or higher, or at least 3 or higher for staining intensity. In some embodiments, in the test biological sample is lower than that observed for a control biological sample (or control expression level). In some embodiments, EphB2 expression is at least about 2-fold, 5-fold, 10-fold, 20-fold, 30-fold, 40-fold, 50-fold, 75-fold, 100-fold, 150-fold lower, or lower in the test biological sample than in the control biological sample.

In some embodiments involving detection, the methods further comprise detection of expression of one or more EphB2 ligand.

Also disclosed are kits, compositions, and articles of manufacture comprising EphB2 polynucleotide(s) and/or polypeptide(s) and/or anti-EphB2 immunoconjugates. In some embodiments, the kits and articles of manufacture further comprise instructions for any method disclosed herein.

### BRIEF DESCRIPTION OF THE FIGURES

FIGURE 1: depicts the predicted amino acid sequence of EphB2 transcript variant 1 (GenBank accession no. NM_017449) (SEQ ID NO:9).
FIGURES 2A and 2B: depict the cDNA sequence of EphB2 transcript variant 1 (GenBank accession no. NM_017449) (SEQ ID NO: 10).
FIGURE 3: depicts the predicted amino acid sequence of EphB2 transcript variant 2 (GenBank accession no. NM_004442) (SEQ ID NO: 11).
FIGURES 4A and 4B: depict the cDNA sequence of EphB2 transcript variant 2 (GenBank accession no. NM_004442) (SEQ ID NO: 12).
FIGURE 5: depicts the predicted amino acid sequence of EphB2 disclosed in Figure 101 of WO03/000113 (SEQ ID NO: 13).
FIGURE 6: depicts the cDNA sequence of EphB2 disclosed in Figure 23 of WO03/000113 (SEQ ID NO:14).
FIGURE 7: depicts EphB2 (interchangeably termed "EphB2") mRNA expression in whole tissues representing colorectal tumor development (A), microdissected epithelium from normal colon and primary cancers (B) (obtained from Gene Logic), and colorectal tumor cell lines (C). EphB2 expression is represented as the mean signal intensity (+/- 95% confidence intervals) from probeset 209588_at on the Affymetrix HG-U133 GeneChip probearray. The cell lines are grouped according to their EphB2 immunohistochemistry (IHC) intensity score.
FIGURE 8: EphB2 is expressed at the base of normal colonic crypts (A) and at all stages of colorectal tumorigenesis, including adenomatous crypts (B top left, adjacent normal crypts bottom right), primary cancers (C), lymph node metastases (D), mesenteric metastases (E), and hepatic metastases (F). Membranous and cytoplasmic EphB2 localization are shown by DAB chromogen deposition (brown) against a hematoxylin counterstain (blue).

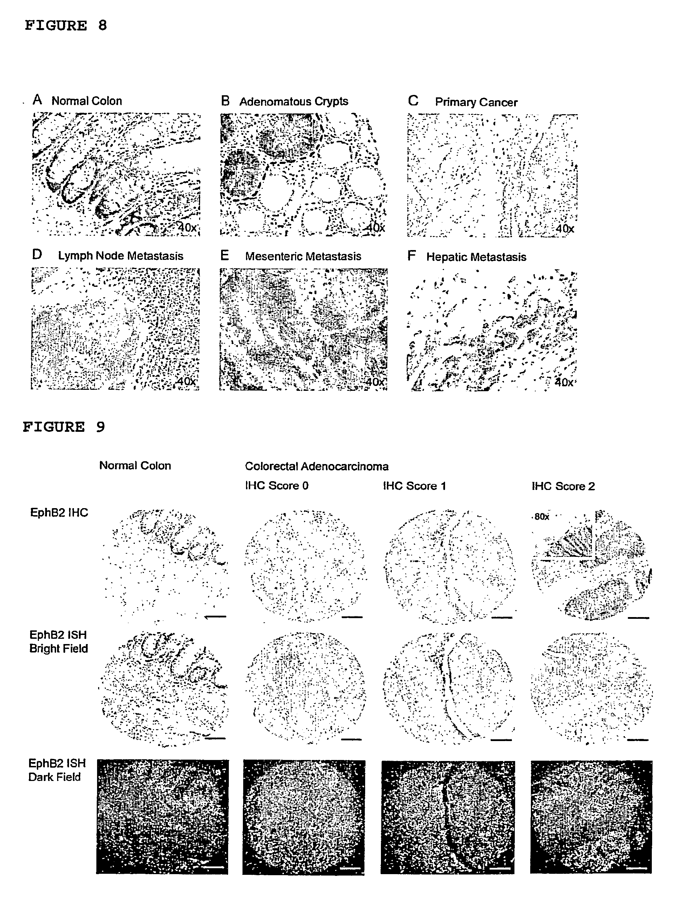

FIGURE 9: shows EphB2 expression by immunohistochemistry (IHC) and in situ hybridization (ISH) in normal colon and colorectal cancers. Shown are representative primary cancers with immunohistochemical scores of zero, one, and two. Membranous and cytoplasmic DAB chromogen deposition (brown), illustrating EphB2 expression, is observed over normal colon and neoplastic cells, against a hematoxylin counterstain (blue). Corresponding bright field (stained with hematoxylin and eosin) and dark field ISH images demonstrate an identical pattern of epithelial-restricted EphB2 expression, shown by the deposition of silver grains in the dark field. Bar = 100µm.
FIGURE 10: depicts the frequency of EphB2 expression in colorectal adenomas (TMA, n = 148), and a series of primary cancers (whole section, n = 28) and metastases (whole section, n = 39).
FIGURE 11: depicts Kaplan-Meier plots demonstrating overall survival (A), and recurrence-free survival (B) for CRC patient subgroups with high EphB2 expression (score 2) or low EphB2 expression (score 0 or 1). Hazard ratios for high EphB2 expression were 0.45, 95% confidence intervals (CI) 0.18-0.95, for overall survival, and 0.60, CI 0.30-1.10, for recurrence-free survival. The dotted line in (A) and (B) represents EphB2 Score 0, 1; and the solid line in (A) and (B) represents EphB2 Score 2.

### DETAILED DESCRIPTION OF THE INVENTION

Disclosed herein are methods to detect a polypeptide(s) (e.g., EphB2) in a biological sample from a subject, such as a human subject. In accordance with the invention, applicants surprisingly found that the expression of EphB2 is predictive of cancer prognosis. Therefore, the disclosed methods can provide for convenient, efficient, and potentially cost-effective means to obtain data and information useful in assessing future course of the disorder, including selection of appropriate therapies for treating patients.

In another aspect, the invention also provides methods for selection of cancer treatment. In some embodiments, the treatment comprises administration of an effective amount of an anti-EphB2 agent (such as an antibody), or an effective amount of an immunoconjugate comprising an anti-EphB2 antibody conjugated to a cytotoxic agent such as a chemotherapeutic agent, a growth inhibitory agent, a toxin (e.g., an active toxin of synthetic, bacterial, fungal, plant, or animal origin, or fragments thereof), or a radioactive isotope (i.e., a radioconjugate).

Also disclosed are methods for detecting EphB2 expression in patient having or suspected of having a colon adenoma disorder, methods for diagnosis of a colon adenoma disorder, and methods for treating colon cancer disorders.

Kits, compositions, and articles of manufacture are also disclosed.

### GENERAL TECHNIQUES

The techniques and procedures described or referenced herein are generally well understood and commonly employed using conventional methodology by those skilled in the art, such as, for example, the widely utilized methodologies described in Sambrook et al., Molecular Cloning: A Laboratory Manual 3rd. edition (2001) Cold Spring Harbor Laboratory Press, Cold Spring Harbor, N.Y. CURRENT PROTOCOLS IN MOLECULAR BIOLOGY (F. M. Ausubel, et al. eds., (2003)); the series METHODS IN ENZYMOLOGY (Academic Press, Inc.): PCR 2: A PRACTICAL APPROACH (M. J. MacPherson, B. D. Hames and G. R. Taylor eds. (1995)), Harlow and Lane, eds. (1988) ANTIBODIES, A LABORATORY MANUAL, and ANIMAL CELL CULTURE (R. I. Freshney, ed. (1987)); Oligonucleotide Synthesis (M. J. Gait, ed., 1984); Methods in Molecular Biology, Humana Press; Cell Biology: A Laboratory Notebook (J. E. Cellis, ed., 1998) Academic Press; Animal Cell Culture (R. I. Freshney), ed., 1987); Introduction to Cell and Tissue Culture (J. P. Mather and P. E. Roberts, 1998) Plenum Press; Cell and Tissue Culture: Laboratory Procedures (A. Doyle, J. B. Griffiths, and D. G. Newell, eds., 1993-8) J. Wiley and Sons; Handbook of Experimental Immunology (D. M. Weir and C. C. Blackwell, eds.); Gene Transfer Vectors for Mammalian Cells (J. M. Miller and M. P. Calos, eds., 1987); PCR: The Polymerase Chain Reaction, (Mullis et al., eds., 1994); Current Protocols in Immunology (J. E. Coligan et al., eds., 1991); Short Protocols in Molecular Biology (Wiley and Sons, 1999); Immunobiology (C. A. Janeway and P. Travers, 1997); Antibodies (P. Finch, 1997); Antibodies: a practical approach (D. Catty., ed., IRL Press, 1988-1989); Monoclonal antibodies: a practical approach (P. Shepherd and C. Dean, eds., Oxford University Press, 2000); Using antibodies: a laboratory manual (E. Harlow and D. Lane (Cold Spring Harbor Laboratory Press, 1999); The Antibodies (M. Zanetti and J. D. Capra, eds., Harwood Academic Publishers, 1995); and Cancer: Principles and Practice of Oncology (V. T. DeVita et al., eds., J.B. Lippincott Company, 1993).

### DEFINITIONS

As used herein, "EphB2" (interchangeably termed "EphB2R") is defined as all mammalian species of native sequence Eph B2 receptor, including human Eph B2 receptor. The term "native sequence" in connection with EphB2 or any other polypeptide refers to a polypeptide that has the same amino acid sequence as a corresponding polypeptide derived from nature, regardless of its mode of preparation. Such native sequence polypeptide can be isolated from nature or can be produced by recombinant and/or synthetic means or any combinations thereof. The term "native sequence" specifically encompasses naturally occurring truncated or secreted forms (e.g., an extracellular domain sequence), naturally occurring variant forms (e.g., alternatively spliced forms) and naturally-occurring allelic variants. As used herein, "EphB2" refer to protein and/or polypeptide expression. Generally, the term refers to expression of both polypeptide and polynucleotide. However, the context may indicate that reference to either polypeptide or polynucleotide is intended.

The term "EphB2 extracellular domain" or "EphB2 ECD" refers to a form of EphB2 which is essentially free of transmembrane and cytoplasmic domains. Ordinarily, the ECD will have less than 1% of such transmembrane and cytoplasmic domains, and preferably, will have less than 0.5% of such domains. It will be understood that any transmembrane domain(s) identified for the polypeptides of the present invention are identified pursuant to criteria routinely employed in the art for identifying that type of hydrophobic domain. The exact boundaries of a transmembrane domain may vary but most likely by no more than about 5 amino acids at either end of the domain as initially identified. In preferred embodiments, the ECD will consist of a soluble, extracellular domain sequence of the polypeptide which is free of the transmembrane and cytoplasmic or intracellular domains (and is not membrane bound).

As used herein, the term "EphB2 ligand" includes all mammalian species of native sequence EphB2 ligand, including all mammalian species of native sequence ephrin-B1, ephrin-B2, ephrin-B3, and ephrin-A4. As used herein, "EphB2 ligand" refers to protein and/or polypeptide expression. Generally, the term refers to expression of both polypeptide and polynucleotide. However, the context may indicate that reference to either polypeptide or polynucleotide is intended.

"Cancer prognosis" generally refers to a forecast or prediction of the probable course or outcome of the cancer. As used herein, cancer prognosis includes the forecast or prediction of any one or more of the following: duration of survival of a patient susceptible to or diagnosed with a cancer, duration of recurrence-free survival, duration of progression free survival of a patient susceptible to or diagnosed with a cancer, response rate in a group of patients susceptible to or diagnosed with a cancer, duration of response in a patient or a group of patients susceptible to or diagnosed with a cancer, and/or likelihood of metastasis in a patient susceptible to or diagnosed with a cancer. As used herein, "prognostic for cancer" means providing a forecast or prediction of the probable course or outcome of the cancer. In some embodiments, "prognostic for cancer" comprises providing the forecast or prediction of (prognostic for) any one or more of the following: duration of survival of a patient susceptible to or diagnosed with a cancer, duration of recurrence-free survival, duration of progression free survival of a patient susceptible to or diagnosed with a cancer, response rate in a group of patients susceptible to or diagnosed with a cancer, duration of response in a patient or a group of patients susceptible to or diagnosed with a cancer, and/or likelihood of metastasis in a patient susceptible to or diagnosed with a cancer.

By "subject" or "patient" is meant any single subject for which therapy is desired, including humans, cattle, dogs, guinea pigs, rabbits, chickens, and so on. Also intended to be included as a subject are any subjects involved in clinical research trials not showing any clinical sign of disease, or subjects involved in epidemiological studies, or subjects used as controls.

The term "mammal" as used herein refers to any animal classified as a mammal, including humans, cows, horses, dogs and cats.

A "biological sample" (interchangeably termed "sample" or "tissue or cell sample") encompasses a variety of sample types obtained from an individual and can be used in a diagnostic or monitoring assay. The definition encompasses blood and other liquid samples of biological origin, solid tissue samples such as a biopsy specimen or tissue cultures or cells derived therefrom, and the progeny thereof. The definition also includes samples that have been manipulated in any way after their procurement, such as by treatment with reagents, solubilization, or enrichment for certain components, such as proteins or polynucleotides, or embedding in a semi-solid or solid matrix for sectioning purposes. The term "biological sample" encompasses a clinical sample, and also includes cells in culture, cell supernatants, cell lysates, serum, plasma, biological fluid, and tissue samples. The source of the biological sample may be solid tissue as from a fresh, frozen and/or preserved organ or tissue sample or biopsy or aspirate; blood or any blood constituents; bodily fluids such as cerebral spinal fluid, amniotic fluid, peritoneal fluid, or interstitial fluid; cells from any time in gestation or development of the subject. In some embodiments, the biological sample is obtained from a primary or metastatic tumor. The biological sample may contain compounds which are not naturally intermixed with the tissue in nature such as preservatives, anticoagulants, buffers, fixatives, nutrients, antibiotics, or the like.

For the purposes herein a "section" of a tissue sample is meant a single part or piece of a tissue sample, e.g. a thin slice of tissue or cells cut from a tissue sample. It is understood that multiple sections of tissue samples may be taken and subjected to analysis according to the present invention. In some embodiments, the same section of tissue sample is analyzed at both morphological and molecular levels, or is analyzed with respect to both protein and nucleic acid.

"Polynucleotide," or "nucleic acid," as used interchangeably herein, refer to polymers of nucleotides of any length, and include DNA and RNA. The nucleotides can be deoxyribonucleotides, ribonucleotides, modified nucleotides or bases, and/or their analogs, or any substrate that can be incorporated into a polymer by DNA or RNA polymerase. A polynucleotide may comprise modified nucleotides, such as methylated nucleotides and their analogs. If present, modification to the nucleotide structure may be imparted before or after assembly of the polymer. The sequence of nucleotides may be interrupted by non-nucleotide components. A polynucleotide may be further modified after polymerization, such as by conjugation with a labeling component. Other types of modifications include, for example, "caps", substitution of one or more of the naturally occurring nucleotides with an analog, internucleotide modifications such as, for example, those with uncharged linkages (e.g., methyl phosphonates, phosphotriesters, phosphoamidates, cabamates, etc.) and with charged linkages (e.g., phosphorothioates, phosphorodithioates, etc.), those containing pendant moieties, such as, for example, proteins (e.g., nucleases, toxins, antibodies, signal peptides, ply-L-lysine, etc.), those with intercalators (e.g., acridine, psoralen, etc.), those containing chelators (e.g., metals, radioactive metals, boron, oxidative metals, etc.), those containing alkylators, those with modified linkages (e.g., alpha anomeric nucleic acids, etc.), as well as unmodified forms of the polynucleotide(s). Further, any of the hydroxyl groups ordinarily present in the sugars may be replaced, for example, by phosphonate groups, phosphate groups, protected by standard protecting groups, or activated to prepare additional linkages to additional nucleotides, or may be conjugated to solid supports. The 5' and 3' terminal OH can be phosphorylated or substituted with amines or organic capping groups moieties of from 1 to 20 carbon atoms. Other hydroxyls may also be derivatized to standard protecting groups. Polynucleotides can also contain analogous forms of ribose or deoxyribose sugars that are generally known in the art, including, for example, 2'O-methyl-, 2'-O-allyl, 2'-fluoro- or 2'-azido-ribose, carbocyclic sugar analogs, alpha.-anomeric sugars, epimeric sugars such as arabinose, xyloses or lyxoses, pyranose sugars, furanose sugars, sedoheptuloses, acyclic analogs and abasic nucleoside analogs such as methyl riboside. One or more phosphodiester linkages may be replaced by alternative linking groups. These alternative linking groups include, but are not limited to, embodiments wherein phosphate is replaced by P(O)S("thioate"), P(S)S ("dithioate"), "(O)NR₂ ("amidate"), P(O)R, P(O)OR, CO or CH₂ ("formacetal"), in which each R or R' is independently H or substituted or unsubstituted alkyl (1-20 C) optionally containing an ether (--O--) linkage, aryl, alkenyl, cycloalkyl, cycloalkenyl or araldyl. Not all linkages in a polynucleotide need be identical. The preceding description applies to all polynucleotides referred to herein, including RNA and DNA.

By "gene" is meant any polynucleotide sequence or portion thereof with a functional role in encoding or transcribing a protein or regulating other gene expression. The gene may consist of all the nucleic acids responsible for encoding a functional protein or only a portion of the nucleic acids responsible for encoding or expressing a protein. The polynucleotide sequence may contain a genetic abnormality within exons, introns, initiation or termination regions, promoter sequences, other regulatory sequences or unique adjacent regions to the gene.

The word "label" when used herein refers to a compound or composition which is conjugated or fused directly or indirectly to a reagent such as a nucleic acid probe or an antibody and facilitates detection of the reagent to which it is conjugated or fused. The label may itself be detectable (e.g., radioisotope labels or fluorescent labels) or, in the case of an enzymatic label, may catalyze chemical alteration of a substrate compound or composition which is detectable.

The term "antibody" herein is used in the broadest sense and specifically covers intact monoclonal antibodies, polyclonal antibodies, multispecific antibodies (e.g. bispecific antibodies) formed from at least two intact antibodies, and antibody fragments.

The term "variable" refers to the fact that certain portions of the variable domains differ extensively in sequence among antibodies and are used in the binding and specificity of each particular antibody for its particular antigen. However, the variability is not evenly distributed throughout the variable domains of antibodies. It is concentrated in three segments called complementarity-determining regions (CDRs) or hypervariable regions both in the light-chain and the heavy-chain variable domains. The more highly conserved portions of variable domains are called the framework (FR). The variable domains of native heavy and light chains each comprise four FR regions, largely adopting a β-sheet configuration, connected by three CDRs, which form loops connecting, and in some cases forming part of, the β-sheet structure. The CDRs in each chain are held together in close proximity by the FR regions and, with the CDRs from the other chain, contribute to the formation of the antigen-binding site of antibodies (see Kabat et al., Sequences of Proteins of Immunological Interest, Fifth Edition, National Institute of Health, Bethesda, MD (1991)). The constant domains are not involved directly in binding an antibody to an antigen, but exhibit various effector functions, such as participation of the antibody in antibody-dependent cellular toxicity.

"Antibody fragments" comprise only a portion of an intact antibody, generally including an antigen binding site of the intact antibody and thus retaining the ability to bind antigen. Examples of antibody fragments encompassed by the present definition include: (i) the Fab fragment, having VL, CL, VH and CH1 domains; (ii) the Fab' fragment, which is a Fab fragment having one or more cysteine residues at the C-terminus of the CH1 domain; (iii) the Fd fragment having VH and CH1 domains; (iv) the Fd' fragment having VH and CH1 domains and one or more cysteine residues at the C-terminus of the CH1 domain; (v) the Fv fragment having the VL and VH domains of a single arm of an antibody; (vi) the dAb fragment (Ward et al., Nature 341, 544-546 (1989)) which consists of a VH domain; (vii) isolated CDR regions; (viii) F(ab')2 fragments, a bivalent fragment including two Fab' fragments linked by a disulphide bridge at the hinge region; (ix) single chain antibody molecules (e.g. single chain Fv; scFv) (Bird et al., Science 242:423-426 (1988); and Huston et al., PNAS (USA) 85:5879-5883 (1988)); (x) "diabodies" with two antigen binding sites, comprising a heavy chain variable domain (VH) connected to a light chain variable domain (VL) in the same polypeptide chain (see, e.g., EP 404,097; WO 93/11161; and Hollinger et al., Proc. Natl. Acad. Sci. USA, 90:6444-6448 (1993)); (xi) "linear antibodies" comprising a pair of tandem Fd segments (VH-CH1-VH-CH1) which, together with complementary light chain polypeptides, form a pair of antigen binding regions (Zapata et al. Protein Eng. 8(10):1057-1062 (1995); and US Patent No. 5,641,870).

The term "monoclonal antibody" as used herein refers to an antibody obtained from a population of substantially homogeneous antibodies, i.e., the individual antibodies comprising the population are identical except for possible naturally occurring mutations that may be present in minor amounts. Monoclonal antibodies are highly specific, being directed against a single antigen. Furthermore, in contrast to polyclonal antibody preparations that typically include different antibodies directed against different determinants (epitopes), each monoclonal antibody is directed against a single determinant on the antigen. The modifier "monoclonal" is not to be construed as requiring production of the antibody by any particular method. For example, the monoclonal antibodies to be used in accordance with the present invention may be made by the hybridoma method first described by Kohler et al., Nature 256:495 (1975), or may be made by recombinant DNA methods (see, e.g., U.S. Patent No. 4,816,567). The "monoclonal antibodies" may also be isolated from phage antibody libraries using the techniques described in Clackson et al., Nature 352:624-628 (1991) or Marks et al., J. Mol. Biol. 222:581-597 (1991), for example.

The monoclonal antibodies herein specifically include "chimeric" antibodies in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (U.S. Patent No. 4,816,567; and Morrison et al., Proc. Natl. Acad. Sci. USA 81:6851-6855 (1984)).

"Humanized" forms of non-human (e.g., murine) antibodies are chimeric antibodies that contain minimal sequence derived from non-human immunoglobulin. For the most part, humanized antibodies are human immunoglobulins (recipient antibody) in which residues from a hypervariable region of the recipient are replaced by residues from a hypervariable region of a non-human species (donor antibody) such as mouse, rat, rabbit or nonhuman primate having the desired specificity, affinity, and capacity. In some instances, framework region (FR) residues of the human immunoglobulin are replaced by corresponding non-human residues. Furthermore, humanized antibodies may comprise residues that are not found in the recipient antibody or in the donor antibody. These modifications are made to further refine antibody performance. In general, the humanized antibody will comprise substantially all of at least one, and typically two, variable domains, in which all or substantially all of the hypervariable loops correspond to those of a non-human immunoglobulin and all or substantially all of the FRs are those of a human immunoglobulin sequence. The humanized antibody optionally will also comprise at least a portion of an immunoglobulin constant region (Fc), typically that of a human immunoglobulin. For further details, see Jones et al., Nature 321:522-525 (1986); Riechmann et al., Nature 332:323-329 (1988); and Presta, Curr. Op. Struct. Biol. 2:593-596 (1992).

The term "hypervariable region", "HVR", or "HV", when used herein refers to the regions of an antibody variable domain which are hypervariable in sequence and/or form structurally defined loops. Generally, antibodies comprise six hypervariable regions; three in the VH (H1, H2, H3), and three in the VL (L1, L2, L3). A number of hypervariable region delineations are in use and are encompassed herein. The Kabat Complementarity Determining Regions (CDRs) are based on sequence variability and are the most commonly used (Kabat et al., Sequences of Proteins of Immunological Interest, 5th Ed. Public Health Service, National Institutes of Health, Bethesda, MD. (1991)). Chothia refers instead to the location of the structural loops (Chothia and Lesk J. Mol. Biol. 196:901-917 (1987)). The AbM hypervariable regions represent a compromise between the Kabat CDRs and Chothia structural loops, and are used by Oxford Molecular's AbM antibody modeling software. The "contact" hypervariable regions are based on an analysis of the available complex crystal structures.

A "human antibody" is one which possesses an amino acid sequence which corresponds to that of an antibody produced by a human and/or has been made using any of the techniques for making human antibodies as disclosed herein. This definition of a human antibody specifically excludes a humanized antibody comprising non-human antigen-binding residues. Human antibodies can be produced using various techniques known in the art. In one embodiment, the human antibody is selected from a phage library, where that phage library expresses human antibodies (Vaughan et al. Nature Biotechnology 14:309-314 (1996): Sheets et al. PNAS (USA) 95:6157-6162 (1998)); Hoogenboom and Winter, J. Mol. Biol., 227:381 (1991); Marks et al., J. Mol. Biol., 222:581 (1991)). Human antibodies can also be made by introducing human immunoglobulin loci into transgenic animals, e.g., mice in which the endogenous immunoglobulin genes have been partially or completely inactivated. Upon challenge, human antibody production is observed, which closely resembles that seen in humans in all respects, including gene rearrangement, assembly, and antibody repertoire. This approach is described, for example, in U.S. Patent Nos. 5,545,807; 5,545,806; 5,569,825; 5,625,126; 5,633,425; 5,661,016, and in the following scientific publications: Marks et al., Bio/Technology 10: 779-783 (1992); Lonberg et al., Nature 368: 856-859 (1994); Morrison, Nature 368:812-13 (1994); Fishwild et al., Nature Biotechnology 14: 845-51 (1996); Neuberger, Nature Biotechnology 14: 826 (1996); Lonberg and Huszar, Intern. Rev. Immunol. 13:65-93 (1995). Alternatively, the human antibody may be prepared via immortalization of human B lymphocytes producing an antibody directed against a target antigen (such B lymphocytes may be recovered from an individual or may have been immunized in vitro). See, e.g., Cole et al., Monoclonal Antibodies and Cancer Therapy, Alan R. Liss, p. 77 (1985); Boerner et al., J. Immunol., 147 (1):86-95 (1991); and US Pat No. 5,750,373.

An "affinity matured" antibody is one with one or more alterations in one or more CDRs thereof which result an improvement in the affinity of the antibody for antigen, compared to a parent antibody which does not possess those alteration(s). Preferred affinity matured antibodies will have nanomolar or even picomolar affinities for the target antigen. Affinity matured antibodies are produced by procedures known in the art. Marks et al. Bio/Technology 10:779-783 (1992) describes affinity maturation by VH and VL domain shuffling. Random mutagenesis of CDR and/or framework residues is described by: Barbas et al. Proc Nat. Acad. Sci, USA 91:3809-3813 (1994); Schier et al. Gene 169:147-155 (1995); Yelton et al. J. Immunol. 155:1994-2004 (1995); Jackson et al., J. Immunol. 154(7):3310-9 (1995); and Hawkins et al, J. Mol. Biol. 226:889-896 (1992).

An antibody "which binds" an antigen of interest is one capable of binding that antigen with sufficient affinity and/or avidity such that the antibody is useful as a prognostic and/or detection (such as diagnostic) and/or therapeutic agent for the antigen. In some embodiments, the antibody "which binds" an antigen of interest specifically or preferentially binds the antigen of interest. In some embodiments, the antibody "which binds" an antigen of interest exclusively binds the antigen of interest.

An "isolated" antibody is one which has been identified and separated and/or recovered from a component of its natural environment. Contaminant components of its natural environment are materials which would interfere with diagnostic or therapeutic uses for the antagonist or antibody, and may include enzymes, hormones, and other proteinaceous or nonproteinaceous solutes. In preferred embodiments, the antibody will be purified (1) to greater than 95% by weight of antibody as determined by the Lowry method, and most preferably more than 99% by weight, (2) to a degree sufficient to obtain at least 15 residues of N-terminal or internal amino acid sequence by use of a spinning cup sequenator, or (3) to homogeneity by SDS-PAGE under reducing or nonreducing conditions using Coomassie blue or, preferably, silver stain. Isolated antibody includes the antibody in situ within recombinant cells since at least one component of the antibody's natural environment will not be present. Ordinarily, however, isolated antibody will be prepared by at least one purification step.

A polypeptide "variant" means a polypeptide having at least about 80% amino acid sequence identity with the native sequence polypeptide. Such variants include, for instance, polypeptides wherein one or more amino acid residues are added, or deleted, at the N- or C-terminus of the polypeptide. Ordinarily, a variant will have at least about 80%, 81%, 82%, 83%, 84%, 85%, 86%, 87%, 88%, 89%, 90%, 91%, 92%, 93%, 94%, 95%, 96%, 97%, 98%, or 99% amino acid identity with the native sequence polypeptide.

A polynucleotide "variant" means a polynucleotide having at least about 80% polynucleotide sequence identity with the native sequence polynucleotide. Such variants include, for instance, polynucleotides wherein one or more nucleotides are added, or deleted, at the 5' or 3' end of the polynucleotide. Ordinarily, a variant will have at least about 80% sequence identity, more preferably at least about 81%, 82%, 83%, 84%, 85%, 86%, 87%, 88%, 89%, 90%, 91%, 92%, 93%, 94%, 95%, 96%, 97%, 98%, or 99% sequence identity with the native sequence polynucleotide.

A polypeptide "fragment" (also called a "region") is a polypeptide comprising an amino acid sequence that has at least about 5, 10, 15, 20, 25, 30, 35, 40, 45, 50, 55, 60, 65, 70, 75, 80, 85, 90, 95, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, 800, 810, 820, 830, 840, 850, 860, 870, 880, 890, 900, 910, 920, 930, 940, 950, or more contiguous amino acids of a polypeptide sequence.

A polynucleotide "fragment" (also called a "region") is a polynucleotide comprising a polynucleotide sequence that has at least about 20, 30, 40, 50, 60, 70, 80, 90, 100, 250, 500, 750, 1000, 1100, 1200, 1300, 1400, 1500, 1600, 1700, 1800, 1900, 2000, 2100, 2200, 2300, 3400, 2500, 2600, 2700, 2800, 2900, 3000, 3100, 3200, 3300, 3400, 3500, 3600, 3700, 3800, 3900, 4000, 4100, 4200, 4300, 4400, 4500, or more contiguous nucleotides of a polynucleotide sequence.

The terms "cancer", "cancerous", or "malignant" refer to or describe the physiological condition in mammals that is typically characterized by unregulated cell growth. Examples of cancer include but are not limited to, carcinoma, blastoma, and sarcoma. More particular examples of such cancers include colorectal cancer, renal cancer, small-cell lung cancer, non-small cell lung cancer, melanoma, and breast cancer.

The term "therapeutically effective amount" refers to an amount of a drug effective to treat a disease or disorder in a mammal. In the case of cancer, the therapeutically effective amount of the drug may reduce the number of cancer cells; reduce the tumor size; inhibit (i.e., slow to some extent and preferably stop) cancer cell infiltration into peripheral organs; inhibit (i.e., slow to some extent and preferably stop) tumor metastasis; inhibit, to some extent, tumor growth; and/or relieve to some extent one or more of the symptoms associated with the disorder. To the extent the drug may prevent growth and/or kill existing cancer cells, it may be cytostatic and/or cytotoxic. For cancer therapy, efficacy in vivo can, for example, be measured by assessing the duration of survival, time to disease progression (TTP), the response rates (RR), duration of response, and/or quality of life. In the case of colon adenoma, the therapeutically effective amount of the drug may, for example, reduce the number of adenoma cells; reduce the adenoma size; reduce adenoma number; inhibit, to some extent, adenoma growth; and/or relieve to some extent one or more of the symptoms associated with the disorder.

As used herein, "treatment" is an approach for obtaining beneficial or desired clinical results. For purposes of this invention, beneficial or desired clinical results include, but are not limited to, one or more of the following: reduce the number of cancer cells; reduce the tumor size; inhibit (i.e., slow to some extent and/or stop) cancer cell infiltration into peripheral organs; inhibit (i.e., slow to some extent and/or stop) tumor metastasis; inhibit, to some extent, tumor growth; and/or relieve to some extent one or more of the symptoms associated with the disorder, shrinking the size of the tumor, decreasing symptoms resulting from the disease, increasing the quality of life of those suffering from the disease, decreasing the dose of other medications required to treat the disease, delaying the progression of the disease, and/or prolonging survival of patients.

"Isolated," when used to describe the various polypeptides or proteins disclosed herein, means polypeptide or protein that has been identified and separated and/or recovered from a component of its natural environment. Contaminant components of its natural environment are materials that would typically interfere with diagnostic or therapeutic uses for the polypeptide or protein, and may include enzymes, hormones, and other proteinaceous or non-proteinaceous solutes. In preferred embodiments, the polypeptide or protein will be purified (1) to a degree sufficient to obtain at least 15 residues of N-terminal or internal amino acid sequence by use of a spinning cup sequenator, or (2) to homogeneity by SDS-PAGE under non-reducing or reducing conditions using Coomassie blue or, preferably, silver stain, or (3) to homogeneity by mass spectroscopic or peptide mapping techniques. Isolated material includes polypeptide or protein in situ within recombinant cells, since at least one component of its natural environment will not be present. Ordinarily, however, isolated polypeptide or protein will be prepared by at least one purification step.

The terms "polypeptide", "oligopeptide", "peptide" and "protein" are used interchangeably herein to refer to polymers of amino acids of any length. The polymer may be linear or branched, it may comprise modified amino acids, and it may be interrupted by non-amino acids. The terms also encompass an amino acid polymer that has been modified naturally or by intervention; for example, disulfide bond formation, glycosylation, lipidation, acetylation, phosphorylation, or any other manipulation or modification, such as conjugation with a labeling component. Also included within the definition are, for example, polypeptides containing one or more analogs of an amino acid (including, for example, unnatural amino acids, etc.), as well as other modifications known in the art.

"Percent (%) amino acid sequence identity" with respect to the sequences identified herein is defined as the percentage of amino acid residues in a candidate sequence that are identical with the amino acid residues in the reference sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art can determine appropriate parameters for measuring alignment, including assigning algorithms needed to achieve maximal alignment over the full-length sequences being compared. For purposes herein, percent amino acid identity values can be obtained using the sequence comparison computer program, ALIGN-2, which was authored by Genentech, Inc. and the source code of which has been filed with user documentation in the US Copyright Office, Washington, DC, 20559, registered under the US Copyright Registration No. TXU510087. The ALIGN-2 program is publicly available through Genentech, Inc., South San Francisco, CA. All sequence comparison parameters are set by the ALIGN-2 program and do not vary.

"Percent (%) nucleic acid sequence identity" is defined as the percentage of nucleotides in a candidate sequence that are identical with the nucleotides in the reference nucleic acid sequence of interest, after aligning the sequences and introducing gaps, if necessary, to achieve the maximum percent sequence identity. Alignment for purposes of determining percent nucleic acid sequence identity can be achieved in various ways that are within the skill in the art, for instance, using publicly available computer software such as BLAST, BLAST-2, ALIGN or Megalign (DNASTAR) software. For purposes herein, % nucleic acid sequence identity values are generated using the sequence comparison computer program ALIGN-2. All sequence comparison parameters are set by the ALIGN-2 program and do not vary.

"Stringency" of hybridization reactions is readily determinable by one of ordinary skill in the art, and generally is an empirical calculation dependent upon probe length, washing temperature, and salt concentration. In general, longer probes require higher temperatures for proper annealing, while shorter probes need lower temperatures. Hybridization generally depends on the ability of denatured DNA to re-anneal when complementary strands are present in an environment below their melting temperature. The higher the degree of desired identity between the probe and hybridizable sequence, the higher the relative temperature which can be used. As a result, it follows that higher relative temperatures would tend to make the reaction conditions more stringent, while lower temperatures less so. For additional details and explanation of stringency of hybridization reactions, see Ausubel et al., Current Protocols in Molecular Biology, Wiley Interscience Publishers, (2003).

"High stringency conditions", as defined herein, are identified by those that: (1) employ low ionic strength and high temperature for washing; 0.015 M sodium chloride/0.0015 M sodium citrate/0.1 % sodium dodecyl sulfate at 50°C; (2) employ during hybridization a denaturing agent; 50% (v/v) formamide with 0.1% bovine serum albumin/0.1 % Ficoll/0.1 % polyvinylpyrrolidone/50mM sodium phosphate buffer at pH 6.5 with 750 mM sodium chloride, 75 mM sodium citrate at 42°C; or (3) employ 50% formamide, 5 x SSC (0.75 M NaCl, 0.075 M sodium citrate), 50 mM sodium phosphate (pH 6.8), 0.1% sodium pyrophosphate, 5 x Denhardt's solution, sonicated salmon sperm DNA (50 µg/ml), 0.1% SDS, and 10% dextran sulfate at 42°C, with washes at 42°C in 0.2 x SSC (sodium chloride/sodium citrate) and 50% formamide at 55°C, followed by a high-stringency wash consisting of 0.1 x SSC containing EDTA at 55°C.

"Moderately stringent conditions" may be identified as described by Sambrook et al., Molecular Cloning: A Laboratory Manual, New York: Cold Spring Harbor Press, 1989, and include overnight incubation at 37°C in a solution comprising: 20% formamide, 5 x SSC (150 mM NaCl, 15 mM trisodium citrate), 50 mM sodium phosphate (pH 7.6), 5 x Denhardt's solution, 10% dextran sulfate, and 20 mg/ml denatured sheared salmon sperm DNA, followed by washing the filters in 1 x SSC at about 37-50°C. The skilled artisan will recognize how to adjust the temperature, ionic strength, etc. as necessary to accommodate factors such as probe length and the like.

"Detection" includes any means of detecting, including direct and indirect detection. For example, "detectably fewer" products may be observed directly or indirectly, and the term indicates any reduction (including no products). Similarly, "detectably more" product means any increase, whether observed directly or indirectly.

### "Comprising" means including.

As used herein, the singular forms "a", "and", and "the" include plural referents unless the context clearly dictates otherwise. Thus, for example, reference to "a genetic alteration" includes a plurality of such alterations and reference to "a probe" includes reference to one or more probes.

The term "cytotoxic agent" as used herein refers to a substance that inhibits or prevents the function of cells and/or causes destruction of cells. The term is intended to include radioactive isotopes (e.g., At²¹¹, I¹³¹, I¹²⁵, Y⁹⁰, Re¹⁸⁶, Re¹⁸⁸, Sm¹⁵³, Bi²¹², P³² and radioactive isotopes of Lu), chemotherapeutic agents e.g. methotrexate, adriamicin, vinca alkaloids (vincristine, vinblastine, etoposide), doxorubicin, melphalan, mitomycin C, chlorambucil, daunorubicin or other intercalating agents, enzymes and fragments thereof such as nucleolytic enzymes, antibiotics, and toxins such as small molecule toxins or enzymatically active toxins of bacterial, fungal, plant or animal origin, including fragments and/or variants thereof, and the various antitumor or anticancer agents disclosed below. Other cytotoxic agents are described below. A tumoricidal agent causes destruction of tumor cells.

A "chemotherapeutic agent" is a chemical compound useful in the treatment of cancer. Examples of chemotherapeutic agents include alkylating agents such as thiotepa and CYTOXAN® cyclosphosphamide; alkyl sulfonates such as busulfan, improsulfan and piposulfan; aziridines such as benzodopa, carboquone, meturedopa, and uredopa; ethylenimines and methylamelamines including altretamine, triethylenemelamine, trietylenephosphoramide, triethiylenethiophosphoramide and trimethylolomelamine; acetogenins (especially bullatacin and bullatacinone); delta-9-tetrahydrocannabinol (dronabinol, MARINOL®); beta-lapachone; lapachol; colchicines; betulinic acid; a camptothecin (including the synthetic analogue topotecan (HYCAMTIN®), CPT-11 (irinotecan, CAMPTOSAR®), acetylcamptothecin, scopolectin, and 9-aminocamptothecin); bryostatin; callystatin; CC-1065 (including its adozelesin, carzelesin and bizelesin synthetic analogues); podophyllotoxin; podophyllinic acid; teniposide; cryptophycins (particularly cryptophycin 1 and cryptophycin 8); dolastatin; duocarmycin (including the synthetic analogues, KW-2189 and CB1-TM1); eleutherobin; pancratistatin; a sarcodictyin; spongistatin; nitrogen mustards such as chlorambucil, chlornaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, and ranimnustine; antibiotics such as the enediyne antibiotics (e. g., calicheamicin, especially calicheamicin gamma1I and calicheamicin omegaI1 (see, e.g., Agnew, Chem Intl. Ed. Engl., 33: 183-186 (1994)); dynemicin, including dynemicin A; an esperamicin; as well as neocarzinostatin chromophore and related chromoprotein enediyne antiobiotic chromophores), aclacinomysins, actinomycin, authramycin, azaserine, bleomycins, cactinomycin, carabicin, carminomycin, carzinophilin, chromomycinis, dactinomycin, daunorubicin, detorubicin, 6-diazo-5-oxo-L-norleucine, ADRIAMYCIN® doxorubicin (including morpholino-doxorubicin, cyanomorpholino-doxorubicin, 2-pyrrolino-doxorubicin and deoxydoxorubicin), epirubicin, esorubicin, idarubicin, marcellomycin, mitomycins such as mitomycin C, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; antimetabolites such as methotrexate and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti- adrenals such as aminoglutethimide, mitotane, trilostane; folic acid replenisher such as frolinic acid; aceglatone; aldophosphamide glycoside; aminolevulinic acid; eniluracil; amsacrine; bestrabucil; bisantrene; edatraxate; defofamine; demecolcine; diaziquone; elfornithine; elliptinium acetate; an epothilone; etoglucid; gallium nitrate; hydroxyurea; lentinan; lonidainine; maytansinoids such as maytansine and ansamitocins; mitoguazone; mitoxantrone; mopidanmol; nitraerine; pentostatin; phenamet; pirarubicin; losoxantrone; 2-ethylhydrazide; procarbazine; PSK® polysaccharide complex (JHS Natural Products, Eugene, OR); razoxane; rhizoxin; sizofiran; spirogermanium; tenuazonic acid; triaziquone; 2,2',2"-trichlorotriethylamine; trichothecenes (especially T-2 toxin, verracurin A, roridin A and anguidine); urethan; vindesine (ELDISINE®, FILDESIN®); dacarbazine; mannomustine; mitobronitol; mitolactol; pipobroman; gacytosine; arabinoside ("Ara-C"); thiotepa; taxoids, e.g., TAXOL® paclitaxel (Bristol-Myers Squibb Oncology, Princeton, N.J.), ABRAXANETM Cremophor-free, albumin-engineered nanoparticle formulation of paclitaxel (American Pharmaceutical Partners, Schaumberg, Illinois), and TAXOTERE® doxetaxel (Rhône-Poulenc Rorer, Antony, France); chloranbucil; gemcitabine (GEMZAR®); 6-thioguanine; mercaptopurine; methotrexate; platinum analogs such as cisplatin and carboplatin; vinblastine (VELBAN®); platinum; etoposide (VP-16); ifosfamide; mitoxantrone; vincristine (ONCOVIN®); oxaliplatin; leucovovin; vinorelbine (NAVELBINE®); novantrone; edatrexate; daunomycin; aminopterin; ibandronate; topoisomerase inhibitor RFS 2000; difluorometlhylornithine (DMFO); retinoids such as retinoic acid; capecitabine (XELODA®); pharmaceutically acceptable salts, acids or derivatives of any of the above; as well as combinations of two or more of the above such as CHOP, an abbreviation for a combined therapy of cyclophosphamide, doxorubicin, vincristine, and prednisolone, and FOLFOX, an abbreviation for a treatment regimen with oxaliplatin (ELOXATINTM) combined with 5-FU and leucovovin.

Also included in this definition are anti-hormonal agents that act to regulate, reduce, block, or inhibit the effects of hormones that can promote the growth of cancer, and are often in the form of systemic, or whole-body treatment. They may be hormones themselves. Examples include anti-estrogens and selective estrogen receptor modulators (SERMs), including, for example, tamoxifen (including NOLVADEX® tamoxifen), EVISTA® raloxifene, droloxifene, 4-hydroxytamoxifen, trioxifene, keoxifene, LY117018, onapristone, and FARESTON® toremifene; anti-progesterones; estrogen receptor down-regulators (ERDs); agents that function to suppress or shut down the ovaries, for example, leutinizing hormone-releasing hormone (LHRH) agonists such as LUPRON® and ELIGARD® leuprolide acetate, goserelin acetate, buserelin acetate and tripterelin; other anti-androgens such as flutamide, nilutamide and bicalutamide; and aromatase inhibitors that inhibit the enzyme aromatase, which regulates estrogen production in the adrenal glands, such as, for example, 4(5)-imidazoles, aminoglutethimide, MEGASE® megestrol acetate, AROMASIN® exemestane, formestanie, fadrozole, RIVISOR® vorozole, FEMARA® letrozole, and ARIMIDEX® anastrozole. In addition, such definition of chemotherapeutic agents includes bisphosphonates such as clodronate (for example, BONEFOS® or OSTAC®), DIDROCAL® etidronate, NE-58095, ZOMETA® zoledronic acid/zoledronate, FOSAMAX® alendronate, AREDIA® pamidronate, SKELID® tiludronate, or ACTONEL® risedronate; as well as troxacitabine (a 1,3-dioxolane nucleoside cytosine analog); antisense oligonucleotides, particularly those that inhibit expression of genes in signaling pathways implicated in abherant cell proliferation, such as, for example, PKC-alpha, Raf, H-Ras, and epidermal growth factor receptor (EGF-R); vaccines such as THERATOPE® vaccine and gene therapy vaccines, for example, ALLOVECTIN® vaccine, LEUVECTIN® vaccine, and VAXID® vaccine; LURTOTECAN® topoisomerase 1 inhibitor; ABARELIX® rmRH; lapatinib ditosylate (an ErbB-2 and EGFR dual tyrosine kinase small-molecule inhibitor also known as GW572016); and pharmaceutically acceptable salts, acids or derivatives of any of the above.

A "growth inhibitory agent" when used herein refers to a compound or composition which inhibits growth of a cell (such as a cell expressing EphB2) either in vitro or in vivo. Thus, the growth inhibitory agent may be one which significantly reduces the percentage of cells (such as a cell expressing EphB2) in S phase. Examples of growth inhibitory agents include agents that block cell cycle progression (at a place other than S phase), such as agents that induce G1 arrest and M-phase arrest. Classical M-phase blockers include the vincas (vincristine and vinblastine), taxanes, and topoisomerase II inhibitors such as doxorubicin, epirubicin, daunorubicin, etoposide, and bleomycin. Those agents that arrest G1 also spill over into S-phase arrest, for example, DNA alkylating agents such as tamoxifen, prednisone, dacarbazine, mechlorethamine, cisplatin, methotrexate, 5-fluorouracil, and ara-C. Further information can be found in The Molecular Basis of Cancer, Mendelsohn and Israel, eds., Chapter 1, entitled "Cell cycle regulation, oncogenes, and antineoplastic drugs" by Murakami et al. (WB Saunders: Philadelphia, 1995), especially p. 13. The taxanes (paclitaxel and docetaxel) are anticancer drugs both derived from the yew tree. Docetaxel (TAXOTERE®, Rhone-Poulenc Rorer), derived from the European yew, is a semisynthetic analogue of paclitaxel (TAXOL®, Bristol-Myers Squibb). Paclitaxel and docetaxel promote the assembly of microtubules from tubulin dimers and stabilize microtubules by preventing depolymerization, which results in the inhibition of mitosis in cells.

"Doxorubicin" is an anthracycline antibiotic. The full chemical name of doxorubicin is (8S-cis)-10-[(3-amino-2,3,6-trideoxy-a-L-lyxo-hexapyranosyl)oxy]-7,8,9,10-tetrahydro-6,8,11-trihydroxy-8-(hydroxyacetyl)-1-methoxy-5,12-naphthacenedione.

### METHODS OF THE INVENTION

### Methods comprising detection of EphB2

In one aspect, the invention provides methods comprising the detection of EphB2 polypeptide(s) and/or polynucleotide(s) in a colorectal tissue or cell sample from a patient having colorectal cancer, wherein the detection is predictive or indicative of cancer prognosis in the patient. Applicants surprisingly found that the expression of EphB2 is predictive of cancer prognosis. Therefore, the disclosed methods can provide for convenient, efficient, and potentially cost-effective means to obtain data and information useful in assessing future course of the disorder, including selection of appropriate therapies for treating patients.

Accordingly, in one aspect, the invention provides methods for prognostic evaluation of a patient having colorectal cancer, the methods comprising: (a) comparing expression of EphB2 in a cancer-derived biological sample from the patient with expression of EphB2 in a control sample (or control reference value); and (b) predicting cancer prognosis of the patient based on the comparison in (a), wherein EphB2 expression is prognostic for colorectal cancer in the patient. In some embodiments, increased EphB2 expression in the patient sample relative to the control sample (or control reference value) is prognostic for cancer in the subject. In some embodiments, decreased EphB2 expression in the patient sample relative to the control sample (or control reference value) is prognostic for cancer in the subject.

In another aspect, the invention provides methods for prognostic evaluation of a patient having colorectal cancer, the methods comprising: (a) obtaining a patient colorectal tissue or cell sample; and (b) detecting EphB2 expression in the sample, wherein EphB2 expression is prognostic for colorectal cancer in the patient. In some embodiments, increased EphB2 expression in the patient biological sample relative to a control sample (or a control reference value) is prognostic for cancer in the subject. In some embodiments, decreased EphB2 expression in the patient sample relative to the control sample (or control reference value) is prognostic for cancer in the subject.

The cancer is colorectal cancer. In other embodiments, the cancer is colon cancer potentially curable by surgery. In other embodiments, the cancer is inoperable colon cancer. In some embodiments, the cancer is metastatic colon cancer.

Generally, as used herein, cancer prognosis encompasses the forecast or prediction of any one or more of the following: duration of survival of a patient susceptible to or diagnosed with a cancer, duration of recurrence-free survival, duration of progression free survival of a patient susceptible to or diagnosed with a cancer, response rate in a group of patients susceptible to or diagnosed with a cancer, duration of response in a patient or a group of patients susceptible to or diagnosed with a cancer, and/or likelihood of metastasis in a patient susceptible to or diagnosed with a cancer. Duration of survival is defined as the time from first administration of the treatment to death. Duration of survival can also be measured by stratified hazard ratio (HR) of the treatment group versus control group, which represents the risk of death for a patient during the treatment. Duration of recurrence-free survival is defined as the time from treatment to recurrence of cancer. Time to disease progression is defined as the time from administration of treatment until disease progression. Response rate is defined as the percentage of treated patients who responded to the treatment. Duration of response is defined as the time from the initial response to treatment to disease progression. In some embodiments, duration of survival and duration of progression free survival are predicted. In some embodiments, the prognosis defines outcome in the absence of adjuvant therapy.

In some embodiment, prognostic for cancer comprises providing the forecast or prediction of any one or more of the following: duration of survival of a patient susceptible to or diagnosed with a cancer, duration of recurrence-free survival, duration of progression free survival of a patient susceptible to or diagnosed with a cancer, response rate in a group of patients susceptible to or diagnosed with a cancer, duration of response in a patient or a group of patients susceptible to or diagnosed with a cancer, and/or likelihood of metastasis in a patient susceptible to or diagnosed with a cancer. In some embodiments, duration of survival is forecast or predicted to be increased. In some embodiment, duration of survival is forecast or predicted to be decreased. In some embodiments, duration of recurrence-free survival is forecast or predicted to be increased. In some embodiment, duration of recurrence-free survival is forecast or predicted to be decreased. In some embodiments, response rate is forecast or predicted to be increased. In some embodiments, response rate is forecast or predicted to be decreased. In some embodiments, duration of response is predicted or forecast to be increased. In some embodiments, duration of response is predicted or forecast to be decreased. In some embodiments, likelihood of metastasis is predicted or forecast to be increased. In some embodiments, likelihood of metastasis is predicted or forecast to be decreased. In some embodiments, increased EphB2 expression in the patient biological sample relative to the control sample is prognostic for longer duration of survival. In some embodiments, increased EphB2 expression in the patient biological sample relative to the control sample is prognostic for longer recurrence-free survival.

It is understood that other prognostic and/or diagnostic factors may be considered in addition to and/or in combination (conjunction) with EphB2 expression. Exemplary prognostic and/or diagnostic factors include age and/or sex of the patient, TNM stage (TNM Classification of Malignant Tumours, Sixth Ed.), tumor grade, presence or absence of lymphatic invasion, presence or absence of blood vessel invasion, and site. For colorectal cancer, the site may be one or more of the caecum, ascending and transverse colon, descending and sigmoid colon, or rectum. See also Nicum et al., Acta Oncol 42:263-275 (2003); Kinzler, KW, and Vogelstein, B. Colorectal Tumors, in Kinzeler KW, Vogelstein B, eds. The Genetic Basis of Human Cancer: McGraw-Hill 1999, p. 565-87. Malignant involvement of surgical circumferential resection margin is a strong prognostic factor for patient survival in rectal cancer. See id.; Birbeck et al. Annals Surg 235:449-457 (2002). Additional factors that may be generally considered are known in the art and include prior course of patient treatment including surgery, radiation treatment, chemotherapy, and treatment with other drugs.

EphB2 is well known in the art. Exemplary EphB2 polynucleotide and amino acid sequences are depicted in Figures 1-6. Exemplary EphB2 sequences are further disclosed in, e.g., Annu. Rev. Neurosci. 21:309-345 (1998), Int. Rev. Cytol. 196:177-244 (2000)); WO2003042661; WO200053216; WO2004065576; WO2004020583; WO2003004529 (Page 128-132); and WO200053216. As used herein, EphB2 encompasses naturally occurring truncated or secreted forms (e.g., an extracellular domain sequence), naturally occurring variant forms (e.g., alternatively spliced forms) and naturally-occurring allelic variants of the full length polypeptides.

The methods of the invention may also detect EphB2 variant polypeptide(s) and/or polynucleotides. In some embodiments, the variant polypeptide has at least about 80%, 81%, 82%, 83%, 84%, 85%, 86%, 87%, 88%, 89%, 90%, 91%, 92%, 93%, 94%, 95%, 96%, 97%, 98%, or 99% amino acid sequence identity with a native sequence polypeptide, in some embodiments, the polypeptide shown in Figures 1, 3, and/or 5. In other embodiments, the variant polypeptide is encoded by a polynucleotide sequence which hybridizes under stringent conditions with an EphB2 polynucleotide sequence, in some embodiments, the polynucleotide sequences shown in figures 2, 4, and/or 6. In some embodiments, the variant polynucleotide has at least about 80%, 81%, 82%, 83%, 84%, 85%, 86%, 87%, 88%, 89%, 90%, 91%, 92%, 93%, 94%, 95%, 96%, 97%, 98%, or 99% polynucleotide sequence identity with a native sequence polynucleotide, in some embodiments, the polynucleotide shown in Figures 2, 4, and/or 6. In other embodiments, the variant polynucleotide comprises a polynucleotide sequence which hybridizes under stringent conditions with an EphB2 polynucleotide sequence, in some embodiments, the polynucleotide sequences shown in figures 1, 3, and/or 5. In some embodiments, the EphB2 variants are biologically active. EphB2 biological activities are well known in the art and include, but are not limited to, any one or more of the following: (a) bind EphB2 ligand(s) (such as ephrin-B1, ephrin-B2, ephrin-B3 and/or ephrin-A4); (b) bind EphB2 ligand and activate EphB2 ligand biological activity or downstream pathways mediated by EphB2 ligand; (c) signal in response to EphB2 ligand binding. The methods of the invention may also detect EphB2 fragments.

Detection of expression may be quantitative or qualitative. Presence and/or absence and/or level (amount) of EphB2 expression may be detected. It is understood that absence of EphB2 expression includes insignificant, or de minimus levels. The expression of EphB2 polynucleotide and/or polypeptide in the test biological sample may be higher than that observed for a control biological sample (or control reference value) (termed "over-expression). In some embodiments, EphB2 expression is at least about 2-fold, 5-fold, 10-fold, 20-fold, 30-fold, 40-fold, 50-fold, 75-fold, 100-fold, 150-fold higher, or higher in the test biological sample. In some embodiments, EphB2 polypeptide expression is determined in an immunohistochemistry ("IHC") assay to score at least 2 or higher for staining intensity. In some embodiments, EphB2 polypeptide expression is determined in an IHC assay to score at least 1 or higher or at least 3 or higher for staining intensity. The expression of EphB2 polynucleotide and/or polypeptide in the test biological sample may be lower than that observed for a control biological sample (termed "under-expression). In some embodiments, EphB2 expression is at least about 2-fold, 5-fold, 10-fold, 20-fold, 30-fold, 40-fold, 50-fold, 75-fold, 100-fold, 150-fold lower, or lower in the test biological sample.

EphB2 expression in the test biological sample (i.e., the biological sample from the patient having cancer or suspected of having cancer) may be compared to a suitable control sample, as is well known in the art. Exemplary controls include comparable normal samples (e.g., normal non-cancerous tissue or cells of the same type as present in the test biological sample), matched normal samples from the same patient, universal control samples, or a normal reference value (also termed a control reference value). As used herein, the term "control" or "control sample" encompasses a normal reference value. Methods for comparison of expression levels (such as presence or absence of or amount of expression) are known in the art, and some are described and exemplified herein.

As discussed herein, EphB2 in a biological sample can be detected by a number of methods which are well-known in the art, including but not limited to, immunohistochemical and/or Western analysis, biochemical enzymatic activity assays, *in situ* hybridization, Northern analysis and/or PCR analysis of mRNAs, and genomic Southern analysis (to examine, for example, gene deletion or amplification), as well as any one of the wide variety of assays that can be performed by gene, protein, and/or tissue array analysis. Typical protocols for evaluating the status of polynucleotides and polypeptides are found, for example in Ausubel et al. eds., 2003, and some are described and exemplified herein.

### Detection of EphB2 polypeptide

Disclosed herein are methods to detect (e.g., presence or absence of or amount of) a polypeptide(s) (e.g., EphB2) in a biological sample from a subject, such as a human subject. A variety of methods for detecting polypeptides can be employed and include, for example, immunohistochemical analysis, immunoprecipitation, Western blot analysis, molecular binding assays, ELISA, ELIFA, fluorescence activated cell sorting (FACS), mass spectroscopy, protein microarray, and the like.

In some embodiments, EphB2 in a biological sample is detected by (a) contacting the sample with an EphB2 binding agent, such as an antibody, a fragment thereof, or a protein (such as a recombinant protein) containing an EphB2 binding region; and (b) detecting the EphB2 binding agent-EphB2 polypeptide complex in the sample.

Anti-EphB2 antibodies are known in the art (e.g., catalog number AF467, R&D Systems, Minneapolis, MN), and may be generated using methods well known in the art. An anti-EphB2 antibody should exhibit any one or more of the following characteristics (assays for which are well known in the art): (a) bind to EphB2; (b) block or decrease EphB2 activation; (c) block or decrease EphB2 ligand (such as ephrins-B1, ephrins-B2, ephrins-B3 and/or ephrins-A4) activation and/or binding). An assay for EphB2 activation is described in Mao et al. Cancer Res. 64: 781-788, 2004. Additional exemplary anti-EphB2 antibodies are described herein.

The anti-EphB2 antibody may bind, preferentially bind or exclusively bind EphB2. In some embodiments, the antibody binds EphB2 and does not significantly cross-react with EphB 1R and/or EphB3R. In some embodiments, the anti-EphB2 antibody binds EphB2 and does not significantly cross react with EphB1R, EphB3R, EphB4R, EphB5R and/or EphB6R. In some embodiments, the anti-EphB2 antibody binds an EphB2 polypeptide fragment comprising at least about 5, 10, 15, 20, 25, 30, 35, 40, 45, 50, 55, 60, 65, 70, 75, 80, 85, 90, 95, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, 800, 810, 820, 830, 840, 850, 860, 870, 880, 890, 900, 910, 920, 930, 940, or more contiguous amino acids. In some embodiments, the anti-EphB2 antibody binds an EphB2 polypeptide fragment comprising at least about 5, 10, 15, 20, 25, 30, 35, 40, 45, 50, 55, 60, 65, 70, 75, 80, 85, 90, 95, 100, 110,120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, 800, 810, 820, 830, 840, 850, 860, 870, 880, 890, 900,910,920,930,940, or more contiguous amino acids shown in Figures 2, 4, or 6. In some embodiments, the anti-EphB2 antibody may bind human EphB2, murine EphB2, rodent EphB2, and/or monkey EphB2. In some embodiments, the anti-EPHB2 antibody binds human EphB2. In some embodiments, the antibody binds to EphB2 ECD. EphB2 ECDs are known in the art. It is understood that an anti-EphB2 agent (such as an anti-EphB2 antibody) may recognize one or more native sequence EphB2 polypeptide. It is routine in the art to select agents (such antibodies) that bind one or more such polypeptide and further to determine whether an agent recognizes one or more such polypeptide.

Anti-EphB2 proteins comprising an EphB2 binding region include, for example, ephrin-B1 immunoadhesins, ephrin-B2 immunoadhesin, ephrin-B3 immunoadhesin and ephrins-A4 immunoadhesin. For convenience, detection using anti-EphB2 antibodies is generally discussed herein. It is understood that anti-EphB2 agents may generally be used instead of (or in addition to) an antibody in the methods described herein.

The expression of proteins in a sample may be examined using immunohistochemistry and staining protocols. Immunohistochemical staining of tissue sections has been shown to be a reliable method of assessing or detecting presence of proteins in a sample. Immunohistochemistry ("IHC") techniques utilize an antibody to probe and visualize cellular antigens *in situ,* generally by chromogenic or fluorescent methods. For sample preparation, a tissue or cell sample from a mammal (typically a human patient) may be used. Examples of samples include, but are not limited to, cancer cells such as colon, breast, prostate, ovary, lung, stomach, pancreas, lymphoma, and leukemia cancer cells. The sample can be obtained by a variety of procedures known in the art including, but not limited to surgical excision, aspiration or biopsy. The tissue may be fresh or frozen. In one embodiment, the sample is fixed and embedded in paraffin or the like. The tissue sample may be fixed (*i.e*. preserved) by conventional methodology (See e.g., "Manual of Histological Staining Method of the Armed Forces Institute of Pathology," 3rd edition (1960) Lee G. Luna, HT (ASCP) Editor, The Blakston Division McGraw-Hill Book Company, New York; The Armed Forces Institute of Pathology Advanced Laboratory Methods in Histology and Pathology (1994) Ulreka V. Mikel, Editor, Armed Forces Institute of Pathology, American Registry of Pathology, Washington, D.C.). One of ordinary skill in the art will appreciate that the choice of a fixative is determined by the purpose for which the sample is to be histologically stained or otherwise analyzed. One of ordinary skill in the art will also appreciate that the length of fixation depends upon the size of the tissue sample and the fixative used. By way of example, neutral buffered formalin, Bouin's or paraformaldehyde, may be used to fix a sample. Generally, the sample is first fixed and is then dehydrated through an ascending series of alcohols, infiltrated and embedded with paraffin or other sectioning media so that the tissue sample may be sectioned. Alternatively, one may section the tissue and fix the sections obtained. By way of example, the tissue sample may be embedded and processed in paraffin by conventional methodology (See *e.g.,* "Manual of Histological Staining Method of the Armed Forces Institute of Pathology", *supra).* Examples of paraffin that may be used include, but are not limited to, Paraplast, Broloid, and Tissuemay. Once the tissue sample is embedded, the sample may be sectioned by a microtome or the like (See e.g., "Manual of Histological Staining Method of the Armed Forces Institute of Pathology", *supra*). By way of example for this procedure, sections may range from about three microns to about five microns in thickness. Once sectioned, the sections may be attached to slides by several standard methods. Examples of slide adhesives include, but are not limited to, silane, gelatin, poly-L-lysine and the like. By way of example, the paraffin embedded sections may be attached to positively charged slides and/or slides coated with poly-L-lysine. If paraffin has been used as the embedding material, the tissue sections are generally deparaffinized and rehydrated to water. The tissue sections may be deparaffinized by several conventional standard methodologies. For example, xylenes and a gradually descending series of alcohols may be used (See e.g., "Manual of Histological Staining Method of the Armed Forces Institute of Pathology", *supra*). Alternatively, commercially available deparaffinizing non-organic agents such as Hemo-De7 (CMS, Houston, Texas) may be used.

Optionally, subsequent to the sample preparation, a tissue section may be analyzed using IHC. IHC may be performed in combination with additional techniques such as morphological staining and/or fluorescence *in-situ* hybridization. Two general methods of IHC are available; direct and indirect assays. According to the first assay, binding of antibody to the target antigen (e.g., EphB2) is determined directly. This direct assay uses a labeled reagent, such as a fluorescent tag or an enzyme-labeled primary antibody, which can be visualized without further antibody interaction. In a typical indirect assay, unconjugated primary antibody binds to the antigen and then a labeled secondary antibody binds to the primary antibody. Where the secondary antibody is conjugated to an enzymatic label, a chromogenic or fluorogenic substrate is added to provide visualization of the antigen. Signal amplification occurs because several secondary antibodies may react with different epitopes on the primary antibody.

The primary and/or secondary antibody used for immunohistochemistry typically will be labeled with a detectable moiety. Numerous labels are available which can be generally grouped into the following categories:
(a) Radioisotopes, such as ³⁵S, ¹⁴C, ¹²⁵I, ³H, and ¹³¹I. The antibody can be labeled with the radioisotope using the techniques described in Current Protocols in Immunology, Volumes 1 and 2, Coligen et al., Ed. Wiley-Interscience, New York, New York, Pubs. (1991) for example and radioactivity can be measured using scintillation counting.
(b) Colloidal gold particles.
(c) Fluorescent labels including, but are not limited to, rare earth chelates (europium chelates), Texas Red, rhodamine, fluorescein, dansyl, Lissamine, umbelliferone, phycocrytherin, phycocyanin, or commercially available fluorophores such SPECTRUM ORANGE7 and SPECTRUM GREEN7 and/or derivatives of any one or more of the above. The fluorescent labels can be conjugated to the antibody using the techniques disclosed in *Current Protocols in Immunology, supra,* for example. Fluorescence can be quantified using a fluorimeter.
(d) Various enzyme-substrate labels are available and U.S. Patent No. 4,275,149 provides a review of some of these. The enzyme generally catalyzes a chemical alteration of the chromogenic substrate that can be measured using various techniques. For example, the enzyme may catalyze a color change in a substrate, which can be measured spectrophotometrically. Alternatively, the enzyme may alter the fluorescence or chemiluminescence of the substrate. Techniques for quantifying a change in fluorescence are described above. The chemiluminescent substrate becomes electronically excited by a chemical reaction and may then emit light which can be measured (using a chemiluminometer, for example) or donates energy to a fluorescent acceptor. Examples of enzymatic labels include luciferases (*e.g.*, firefly luciferase and bacterial luciferase; U.S. Patent No. 4,737,456), luciferin, 2,3-dihydrophthalazinediones, malate dehydrogenase, urease, peroxidase such as horseradish peroxidase (HRPO), alkaline phosphatase, β-galactosidase, glucoamylase, lysozyme, saccharide oxidases (*e.g.*, glucose oxidase, galactose oxidase, and glucose-6-phosphate dehydrogenase), heterocyclic oxidases (such as uricase and xanthine oxidase), lactoperoxidase, microperoxidase, and the like. Techniques for conjugating enzymes to antibodies are described in O'Sullivan et al., Methods for the Preparation of Enzyme-Antibody Conjugates for use in Enzyme Immunoassay, in Methods in Enzym. (ed. J. Langone & H. Van Vunakis), Academic press, New York, 73:147-166 (1981).

Examples of enzyme-substrate combinations include, for example:
(i) Horseradish peroxidase (HRPO) with hydrogen peroxidase as a substrate, wherein the hydrogen peroxidase oxidizes a dye precursor (*e.g.,* orthophenylene diamine (OPD) or 3,3',5,5'-tetramethyl benzidine hydrochloride (TMB));
(ii) alkaline phosphatase (AP) with para-Nitrophenyl phosphate as chromogenic substrate; and
(iii) β-D-galactosidase (β-D-Gal) with a chromogenic substrate (*e.g.*, p-nitrophenyl-β-D-galactosidase) or fluorogenic substrate (*e.g.*, 4-methylumbelliferyl-β-D-galactosidase).

Numerous other enzyme-substrate combinations are available to those skilled in the art. For a general review of these, see U.S. Patent Nos. 4,275,149 and 4,318,980. Sometimes, the label is indirectly conjugated with the antibody. The skilled artisan will be aware of various techniques for achieving this. For example, the antibody can be conjugated with biotin and any of the four broad categories of labels mentioned above can be conjugated with avidin, or *vice versa.* Biotin binds selectively to avidin and thus, the label can be conjugated with the antibody in this indirect manner. Alternatively, to achieve indirect conjugation of the label with the antibody, the antibody is conjugated with a small hapten and one of the different types of labels mentioned above is conjugated with an anti-hapten antibody. Thus, indirect conjugation of the label with the antibody can be achieved.

Aside from the sample preparation procedures discussed above, further treatment of the tissue section prior to, during or following IHC may be desired, For example, epitope retrieval methods, such as heating the tissue sample in citrate buffer may be carried out (see, e.g., Leong et al. Appl. Immunohistochem. 4(3):201 (1996)).

Following an optional blocking step, the tissue section is exposed to primary antibody for a sufficient period of time and under suitable conditions such that the primary antibody binds to the target protein antigen in the tissue sample. Appropriate conditions for achieving this can be determined by routine experimentation. The extent of binding of antibody to the sample is determined by using any one of the detectable labels discussed above. Preferably, the label is an enzymatic label (e.g. HRPO) which catalyzes a chemical alteration of the chromogenic substrate such as 3,3'-diaminobenzidine chromogen. Preferably the enzymatic label is conjugated to antibody which binds specifically to the primary antibody (e.g. the primary antibody is rabbit polyclonal antibody and secondary antibody is goat anti-rabbit antibody).

Specimens thus prepared may be mounted and coverslipped. Slide evaluation is then determined, e.g. using a microscope, and staining intensity criteria, routinely used in the art, may be employed. Staining intensity criteria may be evaluated as follows:

**TABLE 1**

| **Staining Pattern** | **Score** |
|---|---|
| No staining is observed in cells. | 0 |
| Faint/barely perceptible staining is detected in more than 10% of the cells. | 1+ |
| Weak to moderate staining is observed in more than 10% of the cells. | 2+ |
| Moderate to strong staining is observed in more than 10% of the cells. | 3+ |

It is understood that when cells and/or tissue from a tumor or colon adenoma are examined using IHC, staining is generally determined or assessed in tumor cell and/or tissue (as opposed to stromal or surrounding tissue that may be present in the sample). Typically, a staining pattern score of about 2+ or higher in an IHC assay is prognostic. In some embodiments, a staining pattern score of about 1+ or higher is prognostic. In other embodiments, a staining pattern score of about 3 of higher is prognostic.

In some embodiments, the biological sample may be contacted with an anti-EphB2 agent (such as an antibody that binds EphB2) under conditions sufficient for an anti-EphB2 agent--EphB2 complex to form, and then detecting said complex. Detection may be accomplished in a number of ways known in the art, such as by Western blotting and ELISA procedures for assaying a wide variety of tissues and samples, including plasma or serum. A wide range of immunoassay techniques using such an assay format are available, see, e.g., U.S. Pat. Nos. 4,016,043, 4,424,279 and 4,018,653. These include both single-site and two-site or "sandwich" assays of the non-competitive types, as well as in the traditional competitive binding assays. These assays also include direct binding of a labeled antibody to EphB2.

Sandwich assays are among the most useful and commonly used assays. A number of variations of the sandwich assay technique exist, and all are intended to be encompassed by the present invention. Briefly, in a typical forward assay, an unlabeled antibody is immobilized on a solid substrate, and the sample to be tested brought into contact with the bound molecule. After a suitable period of incubation, for a period of time sufficient to allow formation of an antibody-antigen complex, a second antibody specific to the antigen, labeled with a reporter molecule capable of producing a detectable signal is then added and incubated, allowing time sufficient for the formation of another complex of antibody-antigen-labeled antibody. Any unreacted material is washed away, and the presence of the antigen is determined by observation of a signal produced by the reporter molecule. The results may either be qualitative, by simple observation of the visible signal, or may be quantitated by comparing with a control sample containing known amounts of biomarker.

Variations on the forward assay include a simultaneous assay, in which both sample and labeled antibody are added simultaneously to the bound antibody. These techniques are well known to those skilled in the art, including any minor variations as will be readily apparent. In a typical forward sandwich assay, a first antibody having specificity for the biomarker is either covalently or passively bound to a solid surface. The solid surface is typically glass or a polymer, the most commonly used polymers being cellulose, polyacrylamide, nylon, polystyrene, polyvinyl chloride or polypropylene. The solid supports may be in the form of tubes, beads, discs of microplates, or any other surface suitable for conducting an immunoassay. The binding processes are well-known in the art and generally consist of cross-linking covalently binding or physically adsorbing, the polymer-antibody complex is washed in preparation for the test sample. An aliquot of the sample to be tested is then added to the solid phase complex and incubated for a period of time sufficient (e.g. 2-40 minutes or overnight if more convenient) and under suitable conditions (e.g. from room temperature to 40°C such as between 25° C and 32° C inclusive) to allow binding of any subunit present in the antibody. Following the incubation period, the antibody subunit solid phase is washed and dried and incubated with a second antibody specific for a portion of the biomarker. The second antibody is linked to a reporter molecule which is used to indicate the binding of the second antibody to the molecular marker.

Alternative methods involve immobilizing the target biomarkers in the sample and then exposing the immobilized target to specific antibody which may or may not be labeled with a reporter molecule. Depending on the amount of target and the strength of the reporter molecule signal, a bound target may be detectable by direct labeling with the antibody. Alternatively, a second labeled antibody, specific to the first antibody is exposed to the target-first antibody complex to form a target-first antibody-second antibody tertiary complex. The complex is detected by the signal emitted by the reporter molecule. By "reporter molecule", as used in the present specification, is meant a molecule which, by its chemical nature, provides an analytically identifiable signal which allows the detection of antigen-bound antibody. The most commonly used reporter molecules in this type of assay are either enzymes, fluorophores or radionuclide containing molecules (i.e. radioisotopes) and chemiluminescent molecules.

In the case of an enzyme immunoassay, an enzyme is conjugated to the second antibody, generally by means of glutaraldehyde or periodate. As will be readily recognized, however, a wide variety of different conjugation techniques exist, which are readily available to the skilled artisan. Commonly used enzymes include horseradish peroxidase, glucose oxidase, -galactosidase and alkaline phosphatase, amongst others. The substrates to be used with the specific enzymes are generally chosen for the production, upon hydrolysis by the corresponding enzyme, of a detectable color change. Examples of suitable enzymes include alkaline phosphatase and peroxidase. It is also possible to employ fluorogenic substrates, which yield a fluorescent product rather than the chromogenic substrates noted above. In all cases, the enzyme-labeled antibody is added to the first antibody-molecular marker complex, allowed to bind, and then the excess reagent is washed away. A solution containing the appropriate substrate is then added to the complex of antibody-antigen-antibody. The substrate will react with the enzyme linked to the second antibody, giving a qualitative visual signal, which may be further quantitated, usually spectrophotometrically, to give an indication of the amount of biomarker which was present in the sample. Alternately, fluorescent compounds, such as fluorescein and rhodamine, may be chemically coupled to antibodies without altering their binding capacity. When activated by illumination with light of a particular wavelength, the fluorochrome-labeled antibody adsorbs the light energy, inducing a state to excitability in the molecule, followed by emission of the light at a characteristic color visually detectable with a light microscope. As in the EIA, the fluorescent labeled antibody is allowed to bind to the first antibody-molecular marker complex. After washing off the unbound reagent, the remaining tertiary complex is then exposed to the light of the appropriate wavelength, the fluorescence observed indicates the presence of the molecular marker of interest. Immunofluorescence and EIA techniques are both very well established in the art. However, other reporter molecules, such as radioisotope, chemiluminescent or bioluminescent molecules, may also be employed.

In some embodiments, expression of ligands of EphB2, such as ephrin-B1, ephrin-B2, ephrin-B3 and/or ephrin-A4, is detected (alone or in conjunction with EphB2 expression) as further described herein. In still other embodiments, expression of APC, p53, DCC, DPC4, JV18-1/MADR2, and/or ras (such as c-Ki-ras or N-ras) is detected in conjunction with EphB2 expression.

Expression of EphB2 in a biological sample may also be detected using functional or activity-based assays. Methods for assaying EphB2 function are known in the art, and include the assay described in Mao et al. Cancer Res. 64: 781-788, 2004.

### Detection of EphB2 polynucleotides

Disclosed herein are methods to detect (e.g., presence or absence of or amount) a polynucleotide(s) (e.g., EphB2 polynucleotides) in a biological sample from a subject, such as a human subject. A variety of methods for detecting polynucleotides can be employed and include, for example, RT-PCR, taqman, amplification methods, polynucleotide microarray, and the like.

Methods for the detection of polynucleotides (such as mRNA) are well known and include, for example, hybridization assays using complementary DNA probes (such as *in situ* hybridization using labeled EphB2 riboprobes), Northern blot and related techniques, and various nucleic acid amplification assays (such as RT-PCR using complementary primers specific for EphB2, and other amplification type detection methods, such as, for example, branched DNA, SPIA, Ribo-SPIA, SISBA, TMA and the like).

In some embodiments, the polynucleotide(s) (such as a primer and/or probe) suitable for hybridization to EphB2 polynucleotide hybridizes to EphB2 polynucleotide and does not significantly cross-react with EphB1R polynucleotide and/or EphB3R polynucleotide. In some embodiments, the polynucleotide hybridizes to EphB2 polynucleotide and does not significantly cross react with EphB 1R polynucleotide, EphB3R polynucleotide, EphB4R polynucleotide, EphB5R polynucleotide and/or EphB6R polynucleotide. In some embodiments, the polynucleotide that hybridizes to EphB2 polynucleotide comprises at least about 10, 20, 30, 40, 50, 60, 70, 80, 90, 100, 250, 500, 750, 1000, 1100, 1200, 1300, 1400, 1500, 1600, 1700, 1800, 1900, 2000, 2100, 2200, 2300, 3400, 2500, 2600, 2700, 2800, 2900, 3000, 3100, 3200, 3300, 3400, 3500, 3600, 3700, 3800, 3900, 4000, 4100, 4200, 4300, 4400, 4500, or more contiguous nucleotides. In some embodiments, the polynucleotide that hybridizes to EphB2 polynucleotide comprises at least about 20, 30,40, 50, 60, 70, 80, 90, 100, 250, 500, 750, 1000, 1100, 1200, 1300, 1400, 1500, 1600, 1700, 1800, 1900, 2000, 2100, 2200, 2300, 3400, 2500, 2600, 2700, 2800, 2900, 3000, 3100, 3200, 3300, 3400, 3500, 3600, 3700, 3800, 3900, 4000, 4100, 4200, 4300, 4400, 4500, or more contiguous nucleotides shown in Figure 2, 4, and/or 6. In some embodiments, the polynucleotide that hybridizes to EphB2 polynucleotide is human EphB2, murine EphB2, rodent EphB2, and/or monkey EphB2. In some embodiments, the polynucleotide that hybridizes to EphB2 polynucleotide is human EphB2. It is understood that a polynucleotide that hybridizes to EphB2 may hybridize to one or more native sequence EphB2 polynucleotides. It is routine in the art to select polynucleotides that bind one or more native sequence EphB2 polynucleotide and further to determine whether a polynucleotide recognizes one or more native sequence EphB2 polynucleotide.

Biological samples from mammals can be conveniently assayed for, e.g., EphB2 mRNAs using Northern, dot blot or PCR analysis. For example, RT-PCR assays such as quantitative PCR assays are well known in the art. In an illustrative embodiment of the invention, a method for detecting EphB2 mRNA in a biological sample comprises producing cDNA from the sample by reverse transcription using at least one primer; amplifying the cDNA so produced using an EphB2 polynucleotide as sense and antisense primers to amplify EphB2 cDNAs therein; and detecting the presence or absence of the amplified EphB2 cDNA. In addition, such methods can include one or more steps that allow one to determine the amount (levels) of EphB2 mRNA in a biological sample (e.g. by simultaneously examining the levels a comparative control mRNA sequence of a housekeeping gene such as an actin family member). Optionally, the sequence of the amplified EphB2 cDNA can be determined.

Probes and/or primers may be labeled with a detectable marker, such as, for example, a radioisotope, fluorescent compound, bioluminescent compound, a chemiluminescent compound, metal chelator or enzyme. Such probes and primers can be used to detect the presence of EphB2 polynucleotides in a sample and as a means for detecting a cell expressing EphB2 proteins. As will be understood by the skilled artisan, a great many different primers and probes may be prepared (e.g., based on the sequences provided in herein) and used effectively to amplify, clone and/or determine the presence or absence of and/or amount of EphB2 mRNAs.

Optional methods of the invention include protocols comprising detection of polynucleotides, such as EphB2 polynucleotide, in a tissue or cell sample using microarray technologies. For example, using nucleic acid microarrays, test and control mRNA samples from test and control tissue samples are reverse transcribed and labeled to generate cDNA probes. The probes are then hybridized to an array of nucleic acids immobilized on a solid support. The array is configured such that the sequence and position of each member of the array is known. For example, a selection of genes that have potential to be expressed in certain disease states may be arrayed on a solid support. Hybridization of a labeled probe with a particular array member indicates that the sample from which the probe was derived expresses that gene. Differential gene expression analysis of disease tissue can provide valuable information. Microarray technology utilizes nucleic acid hybridization techniques and computing technology to evaluate the mRNA expression profile of thousands of genes within a single experiment. (see, e.g., WO 01/75166 published October 11, 2001; (See, for example, U.S. 5,700,637, U.S. Patent 5,445,934, and U.S. Patent 5,807,522, Lockart, Nature Biotechnology, 14:1675-1680 (1996); Cheung, V.G. et al., Nature Genetics 21(Suppl):15-19 (1999) for a discussion of array fabrication). DNA microarrays are miniature arrays containing gene fragments that are either synthesized directly onto or spotted onto glass or other substrates. Thousands of genes are usually represented in a single array. A typical microarray experiment involves the following steps: 1. preparation of fluorescently labeled target from RNA isolated from the sample, 2. hybridization of the labeled target to the microarray, 3. washing, staining, and scanning of the array, 4. analysis of the scanned image and 5. generation of gene expression profiles. Currently two main types of DNA microarrays are being used: oligonucleotide (usually 25 to 70 mers) arrays and gene expression arrays containing PCR products prepared from cDNAs. In forming an array, oligonucleotides can be either prefabricated and spotted to the surface or directly synthesized on to the surface (in situ).

The Affymetrix GeneChip® system is a commercially available microarray system which comprises arrays fabricated by direct synthesis of oligonucleotides on a glass surface. Probe/Gene Arrays: Oligonucleotides, usually 25 mers, are directly synthesized onto a glass wafer by a combination of semiconductor-based photolithography and solid phase chemical synthesis technologies. Each array contains up to 400,000 different oligos and each oligo is present in millions of copies. Since oligonucleotide probes are synthesized in known locations on the array, the hybridization patterns and signal intensities can be interpreted in terms of gene identity and relative expression levels by the Affymetrix Microarray Suite software. Each gene is represented on the array by a series of different oligonucleotide probes. Each probe pair consists of a perfect match oligonucleotide and a mismatch oligonucleotide. The perfect match probe has a sequence exactly complimentary to the particular gene and thus measures the expression of the gene. The mismatch probe differs from the perfect match probe by a single base substitution at the center base position, disturbing the binding of the target gene transcript. This helps to determine the background and nonspecific hybridization that contributes to the signal measured for the perfect match oligo. The Microarray Suite software subtracts the hybridization intensities of the mismatch probes from those of the perfect match probes to determine the absolute or specific intensity value for each probe set. Probes are chosen based on current information from GenBank and other nucleotide repositories. The sequences are believed to recognize unique regions of the 3' end of the gene. A GeneChip Hybridization Oven ("rotisserie" oven) is used to carry out the hybridization of up to 64 arrays at one time. The fluidics station performs washing and staining of the probe arrays. It is completely automated and contains four modules, with each module holding one probe array. Each module is controlled independently through Microarray Suite software using preprogrammed fluidics protocols. The scanner is a confocal laser fluorescence scanner which measures fluorescence intensity emitted by the labeled cRNA bound to the probe arrays. The computer workstation with Microarray Suite software controls the fluidics station and the scanner. Microarray Suite software can control up to eight fluidics stations using preprogrammed hybridization, wash, and stain protocols for the probe array. The software also acquires and converts hybridization intensity data into a presence/absence call for each gene using appropriate algorithms. Finally, the software detects changes in gene expression between experiments by comparison analysis and formats the output into .txt files, which can be used with other software programs for further data analysis.

In some embodiments, EphB2 gene deletion, gene mutation, or gene amplification is detected. Gene deletion, gene mutation, or amplification may be measured by any one of a wide variety of protocols known in the art, for example, by conventional Southern blotting, Northern blotting to quantitate the transcription of mRNA (Thomas, Proc. Natl. Acad. Sci. USA, 77:5201-5205 (1980)), dot blotting (DNA analysis), or *in situ* hybridization (e.g., FISH), using an appropriately labeled probe, cytogenetic methods or comparative genomic hybridization (CGH) using an appropriately labeled probe. In addition, these methods may be employed to detect EphB2 ligand gene deletion, ligand mutation, or gene amplification. As used herein, "detecting EphB2 expression" encompasses detection of EphB2 gene deletion, gene mutation or gene amplification.

Additionally, one can examine the methylation status of the EphB2 gene in a tissue or cell sample. Aberrant demethylation and/or hypermethylation of CpG islands in gene 5' regulatory regions frequently occurs in immortalized and transformed cells, and can result in altered expression of various genes. A variety of assays for examining methylation status of a gene are well known in the art. For example, one can utilize, in Southern hybridization approaches, methylation-sensitive restriction enzymes which cannot cleave sequences that contain methylated CpG sites to assess the methylation status of CpG islands. In addition, MSP (methylation specific PCR) can rapidly profile the methylation status of all the CpG sites present in a CpG island of a given gene. This procedure involves initial modification of DNA by sodium bisulfite (which will convert all unmethylated cytosines to uracil) followed by amplification using primers specific for methylated versus unmethylated DNA. Protocols involving methylation interference can also be found for example in Current Protocols In Molecular Biology, Unit 12, Frederick M. Ausubel et al. eds., 1995; De Marzo et al., Am. J. Pathol. 155(6): 1985-1992 (1999); Brooks et al, Cancer Epidemiol. Biomarkers Prev., 1998, 7:531-536); and Lethe et al., Int. J. Cancer 76(6): 903-908 (1998). As used herein, "detecting EphB2 expression" encompasses detection of EphB2 gene methylation.

In some embodiments, expression of ligands of EphB2, such as ephrin-B1, ephrin-B2, ephrin-B3 and/or ephrin-A4, is detected (alone or in conjunction (simultaneously and/or sequentially)) with EphB2 expression) as further described herein. In still other embodiments, expression of APC, p53, DCC, DPC4, JV18-1/MADR2, and/or ras (such as c-Ki-ras or N-ras) is detected in conjunction with EphB2 expression.

### Detection of EphB2 ligand

In the methods of the present invention, the biological sample may also be examined (either in conjunction with EphB2 expression or independently) for the expression of EphB2 ligand(s) (such as EphB2 ligand polypeptide and/or polynucleotide). As described above and in the art, it is presently believed that EphB2 binds to at least four different ligands: ephrin-B1, ephrin-B2, ephrin-B3, and ephrin-A4. Using methods known in the art, including those described herein, the polynucleotide and/or polypeptide expression of ephrin-B 1, ephrin-B2, ephrin-B3 and/or ephrin-A4 can be detected. By way of example, the IHC techniques described above may be employed to detect the presence of one of more such molecules in the sample. As used herein, "in conjunction" is meant to encompass any simultaneous and/or sequential detection. Thus, it is contemplated that in embodiments in which a biological sample is being examined not only for the presence of EphB2, but also for the presence, e.g., ephrin-B1, ephrin-B2, ephrin-B3, and/or ephrin-A4, separate slides may be prepared from the same tissue or sample, and each slide tested with a reagent that binds to EphB2 and/or ligand, respectively. Alternatively, a single slide may be prepared from the tissue or cell sample, and antibodies directed to EphB2 and ligand may be used in connection with a multi-color staining protocol to allow visualization and detection of the EphB2 and ligand.

### Data analysis and comparison

Data generated by detection can be analyzed using any suitable means (e.g., visually, by computer, etc.). In one embodiment, data is analyzed with the use of a programmable digital computer. The data analysis can include the steps of determining the intensity of the signal. The intensity can be normalized, whereby the intensity is calibrated relative to some reference value. For example, a reference can be background noise of the binding. Alternatively, a reference can be the protein binding intensity of a control antibody. The comparison of EphB2 expression (e.g., comparison of expression level in a test biological sample and a control biological sample or control reference level) can be performed by standard methods, including standard statistical methods such as chi-squared test, Student's t-test, or Spearman's rank correlation as appropriate. Additional statistical methods are discussed in Wohlgemuth et al., US2004/0009479 A1; Birbeck et al. Annals Surg 235:449-457 (2002) and exemplified herein. Software packages for performing statistical analysis are widely available.

### Methods comprising selection of cancer treatment

Disclosed herein are methods for selection of cancer treatment. As noted above, cancer treatment, such as chemotherapy, radiation and/or surgery, has associated risks, and it would be useful to be able to optimally select patients most likely to benefit. Prognostic testing is useful to, for example, identify patients with poor prognoses such that a more aggressive, higher risk treatment approach is identified, and to identify patients with good prognoses for whom risky therapy would not provide enough benefit to warrant the risks. Accordingly, disclosed are methods for selection of cancer treatment for a patient, the methods comprising (a) comparing expression of EphB2 in a biological sample from the patient with expression of EphB2 in a control sample (or control reference value); (b) predicting cancer prognosis of the patient based on the comparison in (a), wherein EphB2 expression is prognostic for cancer in the patient; and (c) subsequent to steps (a) and (b), selecting cancer treatment for the patient, wherein the selection of treatment is based on the patient prognosis determined in step (b). In some embodiments, increased EphB2 expression in the patient (test) sample is prognostic of cancer in the patient.

Disclosed herein are methods for selecting cancer treatment for patient, the methods comprising: (a) obtaining a patient biological sample; (b) detecting EphB2 expression in the biological sample, wherein EphB2 expression is prognostic for cancer in the patient; and (c) subsequence to steps (a) and (b), selecting cancer treatment for the patient, wherein the selection of treatment is based on the patient prognosis determined in step (b). In some embodiments, increased EphB2 expression in the patient biological sample is prognostic of cancer in the patient. In some embodiments, treatment encompasses ameliorating, reducing incidence of, palliating, delaying the development of, and/or delaying the progression of cancer.

Examplary cancers are described herein. Treatments for cancer are well known in the art. See, e.g., Cancer: Principles and Practice of Oncology (V. T. DeVita et al., eds., Williams & Wilkins Co., 2001); Manual of Clinical Oncology (Casciato, DA, ed., Lippincott, Williams & Wilkins Co., 2000); Bacquiran, DC ed., Lippincott's Cancer Chemotherapy Handbook, Lippincott Co., 2001); Armitage, JO, ed., High-Dose Cancer Therapy, Lippincott, Williams & Wilkins Co., 1999). In some embodiments, the treatment comprises administration of an effective amount of an immunoconjugate comprising an anti-EphB2 antibody conjugated to a cytotoxic agent such as a chemotherapeutic agent, a growth inhibitory agent, a toxin (e.g., an active toxin of synthetic, bacterial, fungal, plant, or animal origin, or fragments thereof), or a radioactive isotope (i.e., a radioconjugate). Immunoconjugates are known in the art and exemplary immunoconjugates are described herein. See also Pennell, CA. Immunol. Res. 25,177-191 (2002; Kreitman, RJ, Curr. Pharm. Biotechnol. 2: 313-325 (2001).

Further disclosed is the use of anti-EphB2 agents (such as an antagonist antibody). An anti-EphB2 protein comprising an EphB2 binding region may be used, for example, an ephrin-B1 immunoadhesin, ephrin-B2 immunoadhesin, ephrin-B3 immunoadhesin and ephrins-A4 immunoadhesin. In some embodiments, the anti-EphB2 antibody is a monoclonal antibody or a polyclonal antibody. In some embodiments, the antibody is a human antibody, a chimeric antibody, an affinity-matured antibody, a humanized antibody, or an antibody fragment. In some embodiments, the anti-EphB2 antibody is the antibody produced by hybridoma cell line 2H9.11.14 having American Tissue Type Culture (ATCC) No. PTA-6606 (deposited February 24, 2005). In some embodiments, the anti-EphB2 antibody is an antibody comprising heavy and/or light chain variable domain(s) of the antibody produced by hybridoma cell line 2H9.11.14 having American Tissue Type Culture (ATCC) No. PTA-6606, wherein said antibody specifically binds human EphB2. In some embodiments, the anti-EphB2 antibody comprises at least one (at least 2, at least 3, at least 4, at least 5, and/or 6) hypervariable sequence(s) (HVR(s)) comprising a sequence selected from the group consisting of HVR-L1, HVR-L2, HVR-L3, HVR-H1, HVR-H2, and/or HVR-H3 of the antibody produced by hybridoma cell line 2H9.11.14 having American Tissue Type Culture (ATCC) No. PTA-6606, wherein said antibody specifically binds human EphB2. In some embodiments, the anti-EphB2 antibody is an antibody that binds to the same epitope on human EphB2 as the antibody produced by hybridoma cell line 2H9.11.14 having American Tissue Type Culture (ATCC) No. PTA-6606. In some embodiments, the anti-EphB2 antibody is an antibody that competes with the antibody produced by hybridoma cell line 2H9.11.14 having American Tissue Type Culture (ATCC) No. PTA-6606 for binding to human EphB2. In some embodiments, the anti-EphB2 antibody comprises: at least one, two, three, four, five, and/or six hypervariable region (HVR) sequences selected from the group consisting of: (a) HVR-L1 comprising sequence KSSQSLLNSGNQENYLA (SEQ ID NO:1); (b) HVR-L2 comprising sequence GASTRES (SEQ ID NO:2); (c) HVR-L3 comprising sequence QNDHSYPFT (SEQ ID NO:3); (d) HVR-H1 comprising sequence SYWMH (SEQ ID NO:4); (e) HVR-H2 comprising sequence FINPSTGYTDYNQKFKD (SEQ ID NO:5); and (f) HVR-H3 comprising sequence RLKLLRYAMDY (SEQ ID NO:6). In one embodiment, the anti-EphB2 antibody comprises a light chain variable domain having the sequence:
DIVMTQSPSSLSVSAGEKVTMNCKSSQSLLNSGNQENYLAWYQQKPGQPPKLLIYGASTRESGVPDRF TGSGSGTDFTLTISSVQAEDLAVYYCQNDHSYPFTFGAGTKVEIKR (SEQ ID NO:7). In one embodiment, the anti-EphB2 antibody comprises a heavy chain variable domain having the sequence:
QVQLQQSGAELAKPGASVKMSCKASGYTFTSYWMHWVKQRPGQGLEWIGFINPSTGYTDYNQKFKD KATLTVKSSNTAYMQLSRLTSEDSAVYYCTRRLKLLRYAMDYWGQGTTLTVSA (SEQ ID NO:8). In one embodiment, the anti-EphB2 antibody comprises a light chain variable domain having the sequence:
DIVMTQSPSSLSVSAGEKVTMNCKSSQSLLNSGNQENYLAWYQQKPGQPPKLLIYGASTRESGVPDRF TGSGSGTDFTLTISSVQAEDLAVYYCQNDHSYPFTFGAGTKVEIKR (SEQ ID NO:7); and comprises a heavy chain variable domain having the sequence:

### Additional exemplary anti-EphB2 antibodies are described herein.

Disclosed herein is the use of immunoconjugates (interchangeably termed "antibody-drug conjugates" or "ADC"), comprising any of the anti-EphB2 antibodies described herein conjugated to a cytotoxic agent such as a chemotherapeutic agent, a drug, a growth inhibitory agent, a toxin (e.g., an enzymatically active toxin of bacterial, fungal, plant, or animal origin, or fragments thereof), or a radioactive isotope (i.e., a radioconjugate).

The use of antibody-drug conjugates for the local delivery of cytotoxic or cytostatic agents, i.e. drugs to kill or inhibit tumor cells in the treatment of cancer (Syrigos and Epenetos (1999) Anticancer Research 19:605-614; Niculescu-Duvaz and Springer (1997) Adv. Drg Del. Rev. 26:151-172; U.S. patent 4,975,278) allows targeted delivery of the drug moiety to tumors, and intracellular accumulation therein, where systemic administration of these unconjugated drug agents may result in unacceptable levels of toxicity to normal cells as well as the tumor cells sought to be eliminated (Baldwin et al., (1986) Lancet pp. (Mar. 15, 1986):603-05; Thorpe, (1985) "Antibody Carriers Of Cytotoxic Agents In Cancer Therapy: A Review," in Monoclonal Antibodies '84: Biological And Clinical Applications, A. Pinchera et al. (ed.s), pp. 475-506). Maximal efficacy with minimal toxicity is sought thereby. Both polyclonal antibodies and monoclonal antibodies have been reported as useful in these strategies (Rowland et al., (1986) Cancer Immunol. Immunother., 21:183-87). Drugs used in these methods include daunomycin, doxorubicin, methotrexate, and vindesine (Rowland et al., (1986) supra). Toxins used in antibody-toxin conjugates include bacterial toxins such as diphtheria toxin, plant toxins such as ricin, small molecule toxins such as geldanamycin (Mandler et al (2000) Jour. of the Nat. Cancer Inst. 92(19):1573-1581; Mandler et al (2000) Bioorganic & Med. Chem. Letters 10:1025-1028; Mandler et al (2002) Bioconjugate Chem. 13:786-791), maytansinoids (EP 1391213; Liu et al., (1996) Proc. Natl. Acad. Sci. USA 93:8618-8623), and calicheamicin (Lode et al (1998) Cancer Res. 58:2928; Hinman et al (1993) Cancer Res. 53:3336-3342). The toxins may effect their cytotoxic and cytostatic effects by mechanisms including tubulin binding, DNA binding, or topoisomerase inhibition. Some cytotoxic drugs tend to be inactive or less active when conjugated to large antibodies or protein receptor ligands.

ZEVALIN® (ibritumomab tiuxetan, Biogen/Idec) is an antibody-radioisotope conjugate composed of a murine IgG1 kappa monoclonal antibody directed against the CD20 antigen found on the surface of normal and malignant B lymphocytes and ¹¹¹In or ⁹⁰Y radioisotope bound by a thiourea linker-chelator (Wiseman et al (2000) Eur. Jour. Nucl. Med. 27(7):766-77; Wiseman et al (2002) Blood 99(12):4336-42; Witzig et al (2002) J. Clin. Oncol. 20(10):2453-63; Witzig et al (2002) J. Clin. Oncol. 20(15):3262-69). Although ZEVALIN has activity against B-cell non-Hodgkin's Lymphoma (NHL), administration results in severe and prolonged cytopenias in most patients. MYLOTARG™ (gemtuzumab ozogamicin, Wyeth Pharmaceuticals), an antibody drug conjugate composed of a hu CD33 antibody linked to calicheamicin, was approved in 2000 for the treatment of acute myeloid leukemia by injection (Drugs of the Future (2000) 25(7):686; US Patent Nos. 4970198; 5079233; 5585089; 5606040; 5693762; 5739116; 5767285; 5773001). Cantuzumab mertansine (Immunogen, Inc.), an antibody drug conjugate composed of the huC242 antibody linked via the disulfide linker SPP to the maytansinoid drug moiety, DM1, is advancing into Phase II trials for the treatment of cancers that express CanAg, such as colon, pancreatic, gastric, and others. MLN-2704 (Millennium Pharm., BZL Biologics, Immunogen Inc.), an antibody drug conjugate composed of the anti-prostate specific membrane antigen (PSMA) monoclonal antibody linked to the maytansinoid drug moiety, DM1, is under development for the potential treatment of prostate tumors. The auristatin peptides, auristatin E (AE) and monomethylauristatin (MMAE), synthetic analogs of dolastatin, were conjugated to chimeric monoclonal antibodies cBR96 (specific to Lewis Y on carcinomas) and cAC10 (specific to CD30 on hematological malignancies) (Doronina et al (2003) Nature Biotechnology 21(7):778-784) and are under therapeutic development.

Chemotherapeutic agents useful in the generation of immunoconjugates are described herein (eg., above). Enzymatically active toxins and fragments thereof that can be used include diphtheria A chain, nonbinding active fragments of diphtheria toxin, exotoxin A chain (from Pseudomonas aeruginosa), ricin A chain, abrin A chain, modeccin A chain, alpha-sarcin, Aleurites fordii proteins, dianthin proteins, Phytolaca americana proteins (PAPI, PAPII, and PAP-S), momordica charantia inhibitor, curcin, crotin, sapaonaria officinalis inhibitor, gelonin, mitogellin, restrictocin, phenomycin, enomycin, and the tricothecenes. See, e.g., WO 93/21232 published October 28, 1993. A variety of radionuclides are available for the production of radioconjugated antibodies. Examples include ²¹²Bi, ¹³¹I, ¹³¹In, ⁹⁰Y, and ¹⁸⁶Re. Conjugates of the antibody and cytotoxic agent are made using a variety of bifunctional protein-coupling agents such as N-succinimidyl-3-(2-pyridyldithiol) propionate (SPDP), iminothiolane (IT), bifunctional derivatives of imidoesters (such as dimethyl adipimidate HCl), active esters (such as disuccinimidyl suberate), aldehydes (such as glutaraldehyde), bis-azido compounds (such as bis (p-azidobenzoyl) hexanediamine), bis-diazonium derivatives (such as bis-(p-diazoniumbenzoyl)-ethylenediamine), diisocyanates (such as toluene 2,6-diisocyanate), and bis-active fluorine compounds (such as 1,5-difluoro-2,4-dinitrobenzene). For example, a ricin immunotoxin can be prepared as described in Vitetta et al., Science, 238: 1098 (1987). Carbon-14-labeled 1-isothiocyanatobenzyl-3-methyldiethylene triaminepentaacetic acid (MX-DTPA) is an exemplary chelating agent for conjugation of radionucleotide to the antibody. See WO94/11026.

Conjugates of an antibody and one or more small molecule toxins, such as a calicheamicin, maytansinoids, dolastatins, aurostatins, a trichothecene, and CC1065, and the derivatives of these toxins that have toxin activity, are also contemplated herein.

### i. Maytansine and maytansinoids

In some embodiments, the immunoconjugate comprises an antibody (full length or fragments) of the invention conjugated to one or more maytansinoid molecules.

Maytansinoids are mitototic inhibitors which act by inhibiting tubulin polymerization. Maytansine was first isolated from the east African shrub Maytenus serrata (U.S. Patent No. 3,896,111). Subsequently, it was discovered that certain microbes also produce maytansinoids, such as maytansinol and C-3 maytansinol esters (U.S. Patent No. 4,151,042). Synthetic maytansinol and derivatives and analogues thereof are disclosed, for example, in U.S. Patent Nos. 4,137,230; 4,248,870; 4,256,746; 4,260,608; 4,265,814; 4,294,757; 4,307,016; 4,308,268; 4,308,269; 4,309,428; 4,313,946; 4,315,929; 4,317,821; 4,322,348; 4,331,598; 4,361,650; 4,364,866; 4,424,219; 4,450,254; 4,362,663; and 4,371,533.

Maytansinoid drug moieties are attractive drug moieties in antibody drug conjugates because they are: (i) relatively accessible to prepare by fermentation or chemical modification, derivatization of fermentation products, (ii) amenable to derivatization with functional groups suitable for conjugation through the non-disulfide linkers to antibodies, (iii) stable in plasma, and (iv) effective against a variety of tumor cell lines.

Immunoconjugates containing maytansinoids, methods of making same, and their therapeutic use are disclosed, for example, in U.S. Patent Nos. 5,208,020, 5,416,064 and European Patent EP 0 425 235 B1. Liu et al., Proc. Natl. Acad. Sci. USA 93:8618-8623 (1996) described immunoconjugates comprising a maytansinoid designated DM1 linked to the monoclonal antibody C242 directed against human colorectal cancer. The conjugate was found to be highly cytotoxic towards cultured colon cancer cells, and showed antitumor activity in an in vivo tumor growth assay. Chari et al., Cancer Research 52:127-131 (1992) describe immunoconjugates in which a maytansinoid was conjugated via a disulfide linker to the murine antibody A7 binding to an antigen on human colon cancer cell lines, or to another murine monoclonal antibody TA.1 that binds the HER-2/neu oncogene. The cytotoxicity of the TA.1-maytansinoid conjugate was tested in vitro on the human breast cancer cell line SK-BR-3, which expresses 3 x 10⁵ HER-2 surface antigens per cell. The drug conjugate achieved a degree of cytotoxicity similar to the free maytansinoid drug, which could be increased by increasing the number of maytansinoid molecules per antibody molecule. The A7-maytansinoid conjugate showed low systemic cytotoxicity in mice.

Antibody-maytansinoid conjugates are prepared by chemically linking an antibody to a maytansinoid molecule without significantly diminishing the biological activity of either the antibody or the maytansinoid molecule. See, e.g., U.S. Patent No. 5,208,0200. An average of 3-4 maytansinoid molecules conjugated per antibody molecule has shown efficacy in enhancing cytotoxicity of target cells without negatively affecting the function or solubility of the antibody, although even one molecule of toxin/antibody would be expected to enhance cytotoxicity over the use of naked antibody. Maytansinoids are well known in the art and can be synthesized by known techniques or isolated from natural sources. Suitable maytansinoids are disclosed, for example, in U.S. Patent No. 5,208,020 and in the other patents and nonpatent publications referred to hereinabove. Preferred maytansinoids are maytansinol and maytansinol analogues modified in the aromatic ring or at other positions of the maytansinol molecule, such as various maytansinol esters.

There are many linking groups known in the art for making antibody-maytansinoid conjugates, including, for example, those disclosed in U.S. Patent No. 5,208,020 or EP Patent 0 425 235 B1, Chari et al., Cancer Research 52:127-131 (1992). Antibody-maytansinoid conjugates comprising the linker component SMCC may be prepared as disclosed in U.S. Patent Application No. 2005/169933 1. The linking groups include disulfide groups, thioether groups, acid labile groups, photolabile groups, peptidase labile groups, or esterase labile groups, as disclosed in the above-identified patents, disulfide and thioether groups being preferred. Additional linking groups are described and exemplified herein.

Conjugates of the antibody and maytansinoid may be made using a variety of bifunctional protein coupling agents such as N-succinimidyl-3-(2-pyridyldithio) propionate (SPDP), succinimidyl-4-(N-maleimidomethyl) cyclohexane-1-carboxylate (SMCC), iminothiolane (IT), bifunctional derivatives of imidoesters (such as dimethyl adipimidate HCl), active esters (such as disuccinimidyl suberate), aldehydes (such as glutaraldehyde), bis-azido compounds (such as bis (p-azidobenzoyl) hexanediamine), bis-diazonium derivatives (such as bis-(p-diazoniumbenzoyl)-ethylenediamine), diisocyanates (such as toluene 2,6-diisocyanate), and bis-active fluorine compounds (such as 1,5-difluoro-2,4-dinitrobenzene). Particularly preferred coupling agents include N-succinimidyl-3-(2-pyridyldithio) propionate (SPDP) (Carlsson et al., Biochem. J. 173:723-737 (1978)) and N-succinimidyl-4-(2-pyridylthio)pentanoate (SPP) to provide for a disulfide linkage.

The linker may be attached to the maytansinoid molecule at various positions, depending on the type of the link. For example, an ester linkage may be formed by reaction with a hydroxyl group using conventional coupling techniques. The reaction may occur at the C-3 position having a hydroxyl group, the C-14 position modified with hydroxymethyl, the C-15 position modified with a hydroxyl group, and the C-20 position having a hydroxyl group. In a preferred embodiment, the linkage is formed at the C-3 position of maytansinol or a maytansinol analogue.

### ii. Auristatins and dolastatins

In some embodiments, the immunoconjugate comprises an antibody of the invention conjugated to dolastatins or dolostatin peptidic analogs and derivatives, the auristatins (US Patent Nos. 5635483; 5780588). Dolastatins and auristatins have been shown to interfere with microtubule dynamics, GTP hydrolysis, and nuclear and cellular division (Woyke et al (2001) Antimicrob. Agents and Chemother. 45(12):3580-3584) and have anticancer (US 5663149) and antifungal activity (Pettit et al (1998) Antimicrob. Agents Chemother. 42:2961-2965). The dolastatin or auristatin drug moiety may be attached to the antibody through the N (amino) terminus or the C (carboxyl) terminus of the peptidic drug moiety (WO 02/088172).

Exemplary auristatin embodiments include the N-terminus linked monomethylauristatin drug moieties DE and DF, disclosed in "Monomethylvaline Compounds Capable of Conjugation to Ligands", US application 2005/238649 published on 27-10-2005.

Typically, peptide-based drug moieties can be prepared by forming a peptide bond between two or more amino acids and/or peptide fragments. Such peptide bonds can be prepared, for example, according to the liquid phase synthesis method (see E. Schröder and K. Lübke, "The Peptides", volume 1, pp 76-136, 1965, Academic Press) that is well known in the field of peptide chemistry. The auristatin/dolastatin drug moieties may be prepared according to the methods of: US 5635483; US 5780588; Pettit et al (1989) J. Am. Chem. Soc. 111:5463-5465; Pettit et al (1998) Anti-Cancer Drug Design 13:243-277; Pettit, G.R., et al. Synthesis, 1996, 719-725; and Pettit et al (1996) J. Chem. Soc. Perkin Trans. 15:859-863. See also Doronina (2003) Nat Biotechnol 21(7):778-784; "Monomethylvaline Compounds Capable of Conjugation to Ligands", US application 2005/238649 (disclosing, e.g., linkers and methods of preparing monomethylvaline compounds such as MMAE and MMAF conjugated to linkers).

### iii. Calicheamicin

In other embodiments, the immunoconjugate comprises an antibody of the invention conjugated to one or more calicheamicin molecules. The calicheamicin family of antibiotics are capable of producing doublestranded DNA breaks at sub-picomolar concentrations. For the preparation of conjugates of the calicheamicin family, see U.S. patents 5,712,374, 5,714,586, 5,739,116, 5,767,285, 5,770,701, 5,770,710, 5,773,001, 5,877,296 (all to American Cyanamid Company). Structural analogues of calicheamicin which may be used include, but are not limited to, γ₁^{I}, α₂^{I}, α₃^{I}, N-acetyl-γ₁^{I} PSAG and θ^{I}₁ (Hinman et al., Cancer Research 53:3336-3342 (1993), Lode et al., Cancer Research 58:2925-2928 (1998) and the aforementioned U.S. patents to American Cyanamid). Another anti-tumor drug that the antibody can be conjugated is QFA which is an antifolate. Both calicheamicin and QFA have intracellular sites of action and do not readily cross the plasma membrane. Therefore, cellular uptake of these agents through antibody mediated internalization greatly enhances their cytotoxic effects.

### iv. Other cytotoxic agents

Other antitumor agents that can be conjugated to the antibodies of the invention include BCNU, streptozoicin, vincristine and 5-fluorouracil, the family of agents known collectively LL-E33288 complex described in U.S. patents 5,053,394, 5,770,710, as well as esperamicins (U.S. patent 5,877,296).

Enzymatically active toxins and fragments thereof which can be used include diphtheria A chain, nonbinding active fragments of diphtheria toxin, exotoxin A chain (from Pseudomonas aeruginosa), ricin A chain, abrin A chain, modeccin A chain, alpha-sarcin, Aleurites fordii proteins, dianthin proteins, Phytolaca americana proteins (PAPI, PAPII, and PAP-S), momordica charantia inhibitor, curcin, crotin, sapaonaria officinalis inhibitor, gelonin, mitogellin, restrictocin, phenomycin, enomycin and the tricothecenes. See, for example, WO 93/21232 published October 28, 1993.

The present disclosure further contemplates an immunoconjugate formed between an antibody and a compound with nucleolytic activity (e.g., a ribonuclease or a DNA endonuclease such as a deoxyribonuclease; DNase).

For selective destruction of the tumor, the antibody may comprise a highly radioactive atom. A variety of radioactive isotopes are available for the production of radioconjugated antibodies. Examples include At²¹¹, I¹³¹, I¹²⁵, Y⁹⁰, Re¹⁸⁶, Re¹⁸⁸, Sm¹⁵³, Bi²¹², p³² Pb²¹² and radioactive isotopes of Lu. When the conjugate is used for detection, it may comprise a radioactive atom for scintigraphic studies, for example tc^{99m} or I¹²³, or a spin label for nuclear magnetic resonance (NMR) imaging (also known as magnetic resonance imaging, mri), such as iodine-123 again, iodine-131, indium-111, fluorine-19, carbon-13, nitrogen-15, oxygen-17, gadolinium, manganese or iron.

The radio- or other labels may be incorporated in the conjugate in known ways. For example, the peptide may be biosynthesized or may be synthesized by chemical amino acid synthesis using suitable amino acid precursors involving, for example, fluorine-19 in place of hydrogen. Labels such as tc^{99m} or I¹²³, .Re¹⁸⁶, Re¹⁸⁸ and In¹¹¹ can be attached via a cysteine residue in the peptide. Yttrium-90 can be attached via a lysine residue. The IODOGEN method (Fraker et al (1978) Biochem. Biophys. Res. Commun. 80: 49-57 can be used to incorporate iodine-123. "Monoclonal Antibodies in Immunoscintigraphy" (Chatal,CRC Press 1989) describes other methods in detail.

Conjugates of the antibody and cytotoxic agent may be made using a variety of bifunctional protein coupling agents such as N-succinimidyl-3-(2-pyridyldithio) propionate (SPDP), succinimidyl-4-(N-maleimidomethyl) cyclohexane-1-carboxylate (SMCC), iminothiolane (IT), bifunctional derivatives of imidoesters (such as dimethyl adipimidate HCl), active esters (such as disuccinimidyl suberate), aldehydes (such as glutaraldehyde), bis-azido compounds (such as bis (p-azidobenzoyl) hexanediamine), bis-diazonium derivatives (such as bis-(p-diazoniumbenzoyl)-ethylenediamine), diisocyanates (such as toluene 2,6-diisocyanate), and bis-active fluorine compounds (such as 1,5-difluoro-2,4-dinitrobenzene). For example, a ricin immunotoxin can be prepared as described in Vitetta et al., Science 238:1098 (1987). Carbon-14-labeled 1-isothiocyanatobenzyl-3-methyldiethylene triaminepentaacetic acid (MX-DTPA) is an exemplary chelating agent for conjugation of radionucleotide to the antibody. See WO94/11026. The linker may be a "cleavable linker" facilitating release of the cytotoxic drug in the cell. For example, an acid-labile linker, peptidase-sensitive linker, photolabile linker, dimethyl linker or disulfide-containing linker (Chari et al., Cancer Research 52:127-131 (1992); U.S. Patent No. 5,208,020) may be used.

The compounds of the invention expressly contemplate, but are not limited to, ADC prepared with cross-linker reagents: BMPS, EMCS, GMBS, HBVS, LC-SMCC, MBS, MPBH, SBAP, SIA, SIAB, SMCC, SMPB, SMPH, sulfo-EMCS, sulfo-GMBS, sulfo-KMUS, sulfo-MBS, sulfo-SIAB, sulfo-SMCC, and sulfo-SMPB, and SVSB (succinimidyl-(4-vinylsulfone)benzoate) which are commercially available (e.g., from Pierce Biotechnology, Inc., Rockford, IL., U.S.A). See pages 467-498, 2003-2004 Applications Handbook and Catalog.

### v. Preparation of antibody drug conjugates

In the antibody drug conjugates (ADC) of the invention, an antibody (Ab) is conjugated to one or more drug moieties (D), e.g. about 1 to about 20 drug moieties per antibody, through a linker (L). The ADC of Formula I may be prepared by several routes, employing organic chemistry reactions, conditions, and reagents known to those skilled in the art, including: (1) reaction of a nucleophilic group of an antibody with a bivalent linker reagent, to form Ab-L, via a covalent bond, followed by reaction with a drug moiety D; and (2) reaction of a nucleophilic group of a drug moiety with a bivalent linker reagent, to form D-L, via a covalent bond, followed by reaction with the nucleophilic group of an antibody. Additional methods for preparing ADC are described herein.

Ab-(L-D)ₚ I

The linker may be composed of one or more linker components. Exemplary linker components include 6-maleimidocaproyl ("MC"), maleimidopropanoyl ("MP"), valine-citrulline ("val-cit"), alanine-phenylalanine ("ala-phe"), p-aminobenzyloxycarbonyl ("PAB"), N-Succinimidyl 4-(2-pyridylthio) pentanoate ("SPP"), N-Succinimidyl 4-(N-maleimidomethyl) cyclohexane-I carboxylate ("SMCC'), and N-Succinimidyl (4-iodoacetyl) aminobenzoate ("SIAB"). Additional linker components are known in the art and some are described herein. See also "Monomethylvaline Compounds Capable of Conjugation to Ligands", US application 2005/238649.

In some embodiments, the linker may comprise amino acid residues. Exemplary amino acid linker components include a dipeptide, a tripeptide, a tetrapeptide or a pentapeptide. Exemplary dipeptides include: valine-citrulline (vc or val-cit), alanine-phenylalanine (af or ala-phe). Exemplary tripeptides include: glycine-valine-citrulline (gly-val-cit) and glycine-glycine-glycine (gly-gly-gly). Amino acid residues which comprise an amino acid linker component include those occurring naturally, as well as minor amino acids and non-naturally occurring amino acid analogs, such as citrulline. Amino acid linker components can be designed and optimized in their selectivity for enzymatic cleavage by a particular enzymes, for example, a tumor-associated protease, cathepsin B, C and D, or a plasmin protease.

Nucleophilic groups on antibodies include, but are not limited to: (i) N-terminal amine groups, (ii) side chain amine groups, e.g. lysine, (iii) side chain thiol groups, e.g. cysteine, and (iv) sugar hydroxyl or amino groups where the antibody is glycosylated. Amine, thiol, and hydroxyl groups are nucleophilic and capable of reacting to form covalent bonds with electrophilic groups on linker moieties and linker reagents including: (i) active esters such as NHS esters, HOBt esters, haloformates, and acid halides; (ii) alkyl and benzyl halides such as haloacetamides; (iii) aldehydes, ketones, carboxyl, and maleimide groups. Certain antibodies have reducible interchain disulfides, i.e. cysteine bridges. Antibodies may be made reactive for conjugation with linker reagents by treatment with a reducing agent such as DTT (dithiothreitol). Each cysteine bridge will thus form, theoretically, two reactive thiol nucleophiles. Additional nucleophilic groups can be introduced into antibodies through the reaction of lysines with 2-iminothiolane (Traut's reagent) resulting in conversion of an amine into a thiol. Reactive thiol groups may be introduced into the antibody (or fragment thereof) by introducing one, two, three, four, or more cysteine residues (e.g., preparing mutant antibodies comprising one or more non-native cysteine amino acid residues).

Antibody drug conjugates of the invention may also be produced by modification of the antibody to introduce electrophilic moieties, which can react with nucleophilic substituents on the linker reagent or drug. The sugars of glycosylated antibodies may be oxidized, e.g. with periodate oxidizing reagents, to form aldehyde or ketone groups which may react with the amine group of linker reagents or drug moieties. The resulting imine Schiff base groups may form a stable linkage, or may be reduced, e.g. by borohydride reagents to form stable amine linkages. In one embodiment, reaction of the carbohydrate portion of a glycosylated antibody with either glactose oxidase or sodium meta-periodate may yield carbonyl (aldehyde and ketone) groups in the protein that can react with appropriate groups on the drug (Hermanson, Bioconjugate Techniques). In another embodiment, proteins containing N-terminal serine or threonine residues can react with sodium meta-periodate, resulting in production of an aldehyde in place of the first amino acid (Geoghegan & Stroh, (1992) Bioconjugate Chem. 3:138-146; US 5362852). Such aldehyde can be reacted with a drug moiety or linker nucleophile.

Likewise, nucleophilic groups on a drug moiety include, but are not limited to: amine, thiol, hydroxyl, hydrazide, oxime, hydrazine, thiosemicarbazone, hydrazine carboxylate, and arylhydrazide groups capable of reacting to form covalent bonds with electrophilic groups on linker moieties and linker reagents including: (i) active esters such as NHS esters, HOBt esters, haloformates, and acid halides; (ii) alkyl and benzyl halides such as haloacetamides; (iii) aldehydes, ketones, carboxyl, and maleimide groups.

Alternatively, a fusion protein comprising the antibody and cytotoxic agent may be made, e.g., by recombinant techniques or peptide synthesis. The length of DNA may comprise respective regions encoding the two portions of the conjugate either adjacent one another or separated by a region encoding a linker peptide which does not destroy the desired properties of the conjugate.

In yet another embodiment, the antibody may be conjugated to a "receptor" (such streptavidin) for utilization in tumor pre-targeting wherein the antibody-receptor conjugate is administered to the patient, followed by removal of unbound conjugate from the circulation using a clearing agent and then administration of a "ligand" (e.g., avidin) which is conjugated to a cytotoxic agent (e.g., a radionucleotide).

Therapeutic formulations of anti-EphB2 antibody are prepared for storage by mixing the antibody having the desired degree of purity with optional pharmaceutically acceptable carriers, excipients or stabilizers (Remington: The Science and Practice of Pharmacy 20th edition (2000)), in the form of lyophilized formulations or aqueous solutions. Acceptable carriers, excipients, or stabilizers are nontoxic to recipients at the dosages and concentrations employed, and include buffers such as acetate, Tris, phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; tonicifiers such as trehalose and sodium chloride; sugars such as sucrose, mannitol, trehalose or sorbitol; surfactant such as polysorbate; salt-forming counter-ions such as sodium; metal complexes (e.g., Zn-protein complexes); and/or non-ionic surfactants such as TWEEN®, PLURONICS® or polyethylene glycol (PEG). The formulation preferably comprises the antibody at a concentration of between 5-200 mg/ml, preferably between 10-100 mg/ml.

The active ingredients may also be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nano-particles and nanocapsules) or in macroemulsions. Such techniques are disclosed in Remington: The Science and Practice of Pharmacy 20th edition (2000).

Sustained-release preparations may be prepared. Suitable examples of sustained-release preparations include semi-permeable matrices of solid hydrophobic polymers containing the antibody, which matrices are in the form of shaped articles, e.g., films, or microcapsules. Examples of sustained-release matrices include polyesters, hydrogels (for example, poly(2-hydroxyethyl-methacrylate), or poly(vinylalcohol)), polylactides (U.S. Pat. No. 3,773,919), copolymers of L-glutamic acid and y ethyl-L-glutamate, non-degradable ethylenevinyl acetate, degradable lactic acid-glycolic acid copolymers such as the LUPRON DEPOT® (injectable microspheres composed of lactic acid-glycolic acid copolymer and leuprolide acetate), and poly-D-(-)-3-hydroxybutyric acid.

The formulations to be used for in vivo administration must be sterile. This is readily accomplished by filtration through sterile filtration membranes.

The antibodies (such as pharmaceutical compositions comprising the antibody) may be administered to a patient, in accord with known methods, such as intravenous administration as a bolus or by continuous infusion over a period of time, by intramuscular, intraperitoneal, intracerobrospinal, subcutaneous, intra-articular, intrasynovial, intrathecal, oral, topical, or inhalation routes. Intravenous or subcutaneous administration of the antibody is preferred.
In one embodiment, the treatment of the present invention involves the combined administration of an anti-EphB2 antibody and one or more chemotherapeutic agents. The present invention contemplates administration of cocktails of different chemotherapeutic agents. The combined administration includes co administration, using separate formulations or a single pharmaceutical formulation, and consecutive administration in either order, wherein preferably there is a time period while both (or all) active agents simultaneously exert their biological activities.

Preparation and dosing schedules for such chemotherapeutic agents may be used according to manufacturers' instructions or as determined empirically by the skilled practitioner. Preparation and dosing schedules for chemotherapy are also described in, e.g., Chemotherapy Service Ed., M.C. Perry, Williams & Wilkins, Baltimore, MD (1992) and Lippincott's Cancer Chemotherapy handbook, Baquiran et al, eds. Lippincott, Williams and Wilkins (2002). The chemotherapeutic agent may precede, or follow administration of the antibody or may be given simultaneously therewith.

For the prevention or treatment of disease, the appropriate dosage of antibody will depend on the type of disease to be treated, as defined above, the severity and course of the disease, whether the antibody is administered for preventive or therapeutic purposes, previous therapy, the patient's clinical history and response to the antibody, and the discretion of the attending physician. The antibody is suitably administered to the patient at one time or over a series of treatments. In a combination therapy regimen, the compositions of the present invention are administered in a therapeutically effective or synergistic amount. As used herein, a therapeutically effective amount is such that co-administration of anti-EphB2 antibody and one or more other therapeutic agents, or administration of a composition of the present invention, results in reduction or inhibition of the targeting disease or condition. A therapeutically synergistic amount is that amount of anti-EphB2 antibody and one or more other therapeutic agents necessary to synergistically or significantly reduce or eliminate conditions or symptoms associated with a particular disease.

Depending on the type and severity of the disease, about 1 µg/kg to 50 mg/kg (e.g. 0.1-20mg/kg) of antibody is an initial candidate dosage for administration to the patient, whether, for example, by one or more separate administrations, or by continuous infusion. A typical daily dosage might range from about 1 µg/kg to about 100 mg/kg or more, depending on the factors mentioned above. For repeated administrations over several days or longer, depending on the condition, the treatment is sustained until a desired suppression of disease symptoms occurs. However, other dosage regimens may be useful. In a preferred aspect, the antibody of the invention is administered every two to three weeks, at a dose ranged from about 5mg/kg to about 15 mg/kg. The progress of the therapy of the invention is easily monitored by conventional techniques and assays. The efficacy of the treatment of the invention can be measured by various endpoints commonly used in evaluating cancer treatments, including but not limited to, tumor regression, tumor weight or size shrinkage, time to progression, duration of survival, progression free survival, overall response rate, duration of response, and quality of life.

### Methods of treatment of disorders comprising colon adenomas

Further disclosed herein methods for detecting EphB2 expression in colon adenomas, and methods for treating disorders characterized by colon adenomas (interchangeably termed "colon adenoma disorders"). The applicants surprising found that EphB2 is over-expressed in colon adenomas. Accordingly, EphB2 is an attractive target for immunoconjugate therapy for disorders featuring colon adenomas, for example, familial adenomatous polyposis (FAP), in which patients develop large numbers of colonic adenomatous polyps. Accordingly, in one aspect, the invention provides methods for treating a patient having or suspected of having a disorder characterized by colon adenoma(s) by administering an effective amount of an anti-EphB2 immunoconjugate to the patient. Further disclosed are methods for ameliorating, reducing incidence of, palliating, delaying the development of, delaying the progression of, or preventing a disorder characterized by colon adenoma(s) by administering an effective amount of an anti-EphB2 immunoconjugate to a patient having or suspected of having a disorder characterized by colon adenoma(s). In another aspect, the invention provides methods for treating a patient having or suspected of having a disorder characterized by colon adenoma(s) by administering an effective amount of an anti-EphB2 agent (such as an antibody) to the patient. Further disclosed are methods for ameliorating, reducing incidence of, palliating, delaying the development of, delaying the progression of, or preventing a disorder characterized by colon adenoma(s) by administering an effective amount of an anti-EphB2 agent (such as an antibody) to a patient having or suspected of having a disorder characterized by colon adenoma(s).

The methods of the disclosure are particularly suitable for disorders characterized by a plurality of colon adenomas (such as more than 10, 20, 30, 40, 50, 100, 200, 300, 400, 500, 600, 700, 800, 900, 1000, or more colon adenomas), including at least the following: autosomal dominant familial adenomatous polyposis (FAP) disorder caused by mutation in the APC gene (Tomlinson et al., J Med Genet 1996;33:268-73); Peutz-Jegher's syndrome (PJS), Juvenile Polyposis Syndrome (JPS), Attenuated FAP caused by mutations in the MYH gene (Sieber et al. N Eng J Med 348:791-9 (2003)), Hereditary Mixed Polyposis syndrome (HMPS), Cowden disease, and Bannayan-Ruvalcaba-Riley syndrome. See also Crawford JM, the Gastrointestinal tract, in: Cotran RS et al. Robbin's Pathological Basis of Disease, 6th ed. London: Saunders (1999) pp775-844; Nicum et al., Acta Oncol 42:263-275 (2003); Kinzler, KW, and Vogelstein, B. Colorectal Tumors, in Kinzeler KW, Vogelstein B, eds. The Genetic Basis of Human Cancer: McGraw-Hill 1999, p. 565-87.

Exemplary anti-EphB2 agents (such as antibodies and immunoconjugates) are described herein. Exemplary formulations and dosing are described herein. The progress of the therapy of the invention is easily monitored by conventional techniques and assays as known in the art and disclosed herein. The efficacy of the treatment of the invention can be measured by various endpoints commonly used in evaluating treatments, including but not limited to, adenoma regression, adenoma weight or size shrinkage, time to progression, duration of survival, progression free survival, overall response rate, duration of response, and quality of life.

### Methods for detection of EphB2 expression in colon adenoma

In another aspect, the disclosure provides methods comprising the detection of EphB2 polypeptide(s) and/or polynucleotide(s) in a biological sample from a patient having or suspected of having a colon adenoma disorder. As noted herein, the applicants surprising found that EphB2 is over-expressed in colon adenomas, such as such as flat, tubular, tubulovillous, and villous adenomas. Detection of EphB2 expression in colon adenomas is useful, for example, to determine suitability for treatment with an anti-EphB2 agent (such as an immunoconjugate comprising an anti-EphB2 antibody), monitor treatment with anti-EphB2 immunoconjugates or other agents or interventions (e.g., surgery, radiation), determine recurrence of adenomas, monitor progression of adenomas, and the like. In some embodiments, EphB2 expression is detected before; during; after; before and during; before and after; during and after; before, during and after treatment (for example, treatment with an anti-EphB2 agent).

Accordingly, in one aspect, provided are methods for detection of EphB2 polynucleotide and/or polypeptide in a biological sample from a patient having or suspected of having a colon adenoma disorder, the method comprising detecting expression of EphB2 polynucleotide and/or polypeptide in the biological sample. In another aspect, the invention provides methods for detection of EphB2 polynucleotide and/or polypeptide in a biological sample from a patient having or suspected of having a colon adenoma disorder, the methods comprising comparing expression of EphB2 polynucleotide and/or polypeptide in the biological sample with expression of EphB2 in a control sample (or control reference value). In some embodiments, increased EphB2 expression in the patient sample relative to the control sample (or control reference value) is prognostic for cancer in the subject. In some embodiments, decreased EphB2 expression in the patient sample relative to the control sample (or control reference value) is prognostic for cancer in the subject.

Further provided are methods for detecting EphB2 polynucleotide and/or polypeptide expression in colon adenoma cells and/or tissue from a patient, the methods comprising: (a) obtaining the colon adenoma cells and/or tissue; and (b) detecting EphB2 expression in the colon adenoma cells and/or tissue. In some embodiments, increased EphB2 expression in the patient biological sample relative to a control sample (or a control reference value) is prognostic for cancer in the subject. In some embodiments, decreased EphB2 expression in the patient sample relative to the control sample (or control reference value) is prognostic for cancer in the subject.

The methods of the disclosure may also detect EphB2 variant polypeptide(s) and/or polynucleotide(s) and/or EphB2 fragments. Detection of EphB2 variants and fragments is described herein.

Detection of expression may be quantitative or qualitative. Presence and/or absence and/or level of EphB2 expression may be detected. It is understood that absence of EphB2 expression includes insignificant, or de minimus levels. The expression of EphB2 polynucleotide and/or polypeptide in the test biological sample may be higher than that observed for a control biological sample (termed "over-expression). In some embodiments, EphB2 expression is at least about 2-fold, 5-fold, 10-fold, 20-fold, 30-fold, 40-fold, 50-fold, 75-fold, 100-fold, 150-fold higher, or higher in the test biological sample. In some embodiments, EphB2 polypeptide expression is determined in an immunohistochemistry ("IHC") assay to score at least 2 or higher for staining intensity. In some embodiments, EphB2 polypeptide expression is determined in an IHC assay to score at least 1 or higher, or at least 3 or higher for staining intensity. The expression of EphB2 polynucleotide and/or polypeptide in the test biological sample may be lower than that observed for a control biological sample (termed "under-expression). In some embodiments, EphB2 expression is at least about 2-fold, 5-fold, 10-fold, 20-fold, 30-fold, 40-fold, 50-fold, 75-fold, 100-fold, 150-fold lower, or lower in the test biological sample. Methods for detection of EphB2 polynucleotide and/or polypeptide are known in the art and disclosed and exemplified herein.

In another aspect, the disclosure provides methods for diagnosis of disorders characterized by colon adenomas, said methods comprising detection of EphB2 polynucleotide and/or polypeptide in a biological sample from a patient having or suspected of having a colon adenoma disorder. In some embodiments, increased EphB2 expression in the patient biological sample relative to a control sample (or a control reference value) is diagnostic for a colon adenoma disorder in the subject.

### Kit, compositions, and articles of manufacture

For use in the applications described or suggested above, kits, compositions, and articles of manufacture are also provided. In some embodiments, the kits comprise one or more containers comprising an anti-EphB2 agent (such as an antibody), an anti-EphB2 immunoconjugate, an EphB2 polynucleotide, and/or an EphB2 polypeptide, and in some embodiments, further comprise instructions for use in accordance of any of the methods described herein (such as methods for evaluation (such as prognostic evaluation) of a patient having or suspected of having cancer, method for selection of cancer treatment for a patient, methods for treating a patient having or suspected of having a colon adenoma disorder, methods for detecting EphB2 polynucleotide or polypeptide in a biological sample from a patient having or suspected of having a colon adenoma disorder, methods for detecting EphB2 polynucleotide or polypeptide expression in colon adenoma cells or tissue from a patient, and/or methods comprising detecting expression of EphB2 polynucleotide or polypeptide in the biological sample). The kits may further comprise a suitable control (control reference value).

Such kits may comprise a carrier means being compartmentalized to receive in close confinement one or more container means such as vials, tubes, and the like, each of the container means comprising one of the separate elements to be used in the method. For example, one of the container means may comprise a polynucleotide (such as a probe or primer) that is or can be detectably labeled. Such polynucleotide may be an antibody or polynucleotide specific for an EphB2 protein or an EphB2 polynucleotide, respectively. Where the kit utilizes nucleic acid hybridization to detect the target nucleic acid, the kit may also have, for example, containers containing nucleotide(s) for amplification of the target nucleic acid sequence and/or a container comprising a reporter-means, such as a biotin-binding protein, such as avidin or streptavidin, bound to a reporter molecule, such as an enzymatic, florescent, or radioisotope label.

The kit will typically comprise the container described above and one or more other containers comprising materials desirable from a commercial and user standpoint, including buffers, diluents, filters, needles, syringes, and package inserts with instructions for use. A label may be present on the container to indicate that the composition is used for a specific therapy or non-therapeutic application, and may also indicate directions for either *in vivo* or *in vitro* use, such as those described above.

The kits have a number of embodiments. A typical embodiment is a kit comprising a container, a label on said container, and a composition contained within said container; wherein the composition includes a primary antibody that binds to a EphB2 polypeptide sequence, the label on said container indicates that the composition can be used to evaluate the presence of EphB2 proteins in at least one type of mammalian cell, and instructions for using the EphB2 antibody comprising detection of the presence or absence of EphB2 protein in at least one type of mammalian cell. The kit can further comprise a set of instructions and materials for preparing a tissue sample and applying antibody and probe to the same section of a tissue sample. The kit may include both a primary and secondary antibody, wherein the secondary antibody is conjugated to a label, e.g., an enzymatic label.

Another embodiment is a kit comprising a container, a label on said container, and a composition contained within said container; wherein the composition includes a polynucleotide that hybridizes to a complement of the EphB2 polynucleotide under stringent conditions, the label on said container indicates that the composition can be used to evaluate the presence of EphB2 in at least one type of mammalian cell, and instructions comprising use of the EphB2 polynucleotide for evaluating the presence of EphB2 RNA or DNA in at least one type of mammalian cell.

Other optional components in the kit include one or more buffers (*e.g*., block buffer, wash buffer, substrate buffer, etc), other reagents such as substrate (*e.g*., chromogen) which is chemically altered by an enzymatic label, epitope retrieval solution, control samples (positive and/or negative controls), control slide(s) etc.

Compositions (such a pharmaceutical compositions) comprise an anti-EphB2 agent (such as an antibody), and anti-EphB2 immunoconjugate, an EphB2 polynucleotide, and/or an EphB2 polypeptide, and in some embodiments, further comprise instructions for use in accordance of any of the methods described herein (such as methods for evaluation (such as prognostic evaluation) of a patient having or suspected of having cancer, method for selection of cancer treatment for a patient, methods for treating a patient having or suspected of having a colon adenoma disorder, methods for detecting EphB2 polynucleotide or polypeptide in a biological sample from a patient having or suspected of having a colon adenoma disorder, methods for detecting EphB2 polynucleotide or polypeptide expression in colon adenoma cells or tissue from a patient, and/or methods comprising detecting expression of EphB2 polynucleotide or polypeptide in the biological sample).

Articles of manufacture may comprise kit components and/or compositions are described herein.

Various aspects of the invention are further described and illustrated by way of the examples that follow, none of which are intended to limit the scope of the invention.

### EXAMPLES

### METHODS AND MATERIALS

### Gene Logic Database

The expression of EphB2 was examined in the Gene Logic (Gaithersburg, MD) database of Affymetrix HG-U133 probearray data (probeset 209588_at) for colorectal tissues, including normal colon (n = 288), adenomas (n = 30), primary cancers (n = 122), distant metastases (n = 55), laser microdissected normal epithelium (n = 16), and laser microdissected epithelium from primary cancers (n = 9). Briefly, analysis of the GeneExpress® (Gene Logic Inc., Gaithersburg, MD) database, a proprietary database containing gene expression information, was conducted using either software available through Gene Logic Inc., Gaithersburg, MD, for use with the GeneExpress® database or with proprietary software written and developed at Genentech, Inc. for use with the GeneExpress® database. See Jubb et al., J Clin Pathol 2004 57:514-512 for further description of the GeneLogic system. The rating of positive hits in the analysis is based upon several criteria including, for example, tissue specificity, tumor specificity and expression level in normal essential and/or normal proliferating tissues.

### Tissue Samples and Tissue Microarray (TMA) Construction

### Cell Lines

Colo206 and CX-1 were obtained from DSMZ (Braunschweig, Germany), KM12 was obtained from the National Cancer Institute (Bethesda, MD), and all other CRC cell lines were obtained from ATCC (Manassas, VA). Cells were cultured in 50/50 Ham's F12 and Dulbeco's modified Eagle medium, supplemented with 2 mM L-glutamine and 10% fetal bovine serum. Total RNA was harvested and hybridized to Affymetrix HG-U133 GeneChip probearrays as described previously. (Yang et al., Arterioscler Thromb Vasc Biol 2002;22:1797-803). Cell pellets were also fixed in formalin, embedded in paraffin and represented in TMAs as previously described. (Kononen et al. Nat Med 1998;4:844-7).

### Human Tissues

An ethical review committee (University of Leeds, UK) approved the use of all tissues and clinical information. The Leeds General Infirmary (Leeds, UK) histopathology archive was screened to identify 148 anonymized colorectal adenomas, including 84 tubular adenomas, 27 tubulovillous adenomas, 5 villous adenomas, and 32 flat adenomas. Formalin-fixed paraffin-embedded tissues were biopsied to represent each adenoma in TMAs, as previously described. (Kononen et al. Nat Med 1998;4:844-7).

Formalin-fixed paraffin-embedded tissues from matched primary cancers and metastases from multiple sources were retrieved from the Genentech archives. The series comprised 28 primary CRCs and 30 metastases (six mesenteric, 21 lymph nodes, and 3 hepatic), representing 27 patients. An additional nine unmatched hepatic metastases were also included. Whole sections were cut and used for downstream assays.

This study also investigated 342 CRCs from the University of Leeds tissue archive, representing 330 patients who underwent a resection in the Department of Surgery at the Marien-Hospital (Duesseldorf, Germany) between January 1990 and December 1995. Matched normal mucosa and survival data were available for all patients, including information on recurrence of disease. The median follow-up time was 4.2 years (range: 5 months to 11.4 years). Postoperatively, fourteen patients received chemotherapy, twelve patients received radiotherapy, and seven patients both. One hundred and ten tumors (32 %) were proximal to the splenic flexure, the mean age at diagnosis was 69 years (range: 28-88 years), and 150 patients (45 %) were male. At the end of the follow-up period, local disease recurrence was noted in 25 patients, distant metastasis in 49 patients, and 8 patients showed both. (Grabsch et al. Am J Clin Pathol 2004;122:511-6). TMAs were constructed as previously described. (Kononen et al. Nat Med 1998;4:844-7).

### In Situ Hybridization (ISH)

³³P-labeled riboprobes were employed to evaluate EphB2 mRNA expression. cDNA templates were amplified by polymerase chain reaction from whole human brain marathon-ready cDNA (BD Clontech, Palo Alto, CA). Forward and reverse primers contained 5' T7 and T3 RNA polymerase initiation sites, respectively. Riboprobes were designed to complement nucleotides 3043-3500 of the transcript variant 2 mRNA sequence (GenBank accession NM_004442) and nucleotides 2972-3404 of the transcript variant 1 mRNA sequence (GenBank accession NM_017449): forward 5'-T7-GCCCTCCTGGTGCTCTATCC-3' (SEQ ID NO:15), reverse 5'-T3-TCTGTCCATCTGTCCCGTCCT-3' (SEQ ID NO:16). In vitro transcription of sense and antisense probes, hybridization and development of sections were carried out as described in Jubb et al. J Pathol 2003;200:577-88. Hybridized tissue sections were reviewed by bright and dark-field microscopy and scored on a scale of 0-3 for the maximum intensity of EphB2 expression in >10 % of the epithelium.

### Immunohistochemistry (IHC)

IHC was performed as described in Jubb et al. J Pathol 2003;200:577-88. In brief, heat mediated antigen retrieval was performed on deparaffinized sections (Target retrieval solution, DAKO cytomation, Carpinteria, USA), prior to blocking of endogenous biotin (Avidin-Biotin blocking kit, Vector Labs, Burlingame, CA) and non-specific immunoglobulins with 10 % normal horse serum, according to the manufacturers' instructions. Immunolabeling was performed with an anti-EphB2 polyclonal antibody (catalog number AF467, R&D Systems, Minneapolis, MN) or naïve goat immunoglobulins (R&D Systems) at 1 µg/mL. Immunocomplexes were labeled with a biotinylated anti-goat secondary antibody (Vector Labs) and an avidin-biotin-horseradish peroxidase complex (Vectastain Elite, Burlingame, CA). Tissues were scored on a scale of 0-3 for the maximum intensity of EphB2 expression in >10 % of the epithelium. Cell pellet TMAs were included in each staining run to control for variability in staining intensity.

### Statistical Analysis

The same pathologist scored all cases, blinded to the clinical outcome. Scoring was performed according to the criteria listed in Table 1. In all TMAs, the score from the highest expressing core (n = 1-3 /sample) was used to represent each patient. Mean survival times within each subgroup were estimated from Kaplan-Meier curves and corresponding hazard ratios for overall and recurrence-free survival were estimated by proportional hazards fit modeling using JMP software version 5.1 (SAS Institute Inc., Cary, NC). Statistical associations were assessed using the chi-squared test, Student's t-test, or Spearman's rank correlation as appropriate. Statistical significance was assumed if the P value was < 0.05.

### EXPERIMENTAL RESULTS

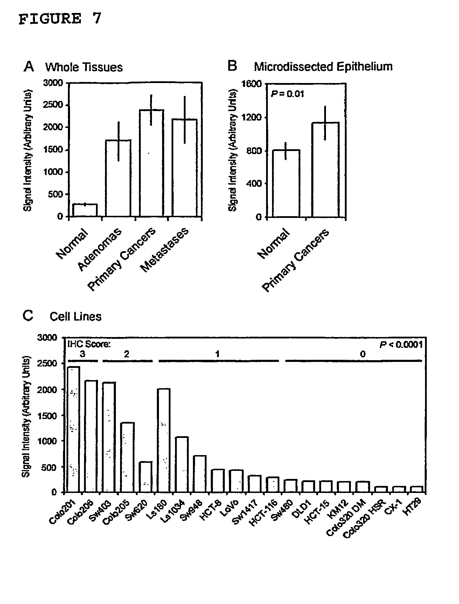

EphB2 expression was analyzed in normal mucosa, adenomas, matched primary cancers and metastases, and a series of primary cancers with detailed clinical follow-up. The aims of this study were to investigate the prognostic impact of EphB2 in colorectal cancer ("CRC"), and to examine the relative levels of EphB2 expression across the adenoma to adenocarcinoma sequence. Experiments were conducted using the methods and materials described above. Results of these experiments are illustrated in Figures 7-11, as discussed below.

### Oligonucleotide Microarray Data

An analysis of the Gene Logic database demonstrated that normal colon had a significantly lower mean EphB2 expression level than colorectal adenomas, primary cancers, and distant metastases, P < 0.0001 (Figure 7A). This trend was preserved in laser microdissected samples, P = 0.01 (Figure 7B). Primary cancers did not have a significantly greater mean expression than adenomas (P = 0.06) or metastases (P = 0.48) (Figure 7A). Expression,profiling revealed that EphB2 was less frequent in CRC cell lines, with most expressing at levels consistent with normal colon (Figure 7C). Levels of EphB2 transcript and EphB2 protein were significantly correlated in CRC cell lines (P < 0.0001).

### Localization

In both normal and neoplastic large intestine, expression of EphB2 mRNA and protein was localized to the epithelium (Figures 8, 9). EphB2 protein expression was predominantly membranous, with weaker cytoplasmic expression. In all samples of normal colon, both mRNA (n = 47) and protein (n = 342) expression were most intense at the base of the crypt (score = 2), with expression declining to the luminal epithelium (score = 0). The maximum intensity of expression in neoplastic cell populations was equivalent to expression at the base of the colonic crypt (Figures 8, 9). All subtypes of colorectal adenomas displayed evidence for homogeneous expression of EphB2, with relatively greater levels in flat compared to polypoid lesions (Table 2). The lack of a correlation with tumor size or the severity of dysplasia, suggests that expression is present from a very early stage in tumorigenesis (data not shown). Although 82 % of primary CRCs and 64 % of metastases showed some level of EphB2 expression in whole sections (Figure 8), EphB2 was not homogeneously or uniformly expressed throughout the malignancies. This is in contrast to adenomatous lesions, which, when present, expressed EphB2 homogeneously throughout the neoplastic cell population. Twenty three percent of all adenomas did not express EphB2. Hybridization of sense ISH probes and staining of naïve immunoglobulins did not exceed background (data not shown). Benign stroma, normal lymph node tissue, and normal hepatic tissue were uniformly negative for EphB2 expression.

**Table 2. Intensity of EphB2 Expression in Different Histological Subtypes of Adenoma**

| Histology | EphB2 IHC Intensity Score, n (%) | | | | *P* (T-test vs. villous adenomas) |
|---|---|---|---|---|---|
| | 0 | 1 | 2 | 3 | |
| Flat Adenomas (n = 32)* | 2 (6) | 13 (41) | 11 (34) | 6 (19) | 0.81 |
| Tubular Adenomas (n = 84) | 22 (26) | 35 (42) | 25 (30) | 2 (2) | 0.10 |
| Tubulovillous Adenomas (n = 27) | 9(33) | 10 (37) | 8 (30) | | 0.05 |
| Villous Adenomas (n = 5) | | 3 (60) | 1 (20) | 1 (20) | |

| | | | | | |
|---|---|---|---|---|---|
| *T-test for flat vs. polypoid adenomas, *P* = 0.001 | | | | | |

### Colorectal Adenomas, Primary Cancers, and Metastases

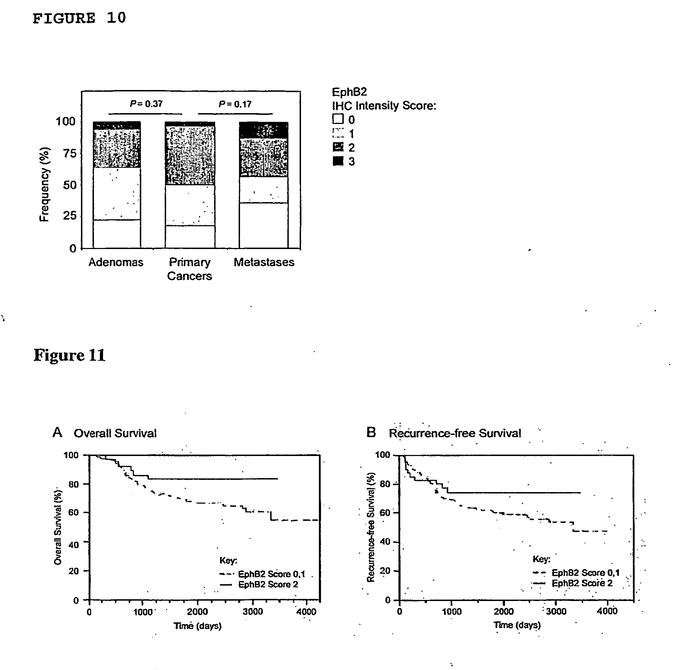

One hundred and fifteen (77 %) of 148 adenomas were positive (score ≥1) for EphB2 expression. This was not statistically different from the intensity of expression observed in primary CRCs, P = 0.37 (Figure 10). EphB2 expression was demonstrated at all stages of colorectal tumorigenesis, including all normal crypts, 77% of adenomas, 82% of primary cancers, and 64% of metastases. Where present, EphB2 was homogeneously expressed throughout the adenomas. Different histological subtypes of polypoid adenomas showed no statistically significant differences in the intensity of EphB2 expression (Table 2), though, expression was significantly greater in flat adenomas (P = 0.001). There were no significant associations with site, severity of dysplasia, polyp frequency, polyp size, or patient sex (data not shown). EphB2 expression was comparable in primary CRCs and metastases, P = 0.17 (Figure 10). Although homogeneous expression was observed in adenomas, the pattern of staining was focal in most malignant lesions, with considerable variation in the intensity of staining throughout the neoplastic cell population. A mean 25 % of tumor cells expressed EphB2 protein in primary and secondary malignancies scored positive for EphB2 expression. All subtypes of colorectal adenomas displayed evidence for homogeneous expression of EphB2, with relatively greater levels in flat compared to polypoid lesions (Table 2). The lack of a correlation with tumor size or the severity of dysplasia, suggests that expression is present from a very early stage in tumorigenesis.

### Prognostic Series of Primary CRCs

Three hundred and twenty seven of 330 patients were informative for EphB2 protein expression: 185 patients scored zero, 101 patients scored 1, and 41 patients scored two. (No patients scored three in this series, and cores were absent from three patients.) ISH was performed on one TMA containing 47 CRCs: eight patients scored zero, 19 patients scored one, 18 patients scored two, and two patients scored three. Matched normal colonic crypts were positive at the base in all cases. A significant association was observed between EphB2 IHC and ISH scores in 46 cancers informative for both, P < 0.01.

The effects of EphB2 protein expression were included in a proportional hazards fit for overall survival. High levels of EphB2 expression were associated with a longer mean duration of survival in colorectal cancer and a longer mean recurrence-free survival. Patients whose tumor stained 2+ for EphB2 expression (vs. 0/1+) exhibited significantly prolonged overall survival: mean duration of survival 2514 vs. 1044 days, hazard ratio 0.45, 95% confidence intervals: 0.18-0.95 (Figure 11A). Similar results were found in analyses of recurrence-free survival (mean survival 795 vs. 2233 days, HR 0.60, CI: 0.30-1.10) (Figure 11B). Survival curves for patients scored 0 or 1 were overlapping. The mean age was significantly lower in patients with high EphB2 protein expression (65 vs. 69.3 years, P < 0.02) (Table 3), though, age was not a prognostic factor in this series (data not shown). No other statistically significant associations were observed (Table 2), and there were no differences in the frequency of adjuvant therapy received by the two groups (data not shown). The survival impact of the clinicopathological variables described in table 2 has been discussed previously. (Grabsch et al. Am J Clin Pathol 2004;122:511-6). In a multivariate analysis, including stage, grade, lymphatic invasion and blood vessel invasion, an EphB2 score of 2 was a significant, independent prognostic factor (HR 0.47, CI 0.18-0.99).

**Table 3. Association of EphB2 Expression and Clinicopathological Variables in 327 CRC Patients**

| Variable | | n | EphB2 IHC Intensity Score, n (%) | | *P* |
|---|---|---|---|---|---|
| | | | 2 | 0 or 1 | |
| Age | | | | | |
| | Mean | | 65 | 69.3 | 0.02 |

| Sex | | | | | |
|---|---|---|---|---|---|
| | Male | 150 | 23 (56) | 127 (44) | 0.22 |
| | Female | 177 | 18(44) | 159 (56) | |

| TNM Stage | | | | | |
|---|---|---|---|---|---|
| | I | 74 | 14 (34) | 60 (21) | 0.28 |
| | II | 140 | 14 (34) | 126 (44) | |
| | III | 107 | 12 (29) | 95 (33) | |
| | IV | 6 | 1 (2) | 5 (2) | |

| Grade | | | | | |
|---|---|---|---|---|---|
| | 1 | 4 | | 4 (1) | 0.82 |
| | 2 | 247 | 31 (76) | 216 (76) | |
| | 3 | 74 | 10 (24) | 64 (22) | |
| | 4 | 2 | | 2 (1) | |

| Lymphatic Invasion | | | | | |
|---|---|---|---|---|---|
| | Absent | 238 | 28 (68) | 210 (73) | 0.62 |
| | Present | 89 | 13 (32) | 76 (27) | |

| Blood Vessel Invasion | | | | | |
|---|---|---|---|---|---|
| | Absent | 300 | 36 (88) | 264 (92) | 0.50 |
| | Present | 27 | 5 (12) | 22 (8) | |

| Site | | | | | |
|---|---|---|---|---|---|
| | Cecum, Ascending and Transverse Colon | 109 | 16 (39) | 93 (33) | 0.16 |
| | Descending and Sigmoid Colon, Rectum | 218 | 25 (61) | 193 (67) | |

Although the foregoing invention has been described in some detail by way of illustration and example for purposes of clarity of understanding, the descriptions and examples should not be construed as limiting the scope of the invention, which is only limited by the scope of the appended claims.

## Claims

1. A method for cancer prognosis in a human subject diagnosed with colorectal cancer, the method comprising: (a) comparing expression of EphB2 in a colorectal tissue or cell sample from the patient with expression of EphB2 in a control sample; and (b) predicting cancer prognosis of the patient based on the comparison in (a), wherein increased EphB2 expression in the patient sample relative to a control sample is prognostic for increased duration of survival or increased duration of recurrence-free survival in the subject.

2. A method for selection of colorectal cancer treatment for a human patient, the method comprising (a) comparing expression of EphB2 in a colorectal tissue or cell sample from the patient with expression of EphB2 in a control sample; (b) predicting cancer prognosis of the patient based on the comparison in (a), wherein increased EphB2 expression in the patient sample relative to the control sample is prognostic for increased duration of survival or increased duration of recurrence-free survival in the subject; and (c) subsequent to steps (a) and (b), selecting cancer treatment for the patient, wherein the selection of treatment is based on the patient prognosis determined in step (b).

3. The method of claim 1 or claim 2, wherein EphB2 polynucleotide expression is detected.

4. The method of any of claims 1-3, wherein EphB2 polypeptide expression is detected.

5. The method of claim 3, wherein EphB2 mRNA expression is detected.

6. The method of claim 4, wherein EphB2 polypeptide expression is detected using an anti-EphB2 agent.

7. The method of claim 6, wherein the anti-EphB2 agent is an antibody.

8. The method of claim 4, wherein EphB2 polypeptide expression is detected using immunohistochemistry.

9. The method of any of claims 1-8, further comprising detection of expression of any one or more EphB2 ligand.

10. The method of claim 2, wherein the treatment comprises administering an effective amount of an immunoconjugate comprising an anti-EphB2 antibody.

11. The method of claim 10, wherein the immunoconjugate comprises a maytansinoid.

12. The method of claim 10, wherein the immunoconjugate comprises MMAE.

13. The method of claim 2, wherein the treatment comprises any one or more of chemotherapy, radiation, and surgery.

14. The method of any of claims 10-13, wherein EphB2 polynucleotide expression is detected.

15. The method of any of claims 10-13, wherein EphB2 polypeptide expression is detected.

16. The method of claim 14, wherein EphB2 mRNA expression is detected.

17. The method of claim 15, wherein EphB2 polypeptide expression is detected using an anti-EphB2 agent.

18. The method of claim 17, wherein the anti-EphB2 agent is an antibody.

19. The method of claim 15, wherein EphB2 polypeptide expression is detected using immunohistochemistry.

20. The methods of any of claims 10-19, further comprising detection of expression of any one or more EphB2 ligand.

21. The method of any one of claims 10-20, wherein the anti-EphB2 antibody present in the immunoconjugate is selected from the group consisting of a monoclonal antibody, a human antibody, a humanized antibody, and an antibody fragment.

## Patentansprüche

1. Verfahren zur Prognose von Krebs in einem menschlichen Individuum, bei dem Kolorektalkrebs diagnostiziert wurde, wobei das Verfahren Folgendes umfasst: (a) Vergleichen der Expression von EphB2 in einer Kolorektalgewebe- oder Kolorektalzellprobe von dem Patienten mit der Expression von EphB2 in einer Kontrollprobe; und (b) Abgeben einer Krebsprognose für den Patienten basierend auf dem Vergleich aus (a), wobei erhöhte EphB2-Expression in der Patientenprobe im Vergleich zur Kontrollprobe eine längere Überlebensdauer oder längere rezidivfreie Überlebensdauer des Individuums prognostiziert.

2. Verfahren zur Auswahl einer Kolorektalkrebsbehandlung für einen menschlichen Patienten, wobei das Verfahren Folgendes umfasst: (a) Vergleichen der Expression von EphB2 in einer Kolorektalgewebe- oder Kolorektalzellprobe von dem Patienten mit der Expression von EphB2 in einer Kontrollprobe; (b) Abgeben einer Krebsprognose für den Patienten basierend auf dem Vergleich aus (a), wobei erhöhte EphB2-Expression in der Patientenprobe im Vergleich zur Kontrollprobe eine längere Überlebensdauer oder längere rezidivfreie Überlebensdauer des Individuums prognostiziert; und (c) nach Schritt (a) und (b) das Auswählen einer Krebsbehandlung für den Patienten, wobei die Wahl der Behandlung auf der in Schritt (b) bestimmten Patientenprognose basiert.

3. Verfahren nach Anspruch 1 oder Anspruch 2, wobei EphB2-Polynucleotidex-pression bestimmt wird.

4. Verfahren nach einem der Ansprüche 1-3, wobei EphB2-Polypeptidexpression bestimmt wird.

5. Verfahren nach Anspruch 3, wobei EphB2-mRNA-Expression bestimmt wird.

6. Verfahren nach Anspruch 4, wobei EphB2-Polypeptidexpression mithilfe eines Anti-EphB2-Mittels detektiert wird.

7. Verfahren nach Anspruch 6, wobei das Anti-EphB2-Mittel ein Antikörper ist.

8. Verfahren nach Anspruch 4, wobei die EphB2-Polypeptidexpression mithilfe von Immunhistochemie detektiert wird.

9. Verfahren nach einem der Ansprüche 1-8, das weiters die Detektion der Expression eines oder mehrerer EphB2-Liganden umfasst.

10. Verfahren nach Anspruch 2, wobei die Behandlung die Verabreichung einer wirksamen Menge eines Immunkonjugats umfasst, das einen Anti-EphB2-Antikörper umfasst.

11. Verfahren nach Anspruch 10, wobei das Immunkonjugat ein Maytansinoid umfasst.

12. Verfahren nach Anspruch 10, wobei das Immunkonjugat MMAE umfasst.

13. Verfahren nach Anspruch 2, wobei die Behandlung eine oder mehrere aus Chemotherapie, Bestrahlung und Operation umfasst.

14. Verfahren nach einem der Ansprüche 10-13, wobei EphB2-Polynucleotidexpression detektiert wird.

15. Verfahren nach einem der Ansprüche 10-13, wobei EphB2-Polypeptidexpression detektiert wird.

16. Verfahren nach Anspruch 14, wobei EphB2-mRNA-Expression detektiert wird.

17. Verfahren nach Anspruch 15, wobei EphB2-Polypeptidexpression unter Verwendung eines Anti-EphB2-Mittels detektiert wird.

18. Verfahren nach Anspruch 17, wobei da Anti-EphB2-Mittel ein Antikörper ist.

19. Verfahren nach Anspruch 15, wobei EphB2-Polypeptidexpression mithilfe von Immunhistochemie detektiert wird.

20. Verfahren nach einem der Ansprüche 10-19, das weiters die Detektion der Expression eines oder mehrerer EphB2-Liganden umfasst.

21. Verfahren nach einem der Ansprüche 10-20, wobei der im Immunkonjugat vorhandene Anti-EphB2-Antikörper aus der aus einem monoklonalen Antikörper, einem menschlichen Antikörper, einem humanisierten Antikörper und einem Antikörperfragment bestehenden Gruppe ausgewählt ist.

## Revendications

1. Procédé pour le pronostic d'un cancer chez un sujet humain chez lequel a été diagnostiqué un cancer colorectal, le procédé comprenant les étapes consistant à : (a) comparer l'expression d'EphB2 dans un échantillon de tissu colorectal ou cellules colorectales provenant du patient à l'expression d'EphB2 dans un échantillon témoin ; et (b) prédire le pronostic de cancer du patient sur la base de la comparaison en (a), dans lequel une expression accrue d'EphB2 dans l'échantillon provenant du patient par rapport à un échantillon témoin est un pronostic d'une durée accrue de survie ou d'une durée accrue de survie sans récidive chez le sujet.

2. Procédé pour la sélection d'un traitement du cancer colorectal pour un patient humain, le procédé comprenant les étapes consistant à : (a) comparer l'expression d'EphB2 dans un échantillon de tissu colorectal ou cellules colorectales provenant du patient à l'expression d'EphB2 dans un échantillon témoin ; (b) prédire le pronostic du cancer du patient sur la base de la comparaison en (a), une expression accrue d'EphB2 dans l'échantillon provenant du patient par rapport à l'échantillon témoin étant le pronostic d'une durée accrue de survie ou d'une durée accrue de survie sans récidive chez le sujet ; et (c), après les étapes (a) et (b), sélectionner le traitement du cancer du patient, la sélection du traitement étant basée sur le pronostic concernant le patient déterminé dans l'étape (b).

3. Procédé suivant la revendication 1 ou la revendication 2, dans lequel l'expression du polynucléotide d'EphB2 est détectée.

4. Procédé suivant l'une quelconque des revendications 1 à 3, dans lequel l'expression du polypeptide EphB2 est détectée.

5. Procédé suivant la revendication 3, dans lequel l'expression de l'ARNm d'EphB2 est détectée.

6. Procédé suivant la revendication 4, dans lequel l'expression du polypeptide EphB2 est détectée en utilisant un agent anti-EphB2.

7. Procédé suivant la revendication 6, dans lequel l'agent anti-EphB2 est un anticorps.

8. Procédé suivant la revendication 4, dans lequel l'expression du polypeptide EphB2 est détectée en utilisant l'immunohistochimie.

9. Procédé suivant l'une quelconque des revendications 1 à 8, comprenant en outre la détection de l'expression d'un ou plusieurs ligands quelconques d'EphB2.

10. Procédé suivant la revendication 2, dans lequel le traitement comprend l'administration d'une quantité efficace d'un immunoconjugué comprenant un anticorps anti-EphB2.

11. Procédé suivant la revendication 10, dans lequel l'immunoconjugué comprend un maytansinoïde.

12. Procédé suivant la revendication 10, dans lequel l'immunoconjugué comprend MMAE.

13. Procédé suivant la revendication 2, dans lequel le traitement comprend une ou plusieurs quelconques de la chimiothérapie, la radiothérapie et la chirurgie.

14. Procédé suivant l'une quelconque des revendications 10 à 13, dans lequel l'expression du polynucléotide EphB2 est détectée.

15. Procédé suivant l'une quelconque des revendications 10 à 13, dans lequel l'expression du polypeptide EphB2 est détectée.

16. Procédé suivant la revendication 14, dans lequel l'expression de l'ARNm d'EphB2 est détectée.

17. Procédé suivant la revendication 15, dans lequel l'expression du polypeptide EphB2 est détectée en utilisant un agent anti-EphB2.

18. Procédé suivant la revendication 17, dans lequel l'agent anti-EphB2 est un anticorps.

19. Procédé suivant la revendication 15, dans lequel l'expression du polypeptide EphB2 est détectée en utilisant l'immunohistochimie.

20. Procédés suivant l'une quelconque des revendications 10 à 19, comprenant en outre la détection de l'expression d'un ou plusieurs quelconques ligands d'EphB2.

21. Procédé suivant l'une quelconque des revendications 10 à 20, dans lequel l'anticorps anti-EphB2 présent dans l'immunoconjugué est choisi dans le groupe consistant en un anticorps monoclonal, un anticorps humain, un anticorps humanisé et un fragment d'anticorps.


### Sequence Listing

<110> Genentech, Inc.
<120> CANCER PROGNOSTIC, DIAGNOSTIC AND TREATMENT METHODS
<130> P2201R1
<141> 2006-01-05
<150> US 60/642,164
   <151> 2005-01-06
<160> 16
<210> 1
   <211> 17
   <212> PRT
   <213> Mus musculus
<400> 1
   Lys Ser Ser Gln Ser Leu Leu Asn Ser Gly Asn Gln Glu Asn Tyr
   1               5                   10                  15
   Leu Ala
<210> 2
   <211> 7
   <212> PRT
   <213> Mus musculus
<400> 2
   Gly Ala Ser Thr Arg Glu Ser
   1               5
<210> 3
   <211> 9
   <212> PRT
   <213> Mus musculus
<400> 3
   Gln Asn Asp His Ser Tyr Pro Phe Thr
   1               5
<210> 4
   <211> 5
   <212> PRT
   <213> Mus musculus
<400> 4
   Ser Tyr Trp Met His
   1               5
<210> 5
   <211> 17
   <212> PRT
   <213> Mus musculus
<400> 5
   Phe Ile Asn Pro Ser Thr Gly Tyr Thr Asp Tyr Asn Gln Lys Phe
   1 5 10 15
   Lys Asp
<210> 6
   <211> 11
   <212> PRT
   <213> Mus musculus
<400> 6
   Arg Leu Lys Leu Leu Arg Tyr Ala Met Asp Tyr
   1 5 10
<210> 7
   <211> 114
   <212> PRT
   <213> Mus musculus
<400> 7
   Asp Ile Val Met Thr Gln Ser Pro Ser Ser Leu Ser Val Ser Ala
   1 5 10 15
   Gly Glu Lys Val Thr Met Asn Cys Lys Ser Ser Gln Ser Leu Leu
   20 25 30
   Asn Ser Gly Asn Gln Glu Asn Tyr Leu Ala Trp Tyr Gln Gln Lys
   35 40 45
   Pro Gly Gln Pro Pro Lys Leu Leu Ile Tyr Gly Ala Ser Thr Arg
   50 55 60
   Glu Ser Gly Val Pro Asp Arg Phe Thr Gly Ser Gly Ser Gly Thr
   65 70 75
   Asp Phe Thr Leu Thr Ile Ser Ser Val Gln Ala Glu Asp Leu Ala
   80 85 90
   Val Tyr Tyr Cys Gln Asn Asp His Ser Tyr Pro Phe Thr Phe Gly
   95 100 105
   Ala Gly Thr Lys Val Glu Ile Lys Arg
   110
<210> 8
   <211> 119
   <212> PRT
   <213> Mus musculus
<400> 8
   Gln Val Gln Leu Gln Gln Ser Gly Ala Glu Leu Ala Lys Pro Gly
   1 5 10 15
   Ala Ser Val Lys Met Ser Cys Lys Ala Ser Gly Tyr Thr Phe Thr
   20 25 30
   Ser Tyr Trp Met His Trp Val Lys Gln Arg Pro Gly Gln Gly Leu
   35 40 45
   Glu Trp Ile Gly Phe Ile Asn Pro Ser Thr Gly Tyr Thr Asp Tyr
   50 55 60
   Asn Gln Lys Phe Lys Asp Lys Ala Thr Leu Thr Val Lys Ser Ser
   65 70 75
   Asn Thr Ala Tyr Met Gln Leu Ser Arg Leu Thr Ser Glu Asp Ser
   80 85 90
   Ala Val Tyr Tyr Cys Thr Arg Arg Leu Lys Leu Leu Arg Tyr Ala
   95 100 105
   Met Asp Tyr Trp Gly Gln Gly Thr Thr Leu Thr Val Ser Ala
   110 115
<210> 9
   <211> 1055
   <212> PRT
   <213> Homo sapiens
<400> 9
   Met Ala Leu Arg Arg Leu Gly Ala Ala Leu Leu Leu Leu Pro Leu
    1               5                  10                  15
   Leu Ala Ala Val Glu Glu Thr Leu Met Asp Ser Thr Thr Ala Thr
                   20                  25                  30
   Ala Glu Leu Gly Trp Met Val His Pro Pro Ser Gly Trp Glu Glu
                   35                  40                  45
   Val Ser Gly Tyr Asp Glu Asn Met Asn Thr Ile Arg Thr Tyr Gln
                   50                  55                  60
   Val Cys Asn Val Phe Glu Ser Ser Gln Asn Asn Trp Leu Arg Thr
                   65                  70                  75
   Lys Phe Ile Arg Arg Arg Gly Ala His Arg Ile His Val Glu Met
                   80                  85                  90
   Lys Phe Ser Val Arg Asp Cys Ser Ser Ile Pro Ser Val Pro Gly
                   95                 100                 105
   Ser Cys Lys Glu Thr Phe Asn Leu Tyr Tyr Tyr Glu Ala Asp Phe
                  110                 115                 120
   Asp Ser Ala Thr Lys Thr Phe Pro Asn Trp Met Glu Asn Pro Trp
                  125                 130                 135
   Val Lys Val Asp Thr Ile Ala Ala Asp Glu Ser Phe Ser Gln Val
                  140                 145                 150
   Asp Leu Gly Gly Arg Val Met Lys Ile Asn Thr Glu Val Arg Ser
                  155                 160                 165
   Phe Gly Pro Val Ser Arg Ser Gly Phe Tyr Leu Ala Phe Gln Asp
                  170                 175                 180
   Tyr Gly Gly Cys Met Ser Leu Ile Ala Val Arg Val Phe Tyr Arg
                  185                 190                 195
   Lys Cys Pro Arg Ile Ile Gln Asn Gly Ala Ile Phe Gln Glu Thr
                   200                 205                 210
   Leu Ser Gly Ala Glu Ser Thr Ser Leu Val Ala Ala Arg Gly Ser
                   215                 220                 225
   Cys Ile Ala Asn Ala Glu Glu Val Asp Val Pro Ile Lys Leu Tyr
                   230                 235                 240
   Cys Asn Gly Asp Gly Glu Trp Leu Val Pro Ile Gly Arg Cys Met
                   245                 250                 255
   Cys Lys Ala Gly Phe Glu Ala Val Glu Asn Gly Thr Val Cys Arg
                   260                 265                 270
   Gly Cys Pro Ser Gly Thr Phe Lys Ala Asn Gln Gly Asp Glu Ala
                   275                 280                 285
   Cys Thr His Cys Pro Ile Asn Ser Arg Thr Thr Ser Glu Gly Ala
                   290                 295                 300
   Thr Asn Cys Val Cys Arg Asn Gly Tyr Tyr Arg Ala Asp Leu Asp
                   305                 310                 315
   Pro Leu Asp Met Pro Cys Thr Thr Ile Pro Ser Ala Pro Gln Ala
                   320                 325                 330
   Val Ile Ser Ser Val Asn Glu Thr Ser Leu Met Leu Glu Trp Thr
                   335                 340                 345
   Pro Pro Arg Asp Ser Gly Gly Arg Glu Asp Leu Val Tyr Asn Ile
                   350                 355                 360
   Ile Cys Lys Ser Cys Gly Ser Gly Arg Gly Ala Cys Thr Arg Cys
                   365                 370                 375
   Gly Asp Asn Val Gln Tyr Ala Pro Arg Gln Leu Gly Leu Thr Glu
                   380                 385                 390
   Pro Arg Ile Tyr Ile Ser Asp Leu Leu Ala His Thr Gln Tyr Thr
                   395                 400                 405
   Phe Glu Ile Gln Ala Val Asn Gly Val Thr Asp Gln Ser Pro Phe
                   410                 415                 420
   Ser Pro Gln Phe Ala Ser Val Asn Ile Thr Thr Asn Gln Ala Ala
                   425                 430                 435
   Pro Ser Ala Val Ser Ile Met His Gln Val Ser Arg Thr Val Asp
                   440                 445                 450
   Ser Ile Thr Leu Ser Trp Ser Gln Pro Asp Gln Pro Asn Gly Val
                   455                 460                 465
   Ile Leu Asp Tyr Glu Leu Gln Tyr Tyr Glu Lys Glu Leu Ser Glu
                   470                 475                 480
   Tyr Asn Ala Thr Ala Ile Lys Ser Pro Thr Asn Thr Val Thr Val
                   485                 490                 495
   Gln Gly Leu Lys Ala Gly Ala Ile Tyr Val Phe Gln Val Arg Ala
   500 505 510
   Arg Thr Val Ala Gly Tyr Gly Arg Tyr Ser Gly Lys Met Tyr Phe
   515 520 525
   Gln Thr Met Thr Glu Ala Glu Tyr Gln Thr Ser Ile Gln Glu Lys
   530 535 540
   Leu Pro Leu Ile Ile Gly Ser Ser Ala Ala Gly Leu Val Phe Leu
   545 550 555
   Ile Ala Val Val Val Ile Ala Ile Val Cys Asn Arg Arg Gly Phe
   560 565 570
   Glu Arg Ala Asp Ser Glu Tyr Thr Asp Lys Leu Gln His Tyr Thr
   575 580 585
   Ser Gly His Met Thr Pro Gly Met Lys Ile Tyr Ile Asp Pro Phe
   590 595 600
   Thr Tyr Glu Asp Pro Asn Glu Ala Val Arg Glu Phe Ala Lys Glu
   605 610 615
   Ile Asp Ile Ser Cys Val Lys Ile Glu Gln Val Ile Gly Ala Gly
   620 625 630
   Glu Phe Gly Glu Val Cys Ser Gly His Leu Lys Leu Pro Gly Lys
   635 640 645
   Arg Glu Ile Phe Val Ala Ile Lys Thr Leu Lys Ser Gly Tyr Thr
   650 655 660
   Glu Lys Gln Arg Arg Asp Phe Leu Ser Glu Ala Ser Ile Met Gly
   665 670 675
   Gln Phe Asp His Pro Asn Val Ile His Leu Glu Gly Val Val Thr
   680 685 690
   Lys Ser Thr Pro Val Met Ile Ile Thr Glu Phe Met Glu Asn Gly
   695 700 705
   Ser Leu Asp Ser Phe Leu Arg Gln Asn Asp Gly Gln Phe Thr Val
   710 715 720
   Ile Gln Leu Val Gly Met Leu Arg Gly Ile Ala Ala Gly Met Lys
   725 730 735
   Tyr Leu Ala Asp Met Asn Tyr Val His Arg Asp Leu Ala Ala Arg
   740 745 750
   Asn Ile Leu Val Asn Ser Asn Leu Val Cys Lys Val Ser Asp Phe
   755 760 765
   Gly Leu Ser Arg Phe Leu Glu Asp Asp Thr Ser Asp Pro Thr Tyr
   770 775 780
   Thr Ser Ala Leu Gly Gly Lys Ile Pro Ile Arg Trp Thr Ala Pro
   785 790 795
   Glu Ala Ile Gln Tyr Arg Lys Phe Thr Ser Ala Ser Asp Val Trp
                   800                 805                 810
   Ser Tyr Gly Ile Val Met Trp Glu Val Met Ser Tyr Gly Glu Arg
                   815                 820                 825
   Pro Tyr Trp Asp Met Thr Asn Gln Asp Val Ile Asn Ala Ile Glu
                   830                 835                 840
   Gln Asp Tyr Arg Leu Pro Pro Pro Met Asp Cys Pro Ser Ala Leu
                   845                 850                 855
   His Gln Leu Met Leu Asp Cys Trp Gln Lys Asp Arg Asn His Arg
                   860                 865                 870
   Pro Lys Phe Gly Gln Ile Val Asn Thr Leu Asp Lys Met Ile Arg
                   875                 880                 885
   Asn Pro Asn Ser Leu Lys Ala Met Ala Pro Leu Ser Ser Gly Ile
                   890                 895                 900
   Asn Leu Pro Leu Leu Asp Arg Thr Ile Pro Asp Tyr Thr Ser Phe
                   905                 910                 915
   Asn Thr Val Asp Glu Trp Leu Glu Ala Ile Lys Met Gly Gln Tyr
                   920                 925                 930
   Lys Glu Ser Phe Ala Asn Ala Gly Phe Thr Ser Phe Asp Val Val
                   935                 940                 945
   Ser Gln Met Met Met Glu Asp Ile Leu Arg Val Gly Val Thr Leu
                   950                 955                 960
   Ala Gly His Gln Lys Lys Ile Leu Asn Ser Ile Gln Val Met Arg
                   965                 970                 975
   Ala Gln Met Asn Gln Ile Gln Ser Val Glu Gly Gln Pro Leu Ala
                   980                 985                 990
   Arg Arg Pro Arg Ala Thr Gly Arg Thr Lys Arg Cys Gln Pro Arg
                   995                 1000                1005
   Asp Val Thr Lys Lys Thr Cys Asn Ser Asn Asp Gly Lys Lys Lys
                   1010                1015                1020
   Gly Met Gly Lys Lys Lys Thr Asp Pro Gly Arg Gly Arg Glu Ile
                   1025                1030                1035
   Gln Gly Ile Phe Phe Lys Glu Asp Ser His Lys Glu Ser Asn Asp
                   1040                1045                1050
   Cys Ser Cys Gly Gly
                   1055
<210> 10
   <211> 4641
   <212> DNA
   <213> Homo sapiens
<400> 10
   gccccgggaa gcgcagccat ggctctgcgg aggctggggg ccgcgctgct 50
   gctgctgccg ctgctcgccg ccgtggaaga aacgctaatg gactccacta 100
   cagcgactgc tgagctgggc tggatggtgc atcctccatc agggtgggaa 150
   gaggtgagtg gctacgatga gaacatgaac acgatccgca cgtaccaggt 200
   gtgcaacgtg tttgagtcaa gccagaacaa ctggctacgg accaagttta 250
   tccggcgccg tggcgcccac cgcatccacg tggagatgaa gttttcggtg 300
   cgtgactgca gcagcatccc cagcgtgcct ggctcctgca aggagacctt 350
   caacctctat tactatgagg ctgactttga ctcggccacc aagaccttcc 400
   ccaactggat ggagaatcca tgggtgaagg tggataccat tgcagccgac 450
   gagagcttct cccaggtgga cctgggtggc cgcgtcatga aaatcaacac 500
   cgaggtgcgg agcttcggac ctgtgtcccg cagcggcttc tacctggcct 550
   tccaggacta tggcggctgc atgtccctca tcgccgtgcg tgtcttctac 600
   cgcaagtgcc cccgcatcat ccagaatggc gccatcttcc aggaaaccct 650
   gtcgggggct gagagcacat cgctggtggc tgcccggggc agctgcatcg 700
   ccaatgcgga agaggtggat gtacccatca agctctactg taacggggac 750
   ggcgagtggc tggtgcccat cgggcgctgc atgtgcaaag caggcttcga 800
   ggccgttgag aatggcaccg tctgccgagg ttgtccatct gggactttca 850
   aggccaacca aggggatgag gcctgtaccc actgtcccat caacagccgg 900
   accacttctg aaggggccac caactgtgtc tgccgcaatg gctactacag 950
   agcagacctg gaccccctgg acatgccctg cacaaccatc ccctccgcgc 1000
   cccaggctgt gatttccagt gtcaatgaga cctccctcat gctggagtgg 1050
   acccctcccc gcgactccgg aggccgagag gacctcgtct acaacatcat 1100
   ctgcaagagc tgtggctcgg gccggggtgc ctgcacccgc tgcggggaca 1150
   atgtacagta cgcaccacgc cagctaggcc tgaccgagcc acgcatttac 1200
   atcagtgacc tgctggccca cacccagtac accttcgaga tccaggctgt 1250
   gaacggcgtt actgaccaga gccccttctc gcctcagttc gcctctgtga 1300
   acatcaccac caaccaggca gctccatcgg cagtgtccat catgcatcag 1350
   gtgagccgca ccgtggacag cattaccctg tcgtggtccc agccggacca 1400
   gcccaatggc gtgatcctgg actatgagct gcagtactat gagaaggagc 1450
   tcagtgagta caacgccaca gccataaaaa gccccaccaa cacggtcacc 1500
   gtgcagggcc tcaaagccgg cgccatctat gtcttccagg tgcgggcacg 1550
   caccgtggca ggctacgggc gctacagcgg caagatgtac ttccagacca 1600
   tgacagaagc cgagtaccag acaagcatcc aggagaagtt gccactcatc 1650
   atcggctcct cggccgctgg cctggtcttc ctcattgctg tggttgtcat 1700
   cgccatcgtg tgtaacagac gggggtttga gcgtgctgac tcggagtaca 1750
   cggacaagct gcaacactac accagtggcc acatgacccc aggcatgaag 1800
   atctacatcg atcctttcac ctacgaggac cccaacgagg cagtgcggga 1850
   gtttgccaag gaaattgaca tctcctgtgt caaaattgag caggtgatcg 1900
   gagcagggga gtttggcgag gtctgcagtg gccacctgaa gctgccaggc 1950
   aagagagaga tctttgtggc catcaagacg ctcaagtcgg gctacacgga 2000
   gaagcagcgc cgggacttcc tgagcgaagc ctccatcatg ggccagttcg 2050
   accatcccaa cgtcatccac ctggagggtg tcgtgaccaa gagcacacct 2100
   gtgatgatca tcaccgagtt catggagaat ggctccctgg actcctttct 2150
   ccggcaaaac gatgggcagt tcacagtcat ccagctggtg ggcatgcttc 2200
   ggggcatcgc agctggcatg aagtacctgg cagacatgaa ctatgttcac 2250
   cgtgacctgg ctgcccgcaa catcctcgtc aacagcaacc tggtctgcaa 2300
   ggtgtcggac tttgggctct cacgctttct agaggacgat acctcagacc 2350
   ccacctacac cagtgccctg ggcggaaaga tccccatccg ctggacagcc 2400
   ccggaagcca tccagtaccg gaagttcacc tcggccagtg atgtgtggag 2450
   ctacggcatt gtcatgtggg aggtgatgtc ctatggggag cggccctact 2500
   gggacatgac caaccaggat gtaatcaatg ccattgagca ggactatcgg 2550
   ctgccaccgc ccatggactg cccgagcgcc ctgcaccaac tcatgctgga 2600
   ctgttggcag aaggaccgca accaccggcc caagttcggc caaattgtca 2650
   acacgctaga caagatgatc cgcaatccca acagcctcaa agccatggcg 2700
   cccctctcct ctggcatcaa cctgccgctg ctggaccgca cgatccccga 2750
   ctacaccagc tttaacacgg tggacgagtg gctggaggcc atcaagatgg 2800
   ggcagtacaa ggagagcttc gccaatgccg gcttcacctc ctttgacgtc 2850
   gtgtctcaga tgatgatgga ggacattctc cgggttgggg tcactttggc 2900
   tggccaccag aaaaaaatcc tgaacagtat ccaggtgatg cgggcgcaga 2950
   tgaaccagat tcagtctgtg gagggccagc cactcgccag gaggccacgg 3000
   gccacgggaa gaaccaagcg gtgccagcca cgagacgtca ccaagaaaac 3050
   atgcaactca aacgacggaa aaaaaaaggg aatgggaaaa aagaaaacag 3100
   atcctgggag ggggcgggaa atacaaggaa tattttttaa agaggattct 3150
   cataaggaaa gcaatgactg ttcttgcggg ggataaaaaa gggcttggga 3200
   gattcatgcg atgtgtccaa tcggagacaa aagcagtttc tctccaactc 3250
   cctctgggaa ggtgacctgg ccagagccaa gaaacacttt cagaaaaaca 3300
   aatgtgaagg ggagagacag gggccgccct tggctcctgt ccctgctgct 3350
   cctctaggcc tcactcaaca accaagcgcc tggaggacgg gacagatgga 3400
   cagacagcca ccctgagaac ccctctggga aaatctattc ctgccaccac 3450
   tgggcaaaca gaagaatttt tctgtctttg gagagtattt tagaaactcc 3500
   aatgaaagac actgtttctc ctgttggctc acagggctga aaggggcttt 3550
   tgtcctcctg ggtcagggag aacgcgggga ccccagaaag gtcagccttc 3600
   ctgaggatgg gcaaccccca ggtctgcagc tccaggtaca tatcacgcgc 3650
   acagcctggc agcctggccc tcctggtgcc cactcccgcc agcccctgcc 3700
   tcgaggactg atactgcagt gactgccgtc agctccgact gccgctgaga 3750
   agggttgatc ctgcatctgg gtttgtttac agcaattcct ggactcgggg 3800
   gtattttggt cacagggtgg ttttggttta gggggtttgt ttgttgggtt 3850
   gttttttgtt ttttggtttt ttttaatgac aatgaagtga cactttgaca 3900
   tttcctacct tttgaggact tgatccttct ccaggaagaa ggtgctttct 3950
   gcttactgac ttaggcaata caccaagggc gagattttat atgcacattt 4000
   ctggattttt ttatacggtt ttcattgaca ctcttccctc ctcccacctg 4050
   ccaccaggcc tcaccaaagc ccactgccat ggggccatct gggccattca 4100
   gagactggag tgagatttgg gtgtggaggg ggaggcgcca aggtggagga 4150
   gcttcccact ccaggactgt tgatgaaagg gacagattga ggaggaagtg 4200
   ggctctgagg ctgcagggct ggaagtcctt gcccacttcc cactctcctg 4250
   ccccaatcta tctagtactt cccaggcaaa taggcccctt tgaggctcct 4300
   gagtgccctc agatggtcaa aacccagttt tccctctggg agcctaaacc 4350
   aggctgcatc ggaggccagg acccggatca ttcactgtga taccctgccc 4400
   tccagagggt gcgctcagag acacgggcaa gcatgcctct tcccttccct 4450
   ggagagaaag tgtgtgattt ctctcccacc tccttccccc caccagacct 4500
   ttgctgggcc taaaggtctt ggccatgggg acgccctcag tctagggatc 4550
   tggccacaga ctccctcctg tgaaccaaca cagacaccca agcagagcaa 4600
   tcagttagtg aattgaatgg aaataaacgc tttagttata a 4641
<210> 11
   <211> 987
   <212> PRT
   <213> Homo sapiens
<400> 11
   Met Ala Leu Arg Arg Leu Gly Ala Ala Leu Leu Leu Leu Pro Leu
    1               5                  10                  15
   Leu Ala Ala Val Glu Glu Thr Leu Met Asp Ser Thr Thr Ala Thr
                   20                  25                  30
   Ala Glu Leu Gly Trp Met Val His Pro Pro Ser Gly Trp Glu Glu
                   35                  40                  45
   Val Ser Gly Tyr Asp Glu Asn Met Asn Thr Ile Arg Thr Tyr Gln
                   50                  55                  60
   Val Cys Asn Val Phe Glu Ser Ser Gln Asn Asn Trp Leu Arg Thr
                   65                  70                  75
   Lys Phe Ile Arg Arg Arg Gly Ala His Arg Ile His Val Glu Met
                   80                  85                  90
   Lys Phe Ser Val Arg Asp Cys Ser Ser Ile Pro Ser Val Pro Gly
                   95                  100                 105
   Ser Cys Lys Glu Thr Phe Asn Leu Tyr Tyr Tyr Glu Ala Asp Phe
                   110                 115                 120
   Asp Ser Ala Thr Lys Thr Phe Pro Asn Trp Met Glu Asn Pro Trp
                   125                 130                 135
   Val Lys Val Asp Thr Ile Ala Ala Asp Glu Ser Phe Ser Gln Val
                   140                 145                 150
   Asp Leu Gly Gly Arg Val Met Lys Ile Asn Thr Glu Val Arg Ser
                   155                 160                 165
   Phe Gly Pro Val Ser Arg Ser Gly Phe Tyr Leu Ala Phe Gln Asp
                   170                 175                 180
   Tyr Gly Gly Cys Met Ser Leu Ile Ala Val Arg Val Phe Tyr Arg
                   185                 190                 195
   Lys Cys Pro Arg Ile Ile Gln Asn Gly Ala Ile Phe Gln Glu Thr
                   200                 205                 210
   Leu Ser Gly Ala Glu Ser Thr Ser Leu Val Ala Ala Arg Gly Ser
                   215                 220                 225
   Cys Ile Ala Asn Ala Glu Glu Val Asp Val Pro Ile Lys Leu Tyr
                   230                 235                 240
   Cys Asn Gly Asp Gly Glu Trp Leu Val Pro Ile Gly Arg Cys Met
                   245                 250                 255
   Cys Lys Ala Gly Phe Glu Ala Val Glu Asn Gly Thr Val Cys Arg
                   260                 265                 270
   Gly Cys Pro Ser Gly Thr Phe Lys Ala Asn Gln Gly Asp Glu Ala
                   275                 280                 285
   Cys Thr His Cys Pro Ile Asn Ser Arg Thr Thr Ser Glu Gly Ala
                   290                 295                 300
   Thr Asn Cys Val Cys Arg Asn Gly Tyr Tyr Arg Ala Asp Leu Asp
                   305                 310                 315
   Pro Leu Asp Met Pro Cys Thr Thr Ile Pro Ser Ala Pro Gln Ala
                   320                 325                 330
   Val Ile Ser Ser Val Asn Glu Thr Ser Leu Met Leu Glu Trp Thr
                   335                 340                 345
   Pro Pro Arg Asp Ser Gly Gly Arg Glu Asp Leu Val Tyr Asn Ile
                   350                 355                 360
   Ile Cys Lys Ser Cys Gly Ser Gly Arg Gly Ala Cys Thr Arg Cys
                   365                 370                 375
   Gly Asp Asn Val Gln Tyr Ala Pro Arg Gln Leu Gly Leu Thr Glu
                   380                 385                 390
   Pro Arg Ile Tyr Ile Ser Asp Leu Leu Ala His Thr Gln Tyr Thr
                   395                 400                 405
   Phe Glu Ile Gln Ala Val Asn Gly Val Thr Asp Gln Ser Pro Phe
                   410                 415                 420
   Ser Pro Gln Phe Ala Ser Val Asn Ile Thr Thr Asn Gln Ala Ala
                   425                 430                 435
   Pro Ser Ala Val Ser Ile Met His Gln Val Ser Arg Thr Val Asp
                   440                 445                 450
   Ser Ile Thr Leu Ser Trp Ser Gln Pro Asp Gln Pro Asn Gly Val
                   455                 460                 465
   Ile Leu Asp Tyr Glu Leu Gln Tyr Tyr Glu Lys Glu Leu Ser Glu
                   470                 475                 480
   Tyr Asn Ala Thr Ala Ile Lys Ser Pro Thr Asn Thr Val Thr Val
                   485                 490                 495
   Gln Gly Leu Lys Ala Gly Ala Ile Tyr Val Phe Gln Val Arg Ala
                   500                 505                 510
   Arg Thr Val Ala Gly Tyr Gly Arg Tyr Ser Gly Lys Met Tyr Phe
                   515                 520                 525
   Gln Thr Met Thr Glu Ala Glu Tyr Gln Thr Ser Ile Gln Glu Lys
                   530                 535                 540
   Leu Pro Leu Ile Ile Gly Ser Ser Ala Ala Gly Leu Val Phe Leu
                   545                 550                 555
   Ile Ala Val Val Val Ile Ala Ile Val Cys Asn Arg Arg Arg Gly
                   560                 565                 570
   Phe Glu Arg Ala Asp Ser Glu Tyr Thr Asp Lys Leu Gln His Tyr
   575 580 585
   Thr Ser Gly His Met Thr Pro Gly Met Lys Ile Tyr Ile Asp Pro
   590 595 600
   Phe Thr Tyr Glu Asp Pro Asn Glu Ala Val Arg Glu Phe Ala Lys
   605 610 615
   Glu Ile Asp Ile Ser Cys Val Lys Ile Glu Gln Val Ile Gly Ala
   620 625 630
   Gly Glu Phe Gly Glu Val Cys Ser Gly His Leu Lys Leu Pro Gly
   635 640 645
   Lys Arg Glu Ile Phe Val Ala Ile Lys Thr Leu Lys Ser Gly Tyr
   650 655 660
   Thr Glu Lys Gln Arg Arg Asp Phe Leu Ser Glu Ala Ser Ile Met
   665 670 675
   Gly Gln Phe Asp His Pro Asn Val Ile His Leu Glu Gly Val Val
   680 685 690
   Thr Lys Ser Thr Pro Val Met Ile Ile Thr Glu Phe Met Glu Asn
   695 700 705
   Gly Ser Leu Asp Ser Phe Leu Arg Gln Asn Asp Gly Gln Phe Thr
   710 715 720
   Val Ile Gln Leu Val Gly Met Leu Arg Gly Ile Ala Ala Gly Met
   725 730 735
   Lys Tyr Leu Ala Asp Met Asn Tyr Val His Arg Asp Leu Ala Ala
   740 745 750
   Arg Asn Ile Leu Val Asn Ser Asn Leu Val Cys Lys Val Ser Asp
   755 760 765
   Phe Gly Leu Ser Arg Phe Leu Glu Asp Asp Thr Ser Asp Pro Thr
   770 775 780
   Tyr Thr Ser Ala Leu Gly Gly Lys Ile Pro Ile Arg Trp Thr Ala
   785 790 795
   Pro Glu Ala Ile Gln Tyr Arg Lys Phe Thr Ser Ala Ser Asp Val
   800 805 810
   Trp Ser Tyr Gly Ile Val Met Trp Glu Val Met Ser Tyr Gly Glu
   815 820 825
   Arg Pro Tyr Trp Asp Met Thr Asn Gln Asp Val Ile Asn Ala Ile
   830 835 840
   Glu Gln Asp Tyr Arg Leu Pro Pro Pro Met Asp Cys Pro Ser Ala
   845 850 855
   Leu His Gln Leu Met Leu Asp Cys Trp Gln Lys Asp Arg Asn His
   860 865 870
   Arg Pro Lys Phe Gly Gln Ile Val Asn Thr Leu Asp Lys Met Ile
                   875                 880                 885
   Arg Asn Pro Asn Ser Leu Lys Ala Met Ala Pro Leu Ser Ser Gly
                   890                 895                 900
   Ile Asn Leu Pro Leu Leu Asp Arg Thr Ile Pro Asp Tyr Thr Ser
                   905                 910                 915
   Phe Asn Thr Val Asp Glu Trp Leu Glu Ala Ile Lys Met Gly Gln
                   920                 925                 930
   Tyr Lys Glu Ser Phe Ala Asn Ala Gly Phe Thr Ser Phe Asp Val
                   935                 940                 945
   Val Ser Gln Met Met Met Glu Asp Ile Leu Arg Val Gly Val Thr
                   950                 955                 960
   Leu Ala Gly His Gln Lys Lys Ile Leu Asn Ser Ile Gln Val Met
                   965                 970                 975
   Arg Ala Gln Met Asn Gln Ile Gln Ser Val Glu Val
                   980                 985
<210> 12
   <211> 4737
   <212> DNA
   <213> Homo sapiens
<400> 12
   gccccgggaa gcgcagccat ggctctgcgg aggctggggg ccgcgctgct 50
   gctgctgccg ctgctcgccg ccgtggaaga aacgctaatg gactccacta 100
   cagcgactgc tgagctgggc tggatggtgc atcctccatc agggtgggaa 150
   gaggtgagtg gctacgatga gaacatgaac acgatccgca cgtaccaggt 200
   gtgcaacgtg tttgagtcaa gccagaacaa ctggctacgg accaagttta 250
   tccggcgccg tggcgcccac cgcatccacg tggagatgaa gttttcggtg 300
   cgtgactgca gcagcatccc cagcgtgcct ggctcctgca aggagacctt 350
   caacctctat tactatgagg ctgactttga ctcggccacc aagaccttcc 400
   ccaactggat ggagaatcca tgggtgaagg tggataccat tgcagccgac 450
   gagagcttct cccaggtgga cctgggtggc cgcgtcatga aaatcaacac 500
   cgaggtgcgg agcttcggac ctgtgtcccg cagcggcttc tacctggcct 550
   tccaggacta tggcggctgc atgtccctca tcgccgtgcg tgtcttctac 600
   cgcaagtgcc cccgcatcat ccagaatggc gccatcttcc aggaaaccct 650
   gtcgggggct gagagcacat cgctggtggc tgcccggggc agctgcatcg 700
   ccaatgcgga agaggtggat gtacccatca agctctactg taacggggac 750
   ggcgagtggc tggtgcccat cgggcgctgc atgtgcaaag caggcttcga 800
   ggccgttgag aatggcaccg tctgccgagg ttgtccatct gggactttca 850
   aggccaacca aggggatgag gcctgtaccc actgtcccat caacagccgg 900
   accacttctg aaggggccac caactgtgtc tgccgcaatg gctactacag 950
   agcagacctg gaccccctgg acatgccctg cacaaccatc ccctccgcgc 1000
   cccaggctgt gatttccagt gtcaatgaga cctccctcat gctggagtgg 1050
   acccctcccc gcgactccgg aggccgagag gacctcgtct acaacatcat 1100
   ctgcaagagc tgtggctcgg gccggggtgc ctgcacccgc tgcggggaca 1150
   atgtacagta cgcaccacgc cagctaggcc tgaccgagcc acgcatttac 1200
   atcagtgacc tgctggccca cacccagtac accttcgaga tccaggctgt 1250
   gaacggcgtt actgaccaga gccccttctc gcctcagttc gcctctgtga 1300
   acatcaccac caaccaggca gctccatcgg cagtgtccat catgcatcag 1350
   gtgagccgca ccgtggacag cattaccctg tcgtggtccc agccggacca 1400
   gcccaatggc gtgatcctgg actatgagct gcagtactat gagaaggagc 1450
   tcagtgagta caacgccaca gccataaaaa gccccaccaa cacggtcacc 1500
   gtgcagggcc tcaaagccgg cgccatctat gtcttccagg tgcgggcacg 1550
   caccgtggca ggctacgggc gctacagcgg caagatgtac ttccagacca 1600
   tgacagaagc cgagtaccag acaagcatcc aggagaagtt gccactcatc 1650
   atcggctcct cggccgctgg cctggtcttc ctcattgctg tggttgtcat 1700
   cgccatcgtg tgtaacagaa gacgggggtt tgagcgtgct gactcggagt 1750
   acacggacaa gctgcaacac tacaccagtg gccacatgac cccaggcatg 1800
   aagatctaca tcgatccttt cacctacgag gaccccaacg aggcagtgcg 1850
   ggagtttgcc aaggaaattg acatctcctg tgtcaaaatt gagcaggtga 1900
   tcggagcagg ggagtttggc gaggtctgca gtggccacct gaagctgcca 1950
   ggcaagagag agatctttgt ggccatcaag acgctcaagt cgggctacac 2000
   ggagaagcag cgccgggact tcctgagcga agcctccatc atgggccagt 2050
   tcgaccatcc caacgtcatc cacctggagg gtgtcgtgac caagagcaca 2100
   cctgtgatga tcatcaccga gttcatggag aatggctccc tggactcctt 2150
   tctccggcaa aacgatgggc agttcacagt catccagctg gtgggcatgc 2200
   ttcggggcat cgcagctggc atgaagtacc tggcagacat gaactatgtt 2250
   caccgtgacc tggctgcccg caacatcctc gtcaacagca acctggtctg 2300
   caaggtgtcg gactttgggc tctcacgctt tctagaggac gatacctcag 2350
   accccaccta caccagtgcc ctgggcggaa agatccccat ccgctggaca 2400
   gccccggaag ccatccagta ccggaagttc acctcggcca gtgatgtgtg 2450
   gagctacggc attgtcatgt gggaggtgat gtcctatggg gagcggccct 2500
   actgggacat gaccaaccag gatgtaatca atgccattga gcaggactat 2550
   cggctgccac cgcccatgga ctgcccgagc gccctgcacc aactcatgct 2600
   ggactgttgg cagaaggacc gcaaccaccg gcccaagttc ggccaaattg 2650
   tcaacacgct agacaagatg atccgcaatc ccaacagcct caaagccatg 2700
   gcgcccctct cctctggcat caacctgccg ctgctggacc gcacgatccc 2750
   cgactacacc agctttaaca cggtggacga gtggctggag gccatcaaga 2800
   tggggcagta caaggagagc ttcgccaatg ccggcttcac ctcctttgac 2850
   gtcgtgtctc agatgatgat ggaggacatt ctccgggttg gggtcacttt 2900
   ggctggccac cagaaaaaaa tcctgaacag tatccaggtg atgcgggcgc 2950
   agatgaacca gattcagtct gtggaggttt gacattcacc tgcctcggct 3000
   cacctcttcc tccaagcccc gccccctctg ccccacgtgc cggccctcct 3050
   ggtgctctat ccactgcagg gccagccact cgccaggagg ccacgggcca 3100
   cgggaagaac caagcggtgc cagccacgag acgtcaccaa gaaaacatgc 3150
   aactcaaacg acggaaaaaa aaagggaatg ggaaaaaaga aaacagatcc 3200
   tgggaggggg cgggaaatac aaggaatatt ttttaaagag gattctcata 3250
   aggaaagcaa tgactgttct tgcgggggat aaaaaagggc ttgggagatt 3300
   catgcgatgt gtccaatcgg agacaaaagc agtttctctc caactccctc 3350
   tgggaaggtg acctggccag agccaagaaa cactttcaga aaaacaaatg 3400
   tgaaggggag agacaggggc cgcccttggc tcctgtccct gctgctcctc 3450
   taggcctcac tcaacaacca agcgcctgga ggacgggaca gatggacaga 3500
   cagccaccct gagaacccct ctgggaaaat ctattcctgc caccactggg 3550
   caaacagaag aatttttctg tctttggaga gtattttaga aactccaatg 3600
   aaagacactg tttctcctgt tggctcacag ggctgaaagg ggcttttgtc 3650
   ctcctgggtc agggagaacg cggggacccc agaaaggtca gccttcctga 3700
   ggatgggcaa cccccaggtc tgcagctcca ggtacatatc acgcgcacag 3750
   cctggcagcc tggccctcct ggtgcccact cccgccagcc cctgcctcga 3800
   ggactgatac tgcagtgact gccgtcagct ccgactgccg ctgagaaggg 3850
   ttgatcctgc atctgggttt gtttacagca attcctggac tcgggggtat 3900
   tttggtcaca gggtggtttt ggtttagggg gtttgtttgt tgggttgttt 3950
   tttgtttttt ggtttttttt aatgacaatg aagtgacact ttgacatttc 4000
   ctaccttttg aggacttgat ccttctccag gaagaaggtg ctttctgctt 4050
   actgacttag gcaatacacc aagggcgaga ttttatatgc acatttctgg 4100
   atttttttat acggttttca ttgacactct tccctcctcc cacctgccac 4150
   caggcctcac caaagcccac tgccatgggg ccatctgggc cattcagaga 4200
   ctggagtgag atttgggtgt ggagggggag gcgccaaggt ggaggagctt 4250
   cccactccag gactgttgat gaaagggaca gattgaggag gaagtgggct 4300
   ctgaggctgc agggctggaa gtccttgccc acttcccact ctcctgcccc 4350
   aatctatcta gtacttccca ggcaaatagg cccctttgag gctcctgagt 4400
   gccctcagat ggtcaaaacc cagttttccc tctgggagcc taaaccaggc 4450
   tgcatcggag gccaggaccc ggatcattca ctgtgatacc ctgccctcca 4500
   gagggtgcgc tcagagacac gggcaagcat gcctcttccc ttccctggag 4550
   agaaagtgtg tgatttctct cccacctcct tccccccacc agacctttgc 4600
   tgggcctaaa ggtcttggcc atggggacgc cctcagtcta gggatctggc 4650
   cacagactcc ctcctgtgaa ccaacacaga cacccaagca gagcaatcag 4700
   ttagtgaatt gaatggaaat aaacgcttta gttataa 4737
<210> 13
   <211> 986
   <212> PRT
   <213> Homo sapiens
<400> 13
   Met Ala Leu Arg Arg Leu Gly Ala Ala Leu Leu Leu Leu Pro Leu
    1               5                  10                  15
   Leu Ala Ala Val Glu Glu Thr Leu Met Asp Ser Thr Thr Ala Thr
                   20                  25                  30
   Ala Glu Leu Gly Trp Met Val His Pro Pro Ser Gly Trp Glu Glu
                   35                  40                  45
   Val Ser Gly Tyr Asp Glu Asn Met Asn Thr Ile Arg Thr Tyr Gln
                   50                  55                  60
   Val Cys Asn Val Phe Glu Ser Ser Gln Asn Asn Trp Leu Arg Thr
                   65                  70                  75
   Lys Phe Ile Arg Arg Arg Gly Ala His Arg Ile His Val Glu Met
                   80                  85                  90
   Lys Phe Ser Val Arg Asp Cys Ser Ser Ile Pro Ser Val Pro Gly
                   95                  100                 105
   Ser Cys Lys Glu Thr Phe Asn Leu Tyr Tyr Tyr Glu Ala Asp Phe
                   110                 115                 120
   Asp Ser Ala Thr Lys Thr Phe Pro Asn Trp Met Glu Asn Pro Trp
                   125                 130                 135
   Val Lys Val Asp Thr Ile Ala Ala Asp Glu Ser Phe Ser Gln Val
                   140                 145                 150
   Asp Leu Gly Gly Arg Val Met Lys Ile Asn Thr Glu Val Arg Ser
                   155                 160                 165
   Phe Gly Pro Val Ser Arg Ser Gly Phe Tyr Leu Ala Phe Gln Asp
                   170                 175                 180
   Tyr Gly Gly Cys Met Ser Leu Ile Ala Val Arg Val Phe Tyr Arg
                   185                 190                 195
   Lys Cys Pro Arg Ile Ile Gln Asn Gly Ala Ile Phe Gln Glu Thr
                   200                 205                 210
   Leu Ser Gly Ala Glu Ser Thr Ser Leu Val Ala Ala Arg Gly Ser
                   215                 220                 225
   Cys Ile Ala Asn Ala Glu Glu Val Asp Val Pro Ile Lys Leu Tyr
                   230                 235                 240
   Cys Asn Gly Asp Gly Glu Trp Leu Val Pro Ile Gly Arg Cys Met
                   245                 250                 255
   Cys Lys Ala Gly Phe Glu Ala Val Glu Asn Gly Thr Val Cys Arg
                   260                 265                 270
   Gly Cys Pro Ser Gly Thr Phe Lys Ala Asn Gln Gly Asp Glu Ala
                   275                 280                 285
   Cys Thr His Cys Pro Ile Asn Ser Arg Thr Thr Ser Glu Gly Ala
                   290                 295                 300
   Thr Asn Cys Val Cys Arg Asn Gly Tyr Tyr Arg Ala Asp Leu Asp
                   305                 310                 315
   Pro Leu Asp Met Pro Cys Thr Thr Ile Pro Ser Ala Pro Gln Ala
                   320                 325                 330
   Val Ile Ser Ser Val Asn Glu Thr Ser Leu Met Leu Glu Trp Thr
                   335                 340                 345
   Pro Pro Arg Asp Ser Gly Gly Arg Glu Asp Leu Val Tyr Asn Ile
                   350                 355                 360
   Ile Cys Lys Ser Cys Gly Ser Gly Arg Gly Ala Cys Thr Arg Cys
                   365                 370                 375
   Gly Asp Asn Val Gln Tyr Ala Pro Arg Gln Leu Gly Leu Thr Glu
                   380                 385                 390
   Pro Arg Ile Tyr Ile Ser Asp Leu Leu Ala His Thr Gln Tyr Thr
                   395                 400                 405
   Phe Glu Ile Gln Ala Val Asn Gly Val Thr Asp Gln Ser Pro Phe
                   410                 415                 420
   Ser Pro Gln Phe Ala Ser Val Asn Ile Thr Thr Asn Gln Ala Ala
                   425                 430                 435
   Pro Ser Ala Val Ser Ile Met His Gln Val Ser Arg Thr Val Asp
                   440                 445                 450
   Ser Ile Thr Leu Ser Trp Ser Gln Pro Asp Gln Pro Asn Gly Val
                   455                 460                 465
   Ile Leu Asp Tyr Glu Leu Gln Tyr Tyr Glu Lys Glu Leu Ser Glu
                   470                 475                 480
   Tyr Asn Ala Thr Ala Ile Lys Ser Pro Thr Asn Thr Val Thr Val
                   485                 490                 495
   Gln Gly Leu Lys Ala Gly Ala Ile Tyr Val Phe Gln Val Arg Ala
                   500                 505                 510
   Arg Thr Val Ala Gly Tyr Gly Arg Tyr Ser Gly Lys Met Tyr Phe
                   515                 520                 525
   Gln Thr Met Thr Glu Ala Glu Tyr Gln Thr Ser Ile Gln Glu Lys
                   530                 535                 540
   Leu Pro Leu Ile Ile Gly Ser Ser Ala Ala Gly Leu Val Phe Leu
                   545                 550                 555
   Ile Ala Val Val Val Ile Ala Ile Val Cys Asn Arg Arg Gly Phe
                   560                 565                 570
   Glu Arg Ala Asp Ser Glu Tyr Thr Asp Lys Leu Gln His Tyr Thr
                   575                 580                 585
   Ser Gly His Met Thr Pro Gly Met Lys Ile Tyr Ile Asp Pro Phe
                   590                 595                 600
   Thr Tyr Glu Asp Pro Asn Glu Ala Val Arg Glu Phe Ala Lys Glu
                   605                 610                 615
   Ile Asp Ile Ser Cys Val Lys Ile Glu Gln Val Ile Gly Ala Gly
                   620                 625                 630
   Glu Phe Gly Glu Val Cys Ser Gly His Leu Lys Leu Pro Gly Lys
                   635                 640                 645
   Arg Glu Ile Phe Val Ala Ile Lys Thr Leu Lys Ser Gly Tyr Thr
                   650                 655                 660
   Glu Lys Gln Arg Arg Asp Phe Leu Ser Glu Ala Ser Ile Met Gly
                   665                 670                 675
   Gln Phe Asp His Pro Asn Val Ile His Leu Glu Gly Val Val Thr
                   680                 685                 690
   Lys Ser Thr Pro Val Met Ile Ile Thr Glu Phe Met Glu Asn Gly
   695 700 705
   Ser Leu Asp Ser Phe Leu Arg Gln Asn Asp Gly Gln Phe Thr Val
   710 715 720
   Ile Gln Leu Val Gly Met Leu Arg Gly Ile Ala Ala Gly Met Lys
   725 730 735
   Tyr Leu Ala Asp Met Asn Tyr Val His Arg Asp Leu Ala Ala Arg
   740 745 750
   Asn Ile Leu Val Asn Ser Asn Leu Val Cys Lys Val Ser Asp Phe
   755 760 765
   Gly Leu Ser Arg Phe Leu Glu Asp Asp Thr Ser Asp Pro Thr Tyr
   770 775 780
   Thr Ser Ala Leu Gly Gly Lys Ile Pro Ile Arg Trp Thr Ala Pro
   785 790 795
   Glu Ala Ile Gln Tyr Arg Lys Phe Thr Ser Ala Ser Asp Val Trp
   800 805 810
   Ser Tyr Gly Ile Val Met Trp Glu Val Met Ser Tyr Gly Glu Arg
   815 820 825
   Pro Tyr Trp Asp Met Thr Asn Gln Asp Val Ile Asn Ala Ile Glu
   830 835 840
   Gln Asp Tyr Arg Leu Pro Pro Pro Met Asp Cys Pro Ser Ala Leu
   845 850 855
   His Gln Leu Met Leu Asp Cys Trp Gln Lys Asp Arg Asn His Arg
   860 865 870
   Pro Lys Phe Gly Gln Ile Val Asn Thr Leu Asp Lys Met Ile Arg
   875 880 885
   Asn Pro Asn Ser Leu Lys Ala Met Ala Pro Leu Ser Ser Gly Ile
   890 895 900
   Asn Leu Pro Leu Leu Asp Arg Thr Ile Pro Asp Tyr Thr Ser Phe
   905 910 915
   Asn Thr Val Asp Glu Trp Leu Glu Ala Ile Lys Met Gly Gln Tyr
   920 925 930
   Lys Glu Ser Phe Ala Asn Ala Gly Phe Thr Ser Phe Asp Val Val
   935 940 945
   Ser Gln Met Met Met Glu Asp Ile Leu Arg Val Gly Val Thr Leu
   950 955 960
   Ala Gly His Gln Lys Lys Ile Leu Asn Ser Ile Gln Val Met Arg
   965 970 975
   Ala Gln Met Asn Gln Ile Gln Ser Val Glu Val
   980 985
<210> 14
   <211> 3800
   <212> DNA
   <213> Homo sapiens
<400> 14
   cgggaagcgc agccatggct ctgcggaggc tgggggccgc gctgctgctg 50
   ctgccgctgc tcgccgccgt ggaagaaacg ctaatggact ccactacagc 100
   gactgctgag ctgggctgga tggtgcatcc tccatcaggg tgggaagagg 150
   tgagtggcta cgatgagaac atgaacacga tccgcacgta ccaggtgtgc 200
   aacgtgtttg agtcaagcca gaacaactgg ctacggacca agtttatccg 250
   gcgccgtggc gcccaccgca tccacgtgga gatgaagttt tcggtgcgtg 300
   actgcagcag catccccagc gtgcctggct cctgcaagga gaccttcaac 350
   ctctattact atgaggctga ctttgactcg gccaccaaga ccttccccaa 400
   ctggatggag aatccatggg tgaaggtgga taccattgca gccgacgaga 450
   gcttctccca ggtggacctg ggtggccgcg tcatgaaaat caacaccgag 500
   gtgcggagct tcggacctgt gtcccgcagc ggcttctacc tggccttcca 550
   ggactatggc ggctgcatgt ccctcatcgc cgtgcgtgtc ttctaccgca 600
   agtgcccccg catcatccag aatggcgcca tcttccagga aaccctgtcg 650
   ggggctgaga gcacatcgct ggtggctgcc cggggcagct gcatcgccaa 700
   tgcggaagag gtggatgtac ccatcaagct ctactgtaac ggggacggcg 750
   agtggctggt gcccatcggg cgctgcatgt gcaaagcagg cttcgaggcc 800
   gttgagaatg gcaccgtctg ccgaggttgt ccatctggga ctttcaaggc 850
   caaccaaggg gatgaggcct gtacccactg tcccatcaac agccggacca 900
   cttctgaagg ggccaccaac tgtgtctgcc gcaatggcta ctacagagca 950
   gacctggacc ccctggacat gccctgcaca accatcccct ccgcgcccca 1000
   ggctgtgatt tccagtgtca atgagacctc cctcatgctg gagtggaccc 1050
   ctccccgcga ctccggaggc cgagaggacc tcgtctacaa catcatctgc 1100
   aagagctgtg gctcgggccg gggtgcctgc acccgctgcg gggacaatgt 1150
   acagtacgca ccacgccagc taggcctgac cgagccacgc atttacatca 1200
   gtgacctgct ggcccacacc cagtacacct tcgagatcca ggctgtgaac 1250
   ggcgttactg accagagccc cttctcgcct cagttcgcct ctgtgaacat 1300
   caccaccaac caggcagctc catcggcagt gtccatcatg catcaggtga 1350
   gccgcaccgt ggacagcatt accctgtcgt ggtcccagcc ggaccagccc 1400
   aatggcgtga tcctggacta tgagctgcag tactatgaga aggagctcag 1450
   tgagtacaac gccacagcca taaaaagccc caccaacacg gtcaccgtgc 1500
   agggcctcaa agccggcgcc atctatgtct tccaggtgcg ggcacgcacc 1550
   gtggcaggct acgggcgcta cagcggcaag atgtacttcc agaccatgac 1600
   agaagccgag taccagacaa gcatccagga gaagttgcca ctcatcatcg 1650
   gctcctcggc cgctggcctg gtcttcctca ttgctgtggt tgtcatcgcc 1700
   atcgtgtgta acagacgggg gtttgagcgt gctgactcgg agtacacgga 1750
   caagctgcaa cactacacca gtggccacat gaccccaggc atgaagatct 1800
   acatcgatcc tttcacctac gaggacccca acgaggcagt gcgggagttt 1850
   gccaaggaaa ttgacatctc ctgtgtcaaa attgagcagg tgatcggagc 1900
   aggggagttt ggcgaggtct gcagtggcca cctgaagctg ccaggcaaga 1950
   gagagatctt tgtggccatc aagacgctca agtcgggcta cacggagaag 2000
   cagcgccggg acttcctgag cgaagcctcc atcatgggcc agttcgacca 2050
   tcccaacgtc atccacctgg agggtgtcgt gaccaagagc acacctgtga 2100
   tgatcatcac cgagttcatg gagaatggct ccctggactc ctttctccgg 2150
   caaaacgatg ggcagttcac agtcatccag ctggtgggca tgcttcgggg 2200
   catcgcagct ggcatgaagt acctggcaga catgaactat gttcaccgtg 2250
   acctggctgc ccgcaacatc ctcgtcaaca gcaacctggt ctgcaaggtg 2300
   tcggactttg ggctctcacg ctttctagag gacgatacct cagaccccac 2350
   ctacaccagt gccctgggcg gaaagatccc catccgctgg acagccccgg 2400
   aagccatcca gtaccggaag ttcacctcgg ccagtgatgt gtggagctac 2450
   ggcattgtca tgtgggaggt gatgtcctat ggggagcggc cctactggga 2500
   catgaccaac caggatgtaa tcaatgccat tgagcaggac tatcggctgc 2550
   caccgcccat ggactgcccg agcgccctgc accaactcat gctggactgt 2600
   tggcagaagg accgcaacca ccggcccaag ttcggccaaa ttgtcaacac 2650
   gctagacaag atgatccgca atcccaacag cctcaaagcc atggcgcccc 2700
   tctcctctgg catcaacctg ccgctgctgg accgcacgat ccccgactac 2750
   accagcttta acacggtgga cgagtggctg gaggccatca agatggggca 2800
   gtacaaggag agcttcgcca atgccggctt cacctccttt gacgtcgtgt 2850
   ctcagatgat gatggaggac attctccggg ttggggtcac tttggctggc 2900
   caccagaaaa aaatcctgaa cagtatccag gtgatgcggg cgcagatgaa 2950
   ccagattcag tctgtggagg tttgacattc acctgcctcg gctcacctct 3000
   tcctccaagc cccgccccct ctgccccacg tgccggccct cctggtgctc 3050
   tatccactgc agggccagcc actcgccagg aggccacggg ccacgggaag 3100
   aaccaagcgg tgccagccac gagacgtcac caagaaaaca tgcaactcaa 3150
   acgacggaaa aaaaaaggga atgggaaaaa agaaaacaga tcctgggagg 3200
   gggcgggaaa tacaaggaat attttttaaa gaggattctc ataaggaaag 3250
   caatgactgt tcttgcgggg gataaaaaag ggcttgggag attcatgcga 3300
   tgtgtccaat cggagacaaa agcagtttct ctccaactcc ctctgggaag 3350
   gtgacctggc cagagccaag aaacactttc agaaaaacaa atgtgaaggg 3400
   gagagacagg ggccgccctt ggctcctgtc cctgctgctc ctctaggcct 3450
   cactcaacaa ccaagcgcct ggaggacggg acagatggac agacagccac 3500
   cctgagaacc cctctgggaa aatctattcc tgccaccact gggcaaacag 3550
   aagaattttt ctgtctttgg agagtatttt agaaactcca atgaaagaca 3600
   ctgtttctcc tgttggctca cagggctgaa aggggctttt gtcctcctgg 3650
   gtcagggaga acgcggggac cccagaaagg tcagccttcc tgaggatggg 3700
   caacccccag gtctgcagct ccaggtacat atcacgcgca cagcctggca 3750
   gcctggccct cctggtgccc actcccgcca gcccctgcct cgaggtcgac 3800
<210> 15
   <211> 20
   <212> DNA
   <213> Homo sapiens
<400> 15
   gccctcctgg tgctctatcc 20
<210> 16
   <211> 21
   <212> DNA
   <213> Homo sapiens
<400> 16
   tctgtccatc tgtcccgtcc t 21